# EUROPEAN PATENT APPLICATION

(11) **EP 2 292 587 A1**
(43) Date of publication of application: **09.03.2011**
(21) Application number: 09720498.6
(22) Date of filing: 11.03.2009
(51) Int. Cl.: C07C 233/64, C07C 231/00, A61K 31/194, A61P 35/00

(54) **PHARMACEUTICAL COMPOSITION FOR INHIBITING THE TRANSCRIPTION FACTOR INDUCIBLE BY HYPOXIA, MODULATORS OF PATHOLOGICAL PROCESSES OF ANGIOGENESIS, ONCOGENESIS, INFLAMMATION, APOPTOSIS, AND CELLULAR THERAPY**

(30) Priority: 11.03.2008 ES 200800708
(71) Applicant: Fundacion De La Comunidad Valenciana Centro De Investigacion Principe Felipe, 46013 Valencia (ES); Instituto de Salud Carlos III, 28029 Madrid (ES); Universidad de Valencia, 46010 Valencia (ES)
(72) Inventor: GARIJO OLMO, Raquel, E-46013 Valencia (ES); CHIVA TÁRREGA, E-46013 Valencia (ES); ACEÑA BONILLA, José Luis, E-46013 Valencia (ES); RODRÍGUEZ JIMÉNEZ, Francisco Javier, E-46013 Valencia (ES); FARRÁS RIVERA, Rosa, E-46013 Valencia (ES); LUCAS DOMÍNGUEZ, Rut, E-46013 Valencia (ES); MULLOR SANJOSÉ, José Luis, E-46013 Valencia (ES); SANZ-CERVERA, Juan, Francisco, 46013 Valencia (ES); MORENO MANZANO, E-46013 Valencia (ES); FUSTERO LARDÍES, Santos, E-46013 Valencia (ES); SÁNCHEZ-PUELLES GONZÁLEZ-CARVAJAL, E-46013 Valencia (ES)
(74) Representative: Gonzalez-Alberto Rodriguez, Natalia
(86) International application number: PCT/ES2009/000135
(87) International publication number: WO 2009/112615

(57) **Abstract**

The present invention is included in the field of pharmacology and medical chemistry and relates to novel molecules represented by general formula (I), especially the one called FM19G11, as well as to pharmaceutical compositions containing them. Said pharmacologically optimized compositions are capable of modulating and/or inhibiting the transcription of genes modulated by the hypoxia-inducible transcription factor (HIF) and which are directly and/or indirectly involved in pathological processes related to cancer, inflammation, tissue repair, stem cell differentiation and regenerative therapy.

The present invention also relates to a method for the synthesis of said molecules (I) and to the use thereof in the manufacture of a medicament for the treatment of the mentioned pathological processes.

SEQUENCE LISTING

<110>  Fundación de la Comunidad Valenciana Centro de
       Investigación Príncipe Felipe

<120>  COMPOSICIÓN FARMACÉUTICA PARA INHIBIR EL FACTOR DE TRANSCRIPCIÓN
       INDUCIBLE POR HIPOXIA. MODULADORES DE PROCESOS PATOLÓGICOS DE
       ANGIOGÉNESIS, ONCOGÉNESIS, INFLAMACIÓN, APOPTOSIS Y TERAPIA
       CELULAR

<130>  AX090022WO

<160>  14

<170>  PatentIn version 3.3

<210>  1
<211>  20
<212>  DNA
<213>  Artificial

<220>
<223>  Cebador VEGF FW

<400>  1
cctccgaaac catgaacttt                                                  20


<210>  2
<211>  21
<212>  DNA
<213>  Artificial

<220>
<223>  Cebador VEGF Rev

<400>  2
atctgcatgg tgatgttgga c                                                21


<210>  3
<211>  20
<212>  DNA
<213>  Artificial

<220>
<223>  Cebador GAPDH FW

<400>  3
catcttccag gagcgagatc                                                  20


<210>  4
<211>  21
<212>  DNA
<213>  Artificial

<220>
<223>  Cebador GAPDH Rev

<400>  4
gttcacaccc atgacgaaca t                                                21

<210> 5
<211> 20
<212> DNA
<213> Artificial

<220>
<223> Cebador rSox2 sentido

<400> 5
ggatggttgt ctattaactt 20


<210> 6
<211> 20
<212> DNA
<213> Artificial

<220>
<223> rSox2 antisentido

<400> 6
ctccatcatg ttatacatgc 20


<210> 7
<211> 20
<212> DNA
<213> Artificial

<220>
<223> rOct3/4 sentido

<400> 7
gctggacacc tggcttcaga 20


<210> 8
<211> 20
<212> DNA
<213> Artificial

<220>
<223> rOct3/4 antisentido

<400> 8
tctgaagcca ggtgtccagc 20


<210> 9
<211> 20
<212> DNA
<213> Artificial

<220>
<223> rGAPDH sentido

<400> 9
agcggcatct tcttgtgcag 20


<210> 10
<211> 20

<212>  DNA
<213>  Artificial

<220>
<223>  rGAPDH antisentido

<400>  10
ttgccgtggg tagagtcata                                                    20


<210>  11
<211>  20
<212>  DNA
<213>  Artificial

<220>
<223>  rNanog sentido

<400>  11
tagccctgat tcttctagca                                                    20


<210>  12
<211>  20
<212>  DNA
<213>  Artificial

<220>
<223>  rNanog antisentido

<400>  12
tacaccagga ctgagtgctc                                                    20


<210>  13
<211>  25
<212>  DNA
<213>  Artificial

<220>
<223>  rTGF-  sentido

<400>  13
tcgctctgct agcgctgggt atcct                                              25


<210>  14
<211>  25
<212>  DNA
<213>  Artificial

<220>
<223>  rTGF-  antisentido

<400>  14
actgtctcag agtggcagca ggcag                                              25

## Description

### Technical Field of the Invention

The present invention is included in the field of pharmacology and medical chemistry and relates to novel molecules represented by general formula (I), especially the one called FM19G11, as well as to the pharmaceutical compositions containing them. Said pharmacologically optimized compositions are capable of modulating and/or inhibiting the transcription of genes modulated by the hypoxia-inducible transcription factor (hereinafter, HIF) and which are directly and/or indirectly involved in pathological processes related to cancer, inflammation, tissue repair, stem cell differentiation and regenerative therapy.

The present invention also relates to a method for the synthesis of said molecules (I) and to the use thereof in the manufacture of a medicament for the treatment of the mentioned pathological processes.

### Background of the Invention

Virtually all the cells of the organism are sensitive to variations in the levels of nutrients and among them the most important for cell physiology is oxygen. The decrease in the levels of cellular oxygen is closely related to its importance in cell proliferation, for example in the endothelial cells which form new blood vessels, a phenomenon known as angiogenesis. Tumor cells have responses to the depletion of oxygen by means of the activation of a characteristic series of genes which allow them to survive and react against these adverse conditions. In recent years, hypoxia-inducible transcription factors (HIF) have been the object of a number of studies as key regulators of the cell response to the lack of oxygen and have been widely related in scientific literature to pathological processes of cancer, of inflammation, of cardiovascular and neurodegenerative pathologies and, generally of all those referred to as angiogenic pathologies, such as diabetic retinopathy, rheumatoid arthritis, arteriosclerotic plaques, endometriosis, Crohn's disease, psoriasis, benign prostatic hypertrophy, and cancer. (Folkman, "Angiogenesis: an organizing principle for drug discovery? " Nat. Rev. Drug Disc., 2007; 6, 273-286; Mazure, "Hypoxia signalling in cancer and approaches to enforce tumour regression" Nature, 2006; 44, 437- 443). Therapy, especially in cancer, is increasingly based on the use of compositions which are mixtures of active compounds, with different modes of action and non-accumulative toxicities. Therefore, the need for combination therapies requires the characterization of a larger collection of effective compounds in the fight against oncological disease, especially if the current trend of personalized medicine is furthermore taken into account.

Stem cells are especially relevant for studying the etiology of tumor processes as well as their potential in clinical practice. There is evidence that certain brain tumors, such as the glioblastomas, medulloblastomas and astrocytomas for example, contain cells with multipotentiality capacity similar to certain neural progenitors. It has recently been verified that hypoxia is a factor which is closely associated with processes of undifferentiation of adult stem cells. In fact, the location of some adult stem cells coincides with hypoxic and/or highly vascularized areas (Palmer et al. "Vascular niche for adult hippocampal neurogenesis, "The Journal of Comparative Neurology 2000; 425, 479-494.; Maurer et al., "Expression of vascular endothelial growth factor and its receptors in rat neural stem cells" Neurosci. Lett. 2003; 344, 165-168). The use of stem cells coming from the same tissue, compromised and influenced by the same microenvironment to which they will be subjected, could be a more efficient alternative in the assays for the regeneration of the lesioned cell system. Therefore, the present invention also has a potential relevance within the field of the modern discipline of regenerative medicine.

### HIF and Cancer

Although the cell and molecular bases underlying hypoxia phenomena are complex and varied, some of the key molecules are starting to be known. The adaptive cell response against the decrease in the levels of oxygen (hypoxia) is performed by means of inducing a characteristic gene expression pattern. One of these key molecules regulating the cell response to hypoxia is the hypoxia-inducible transcription factor (HIF). HIF is a heterodimer made up of two subunits expressed both constitutively (HIF 1β) and hypoxia-induced (HIF1α, HIF2α). Under normoxic conditions HIF1α and HIF2α are undetectable, since their regulation is mediated by the processes of ubiquitination and quick degradation by the proteasome. The VHL oncogene protein acts as a substrate of recognition of the E3 Ubiquitin ligase complex which signals HIF1α and HIF2α for the degradation thereof by the proteasome. Both complexes, VHL/HIF1α and VHL/HIF2α depend on the specific hydroxylation of two proline residues located in the protein, which hydroxylation is catalyzed by a new family of 2-oxoglutarate-dependent dioxygenases (prolyl-hydroxylases) (Semenza G.L. "Targeting HIF-1 for cancer therapy" Nature Rev. 2003; 3, 721-732; Lando D. et al. "Oxygen-dependent regulation of hypoxia-inducible factors by prolyl and asparaginyl hydroxylation" Eur J Biochem. 2003; 270, 781-90). Due to the need for oxygen of these enzymes to carry out their catalytic reaction, it is commonly accepted that the latter are the real sensors of the presence of oxygen to regulate the cytoplasmic levels of HIF1α/HIF2α. In normoxic and active proliferation conditions of the cells it is observed that the proliferation and survival pathways triggered by growth factors also induce the expression of HIF1α/HIF2α by the activation of the PI3K/Akt and Ras/RAF/MEKK signaling pathways (Poulaki V. et al. "Acute intensive insulin therapy exacerbates diabetic blood-retinal barrier breakdown via hypoxia-inducible factor-1alpha and VEGF" J Clin Invest. 2002 ; 109, 805-15; Hudson C.C. et al. "Regulation of hypoxia-inducible factor 1 alpha expression and function by the mammalian target of rapamycin" Mol Cell Biol. 2002 ; 22:7004-14; Guba M. et al., "Rapamycin inhibits primary and metastatic tumor growth by antiangiogenesis: involvement of vascular endothelial growth factor" Nat. Med 2002 ; 8, 128-35). When the cells face an oxygen deficiency, HIF1α/HIF2α accumulate in the cytosol and this accumulation allows their interaction with HIF1β, their translocation to the nucleus and the activation of the genes -more than two hundred different genes-, which have been described as target genes of HIF and which mediate the cell response induced by these transcription factors (Semenza G.L. "Targeting HIF-1 for cancer therapy" Nature Rev. 2003; 3, 721-732; Paul S.A et al., "HIF at the crossroads between ischemia and carcinogenesis" J. Cel. Physiol. 2004; 200, 20-30). Some of the HIF-induced genes are included within the molecular factors known as growth factors, such as the vascular endothelial growth factor (VEGF), the insulin growth factor (IGF) and the tissue growth factor β (TGF), to mention a few. Given that processes of active cell proliferation directly or indirectly involve the generation of lack of oxygen in the cell, hypoxia can be considered as even a physiological characteristic. The decrease of the levels of oxygen in the cell and the consequent stabilization of the cytoplasmic HIF protein entails, among other effects, the recruitment of bone marrow-derived circulating endothelial cells and the proliferation of vascular endothelial cells which promote neovascularization in ischemic and tumor processes (Harris et al., "Hypoxia a key regulatory factor in tumour growth." Nat Rev Cancer. 2002; 2, 38-47; Takahashi T. et al., "Ischemia- and cytokine-induced mobilization of bone marrow-derived endothelial progenitor cells for neovascularization" Nat Med. 1999; 5, 434-8). HIF can be controlled by a large variety of genes which mediate responses related to energy, survival, neovascularization, cell migration, cell adhesion metabolisms and its importance as a tumor progression promoter has been demonstrated (Mazure, "Hypoxia signalling in cancer and approaches to enforce tumour regression", Nature 2006; 44, 437- 443; Semenza G.L. "Targeting HIF-1 for cancer therapy" Nature Rev. 2003; 3, 721-732). In addition to acting as an oxygen homeostasis "controller" and promoting cell survival and proliferation, HIF has a dual function as a programmed cell death (apoptosis) inducer, inducing proapoptotic genes which can be responses present in hypoxic tissues (Pouyssegur et al., "Hypoxia signalling in cancer and approaches to enforce tumour regression" Nature 2006; 441, 437-443).

In addition, Keith and Simon have proposed that reducing the activity of HIF in stem cell could promote the differentiation thereof and thus decrease the repopulation of cells with malignant phenotype after radio- and chemotherapies (Keith, B and Simon, MC, (2007) Hypoxia-inducible factors, stem cells, and cancer. Cell 129: 465-72.). The stabilization of HIF promotes a resistance of the vasculature of the tumor to radiotherapy by the release of proangiogenic cytokines such as VEGF or apoptosis (Moeller BJ, Cao Y, Li CY, Dewhirst MW. Radiation activates HIF-1 to regulate vascular radiosensitivity in tumors: role of reoxygenation, free radicals, and stress granules. Cancer Cell. 2004 May;5(5):429-41.); Moeller BJ, Dreher MR, Rabbani ZN, Schroeder T, Cao Y, Li CY, Dewhirst MW, Pleiotropic effects of HIF-1 blockade on tumor radiosensitivity. Cancer Cell. 2005 Aug;8(2):99-110), therefore blocking the activation thereof has been considered highly desirable in certain pathologies by a number of investigators. Some reviews include the way in which HIF is activated and promotes tumor progression, (Rankin EB, Giaccia AJ. The role of hypoxia-inducible factors in tumorigenesis. Cell Death Differ. 2008 Apr;15(4):678-85. Epub 2008 Feb 15; Peinado H, Cano A. A hypoxic twist in metastasis. Nat Cell Biol. 2008 Mar;10(3):253-4; Bertout JA, Patel SA, Simon MC. The impact of 02 availability on human cancer. Nat Rev Cancer. 2008 Dec;8(12):967-75. For these reasons, HIF proteins have been considered as pharmacological targets and a number of strategies have been designed for the discovery of new drugs, however, numerous inhibitors do not adjust to the pharmacological requirements in toxicity profiles necessary for a subsequent approval of the drug by regulatory agencies. Even after more than 20 years of intensive searching, the search for new compounds or small molecules modulating the transcriptional activity and protein expression of HIF is still possible and recommendable. Thus, certain approaches were developed using HIF inhibitors alone or in combination with other drugs effective in angiogenic pathologies, as antitumor agents or even in regenerative medicine, as well as in the preconditioning of stem cells used in transplantation therapies.

The concept of tumor stem cell is arousing an increasing interest in scientists all over the world since cells of different tumors with stem cell characteristics have been isolated. It is known that hypoxic conditions promote molecular events having as a consequence a decrease of expression of the repair genes (Mismatch Repair genes, MMR) for the mutations occurring in DNA during the replication thereof in the S phase of the cell cycle (Mihaylova VT, et al., "Decreased expression of the DNA mismatch repair gene MIh1 under hypoxic stress in mammalian cells" Mol Cell Biol. 2003 May; 23(9):3265-3273.). Certain diseases such as hereditary nonpolyposis colon cancer (nPCC) are associated with a deficiency in these repair genes the consequence of which is the onset of instability in microsatellite sequences of DNA and which are detected in 95% of nPCC tumors or in 15% of sporadic colon tumors. Stem cells persist for long time periods and, therefore, under poor operating conditions of the repair systems for the genetic material, they would have more possibilities of accumulating mutations than other types of cells with a shorter half-life. The decrease of these MMR genes in stem cell under hypoxic conditions could entail an increase and accumulation of non-repaired mutations which could increase the possibilities of acquiring a malignant phenotype. Considering previously published scientific results, it is known that after repression, the expression of repair genes could be recovered by a histone deacetylase inhibitor known as Trichostatin A (TSA) (Cameron EE, et al., "Synergy of demethylation and histone deacetylase inhibition in the re-expression of genes silenced in cancer" Nat Genet. 1999 Jan; 21(1):103-107.). This drug is capable of increasing the degree of acetylation of the histones located in the promoting region of those genes, which confers to chromatin a conformation which is accessible for the transcription factors regulating these genes. The fact that a drug recovers genes which slow down or prevent cancer makes it a possible candidate for an antitumor agent. TSA is a drug which is currently in a preclinical phase for breast cancer treatment (Vigushin DM, et al., "Trichostatin A is a histone deacetylase inhibitor with potent antitumor activity against breast cancer in vivo" Clin Cancer Res. 2001 Apr; 7(4):971-6).

### HIF and Regenerative Medicine

The etiology of certain oncogenic processes is closely linked to the self-renewal and differentiation potential of stem cells (Clarke MF. "Neurobiology: at the root of brain cancer" Nature, 2004; 432, 281-2.) and therefore linked to the therapeutic potential of regenerative medicine. The processes of division, differentiation and functioning of stem cells are regulated by complex signals of their environment, including the availability of nutrients and oxygen. During embryonic development, as well as in the adult brain, physiological hypoxia has been linked to the proliferation and undifferentiation of different progenitor cells and stem cells (Studer L et al., "Enhanced proliferation, survival, and dopaminergic differentiation of CNS precursors in lowered oxygen." ., J Neurosci.,2000; 20(19), 7377-83; Morrison SJ, et al. "Culture in reduced levels of oxygen promotes clonogenic sympathoadrenal differentiation by isolated neural crest stem cells." J Neurosci. 2000; 20(19), 7370-6). According to recent results, neural stem cells are associated to highly angiogenic niches and it has been demonstrated that HIF is essential in the development of the central nervous system (Sharp F.R. and Bernaudin M, "HIF1 and oxygen sensing in the brain.", Nature Rev. 2004, 5, 437-448; Ryan E R, Lo J & Johnson, "HIF-1 alpha is required for solid tumor formation and embryonic vascularization." EMBO J., 1998; 17, 3005-3015; Ding and Schultz, "A role for chemistry in stem cell biology" Nature Biotech., 2004; 22, 833-840). Recent results also demonstrate the synergism of molecular hypoxia mechanisms with molecular undifferentiation factors, such as the transcription factors Notch and Oct-4, which mediate processes of cell differentiation in different tissues. The synergy between these two important cell signaling pathways with hypoxia leads to the modulation of the cell differentiation pathways mediated by these important undifferentiation markers (Gustafsson and et al., "Hypoxia requires notch signaling to maintain the undifferentiated cell state." Developmental Cell, 2005; 9, 617-628; Covello et al., "HIF-2alpha regulates Oct-4: effects of hypoxia on stem cell function, embryonic development, and tumor growth." Genes & Development 2006; 20, 557-570).

The following stem cell models have been used in the present invention:
- The C17.2 cell line, which is a cell line of multipotent neural precursors, which was derived from the external granular layer of newborn mouse cerebellum, and immortalized by transduction of the v-myc gene mediated by retrovirus (Snyder EY, et al., "Multipotent neural cell lines can engraft and participate in development of mouse cerebellum" Cell. 1992 Jan; 10(68), 33-51). This cell line provides homogeneous neural progenitors in an unlimited manner and is considered as a neural stem cell from the operative point of view (Parker MA, et al., "Expression profile of an operationally-defined neural stem cell clone. 1", Exp Neurol. 2005 Aug, 194(2), 320-32). When these cells are transplanted in a newborn mouse cerebellum they seem to participate in the normal development of the cerebellum. These cells are provided with an extraordinary plasticity and given a brain damage they are capable of migrating towards the affected area, differentiating to neurons or glia, connecting and receiving connections of other nerve cells, as well as being included in the normal cytoarchitecture of the damaged tissue in a non-tumorigenic manner (Snyder et al., "Multipotent neural cell lines can engraft and participate in development of mouse cerebellum" Cell. 1992 Jan; 10(68), 33-51). For these reasons, their possible usefulness as cell therapy and transplantation for neural regeneration in neurodegenerative diseases has been suggested (Wei-Guo Liu, et al. "Dopaminergic neuroprotection by nurturing-expressing c17.2 neural stem cells in a rat model of Parkinson disease" Parkinsonism and Related Disorders 2007; 13, 77-88; Snyder EY, et al., "Multipotent neural precursors can differentiate toward replacement of neurons undergoing targeted apoptotic degeneration in adult mouse neocortex." Proc Natl Acad Sci U S A. 1997 Oct; 14, 94(21), 11663-8). C17.2 do not only not seem to evolve towards a tumorigenic phenotype once transplanted, but also seem to show a harassment or persecutory behavior towards tumor cells. When C17.2 were implanted in the brain of mice in which a glioma had been developed, these cells migrated towards the invading edge of the tumor, surrounding it. They even have the capacity to surround small islands of malignant cells escaping from the main tumor mass (Aboody KS, et al. "Neural stem cells display extensive tropism for pathology in adult brain: evidence from intracranial gliomas.", Proc Natl Acad Sci USA 2000 Nov 7; 97(23), 12393-5). The authors of the present invention consider these cells as a possible tool for gene therapy studies, since they can release genes with therapeutic potential which act on the tumor mass. C17.2 have also been assayed in spinal cord regeneration models (Teng YD, "Functional recovery following traumatic spinal cord injury mediated by a unique polymer scaffold seeded with neural stem cells.", Proc Natl Acad Sci USA 2002 Mar 5; 99(5), 3024-9). In animals in which these cells were implanted, the functional recovery of the affected limbs was observed, possibly due to the partial regeneration of immersed nerve fibers in the injured area. Ourednik *et al.* show how these cells contribute to reestablishing dopaminergic neuron functions in an animal Parkinson's disease model (Ourednik J et al., "Neural stem cells display an inherent mechanism for rescuing dysfunctional neurons" Nat Biotechnol. 2002 Nov 20; 11, 1103-10). In this case, their potential in gene therapy, as well as their possible role as a neuroprotector or in the possible reestablishment of functions in damaged neurons, is again shown.
- Primary culture of adult neural progenitors (NPCs) from the ependymal and subependymal area of the central nervous system (CNS). The spinal cord can be regenerated in certain adult vertebrates, as is the case of fish and in the urodele amphibian, however, most vertebrates have lost this potential during development. In the spinal cord in urodele, the axons not only grow and reconnect again with the structures that they innervated before the injury, but are also capable of replacing lost neurons and glial cells. The source of this regeneration seems to be a population of ependymal cells aligned in the central channel and extending to the surface of the *pia mater* (Egar M, Singer M "The role of ependyma in spinal cord regeneration in the urodele, Triturus" Exp Neurol 1972; 37, 422-430; Benraiss A, et al. "Adenoviral brain-derived neurotrophic factor induces both neostriatal and olfactory neuronal recruitment from endogenous progenitor cells in the adult forebrain." J Neurosci 2001; 21, 6718-6731; Echeverri K. and Tanaka EM "Ectoderm to mesoderm lineage switching during axolotl tail regeneration." Science 2002; 298, 1993-1996; Ferretti et al." Changes in spinal cord regenerative ability through phylogenesis and development: lessons to be learnt.", Dev Dyn 2003; 226, 245-256.). In several central nervous system injury models, the existence of ependymal cell proliferation and differentiation has been described, which suggests the existence of an endogenous source with regenerative power (Johansson CB, et al. "Identification of a neural stem cell in the adult mammalian central nervous system.", Cell 1999; 96, 25-34). This process of neurogenesis has been described in both models, both in multiple sclerosis, which presents an inflammatory process and which results in axonal degeneration and demyelination, and in the trauma injury of the spinal cord (Danilov et al., "Neurogenesis in the adult spinal cord in an experimental model of multiple sclerosis.". European J Neuroscience 2006; 23, 394-400; Ke Y, et al. "Early response of endogenous adult neural progenitor cells to acute spinal cord injury in mice." Stem Cells 2006. Published online). However, despite the fact that the process of neurogenesis occurs, these neural precursors are insufficient to replace the degenerated tissue and therefore to recover the lost function. In adult mammals, the ependymal cells aligned in the central channel in the spinal cord can form neurospheres (colonies formed by neural stem cells) *in vitro* (Danilov A, et al. "Neurogenesis in the adult spinal cord in an experimental model of multiple sclerosis." European J Neuroscience 2006; 23, 394 - 400). The adult stem cells coming from the subventricular area of the brain, can proliferate *in vitro* as undifferentiated spheroids and differentiate into neurons, astrocytes or oligodendrocytes (Weiss S, et al. "Multipotent CNS stem cells are present in the adult mammalian spinal cord and ventricular neuroaxis" J Neuroscience 1996; 16, 7599-7609), being an optimal tool for the reconstruction of the injured medullary tissue. However, the *in vivo* transplantation of these stem cells has not given positive results. These cells either remain undifferentiated or appear with astroglial phenotype, contributing to the glial scar (Ricci-Vitiani L, et al. "Influence of local environment on the differentiation of neural stem cells engrafted onto the injured spinal cord." Neurol Res. 2006; 28(5), 488-92). Therefore, the primary culture of adult neural progenitors (NPCs), and specifically those of the ependymal and subependymal area of the central nervous system (CNS) are a suitable model for the *in vitro* study of the behavior of stem cells which are physiologically in a hypoxic environment. NPCs have self-renewal and multipotentiality capacity, they can differentiate into neurons, astrocytes or oligodendrocytes. During recent years, stem cells and/or adult NPCs have proved to be a very attractive source in the processes of repair of neurodegenerative diseases or in trauma injuries of the CNS. The transplantation of neural precursors can be integrated and reconnected along the axonal pathways already existing in the spinal cord after an acute injury (Vroemen, et al. "Adult neural progenitor cell grafts survive after acute spinal cord injury and integrate along axonal pathways" Eur J Neurosci. 2003; 18(4), 743-51). Akiyama *et al.* show that the transplantation of neural precursors derived from adult human brain can facilitate processes of remyelination in demyelinated spinal cord (Akiyama Y, et al. "Transplantation of clonal neural precursor cells derived from adult human brain establishes functional peripheral myelin in the rat spinal cord" Exp Neurol. 2001, 167(1), 27-39).
- Bone marrow mesenchymal stem cells (BMMSCs): they are the cells forming part of the bone marrow (BM) stroma, and can differentiate *in vitro* into various cell types such as chondrocytes, osteocytes and adipocytes, without inducing tumors, they have furthermore proved to have the regenerative capacity for various tissues and are being used in tissue cell therapy. In recent years the interest in BMMSCs has increased, various studies suggested that these cells could differentiate *in vitro* into cardiomyocytes, however, the need to use the teratogenic compound 5-azacytidine as an inducing agent limits the clinical applicability of this strategy (Laflamme M.A. and Murry M.A. "Regenerating the heart" Nature Biotechnology, 2005; 23:845-856.). In addition, there is evidence indicating that the direct injection of non-stimulated BMMSCs into the heart in rats and pigs in a post-infarction state improves ventricular function (Ma J. et al., "Time course of myocardial stromal cell-derived factor 1 expression and beneficial effects of intravenously administered bone marrow stem cells in rats with experimental myocardial infarction" Basic Res Cardiol. 2005; 100:217-223). A new theory coming from the studies by Gnecchi M *et al.* (Gnecchi M. et al. "Paracrine action accounts for marked protection of ischemic heart by Akt-modified mesenchymal stem cells" Nature Medicine. 2005; 111:367-368) involves the secretion of paracrine cytoprotective factors derived from mesenchymal stem cells, such that when the medium produced upon subjecting these BMMSCs to hypoxic conditions is added on rat ventricular cardiomyocytes cultured under hypoxia, the apoptosis of these cardiomyocytes decreases. These results involve a "paracrine hypothesis" theory for the action of BMMSCs, involved in tissue protection and repair; instead of being a consequence of the cardiomyogenic differentiation of BMMSCs. The isolation of these secreted factors can have a high therapeutic application for preventing damage in ischemic tissue. This line of human adult stem cells is obtained from the Inbiomed Foundation, San Sebastián, Spain (www.inbiomed.com), and specifically from its recently created cell bank Inbiobank (www.inbiobank.org). BMMSCs have a CD29+, CD73+, CD105+, CD166+, CD45- and CD31- typical phenotype (Pittenger MF et al., "Multilineage potential of adult human mesenchymal stem cells" Science 1999; 284: 143-147; Javazon EH et al., "Mesenchymal stem cells: paradoxes of passaging", Exp Hematol 2004 May; 32(5):414-25). BMMSCs are seeded at a density of 2000 cells/cm², when they reach a 90% confluence they are subcultured again by means of trypsinization. The culture medium used is Low-Glucose DMEM supplemented with 10% fetal bovine serum.
- Dental pulp cells are the specific mesenchymal stem cells which are located in postnatal human dental pulp tissues, and are responsible for the dentin-pulp complex, which is made up of a mineralized matrix formed by tubules aligned with odontoblasts, and fibrous tissue containing blood vessels and forming the physiological structure of teeth (Gronthos S, et al. "Postnatal human dental pulp stem cells (DPSCs) in vitro and in vivo" Proc Natl Acad Sci U S A. 2000 Dec 5; 97(25):13625-30.; Gronthos S et al. "Stem cell properties of human dental pulp stem cells" J Dent Res 2002 Aug; 81 (8):531-5). These cells can differentiate *in vitro* into odontoblasts, adipocytes and cells with neural morphology. The cell response to hypoxia and reoxygenation in the brain and in the heart has been extensively studied as relevant models for the pathogenesis of ischemic damage; therefore studying the response and activity of dental pulp stem cells under conditions of hypoxia (Kim S, Edwall L, Trowbridge H, Chien S. "Effects of local anesthetics on pulpal blood flow in dogs", J Dent Res. 1984; 63(5), 650-2; Kim S, et al., "Functional alterations in pulpal microcirculation in response to various dental procedures and materials" Proc Finn Dent Soc. 1992; 88, Suppl 1, 65-71) and reoxygenation *in vitro,* with the advantage of using HIF modulators, would constitute a relevant model for the pathogenesis of ischemic damage *in situ.*

### Angiogenesis Inhibitors

There are compounds which protect inhibitors modulating the transcriptional activity of HIF1 by means of different modes of action, which affect cell signaling pathways and/or structural or epigenetic phenomena, some of which as detailed in Table 1 and which can be found extensively referenced and described in the recent review on angiogenesis by J. Folkman ("Angiogenesis: an organizing principle for drug discovery?" Nat. Rev Drug Discovery, 2007, 6, 273-286).

**Table 1: List of the main inhibitors of the transcriptional activity of HIF (see review by J. Folkman "Angiogenesis: an organizing principle for drug discovery?" Nat. Rev Drug Discovery, 2007; 6, 273-286)**

| **Compound** | **Pharmacological target** |
|---|---|
| BAY 43-9006 | RAF Kinase |
| CCI-779 | mTOR |
| Celebrex | COX2 |
| ZD-1839 (Iressa) | EGFR tyrosine kinase |
| 2-ME2 | Microtubule Polymerization |
| 17-AAG | HSP90 |
| FK228 | HDAC |
| SAHA | HDAC |

In the state of the art there are abundant patent documents which describe and claim various compounds and the pharmaceutical compositions containing them which are HIF factor inhibitors for the treatment or prevention of pathologies related to hypoxia, considered as angiogenesis inhibitors. The following will be mentioned among them:
Patent application WO2007/022412 A2 describes a method for treating unwanted angiogenesis in the tissue of a subject having or at risk of having an angiogenic disease or disorder, by means of using a combination therapy which comprises administering a hypoxia-inducible transcription factor inhibitor and a second antiangiogenic agent such as for example: vascular endothelial growth factor (VEGF), angiopoietin 1, interleukin 8 (IL-8) or an angiostatin, thrombospondin or tumstatin direct angiogenesis inhibitor.
Document WO 2005/089490 A2 describes and claims compounds and methods for inhibiting HIF-inducible hypoxia in a patient, which comprises administering to said patient an effective amount of one of the inhibitor compounds of said invention. The claimed method for treating cancer consists of administering one of the compounds together with therapy consisting of radiation or chemotherapy.
Patent application WO 2006/066775 A1 relates to the use of diketodithiopiperazine antibiotics, such as chaetocin and gliotoxin, for the preparation of medicaments for the treatment of pathologies in which the inhibition of HIF 1α to p300 is necessary.
Document WO 02/36574 A1 relates to a geldanamycin derivative used to eliminate a cancer in a cell or to treat a cancer in a patient both *in vitro* and *in vivo* by administering a sufficient amount of the geldanamycin derivative to inhibit HIF 1α.
Document US 2006003961 A1 relates to the interaction of OS-9 with both HIF1 and HIF1α prolyl hydroxylase. The loss of OS-9 function increases the levels of HIF1α protein and the transcription of HIF1 under non-hypoxic conditions. These data indicate that OS-9 is an essential component of the multiprotein complex regulating the levels of HIF1α protein in an O₂-dependent manner. Agents modulating this complex, as well as methods for identifying such agents are also described.
Document WO 2004/091648 A1 relates to methods and pharmaceutical compositions for inhibiting the expression of HIF1 and the genes regulated by HIF1, angiogenesis, tumor growth or the progression/metastasis of a tumor comprising contacting the tumor cells or tissue with a composition containing 3(5'hydroxymethyl 2'furyl) 1-benzyl-indazole or one of the novel derivatives thereof. Subsequently, the same applicant, in document WO 2005/030121 A2 describes the inhibition of the growth of a tumor *in vivo* using certain heterocyclic compounds except 3(5'hydroxymethyl 2'furyl) 1-benzyl-indazole to inhibit the inducible hypoxia expression factor in tumor cells or tissues.
Documents WO 2004/087066 A2 and WO 2007/025169 A2, both belonging to the same applicant, use pharmaceutical compositions comprising, in the case of the first document, compounds derived from 2,2-dimethylbenzopyran, optionally in combination with other cytostatic agents for the treatment or the prevention of pathologies related to HIF1 factor-inducible hypoxia and, in the case of the second document, pharmaceutical compositions comprising aryl-sulfonamide derivatives for lung or bladder cancer, generated by the same mechanism.

Thus, these and other patent documents disclose the inhibition of the activity of HIF and even its relationship with the modulation of angiogenesis. However, at no time does any of these documents refer to compounds which may be related to the compounds represented by the molecule of general formula (I) as an inhibitor of the activity of HIF which is the object of the present invention.

The document "Hypoxia inducible factor-1: a novel target for cancer therapy", (Belozerov Vladimir E. and Van Meir Edwin G., published in Anti-Cancer Drugs, 16(9): 901-909, 2005) shows the hypoxia-inducible transcription factor (HIF 1) as a modulator of the large number of molecular events necessary for the adaptation of the tumor cells to hypoxia. This is why HIF 1 becomes an attractive target for the development of anticancer drugs. This publication mentions some of the small molecules which inhibit the HIF1 function lately developed. These molecules break the HIF1 signaling pathway through a large variety of mechanisms, including the inhibition of the synthesis of the HIF1α protein, stabilization, nuclear translocation and transactivation of the target genes. The publication discloses the relationship between HIF and cancer processes and the use of molecules which inhibit HIF for the development of anticancer drugs, however, it does not mention any compound of general formula (I).

Only two compounds the structure of which complies with general formula (I), in both cases without having any biological activity, have been described. 2-4-(methoxy)phenyl)-2-oxoethyl 3-(2,4-(dinitro)benzamido)benzoate is mentioned as formula B1C11 on page 47 of the document US 2006223812 A1 relates to the use of compounds capable for inhibiting the formation of protein aggregates and of depolymerizing protein aggregates for preparing a pharmaceutical composition for the treatment of neurodegenerative conditions such as Alzheimer's disease and 2-4-(nitro)phenyl)-2-oxoethyl 3-(4-bromo)benzamido)benzoate appears in the document (Kim, S. Y. et al., "Pharmacophore-based virtual screening: the discovery of novel methionyl-tRNA synthetase inhibitors", Bioorg. Med. Chem. Lett. 2006, 16, 4898-4907) which describes performing a virtual selection from a chemical database with 508,143 compounds to search for new methionyl-tRNA synthetase inhibitors. None of these dos compounds is related to the modulation and/or inhibition of the activity of HIF.

Taking into account the foregoing, the design of synthetic molecules aimed at the recognition of molecular mechanisms having the capacity to modulate the transcription of biologically relevant genes is considered as one of the greatest challenges of biotechnology, at the point of convergence between chemistry and biology and with an enormous potential in therapeutics. Therapy, especially in cancer, is increasingly based on the use of compositions which are mixtures of active compounds, with different modes of action and non-accumulative toxicities.

### Brief Description of the Drawing

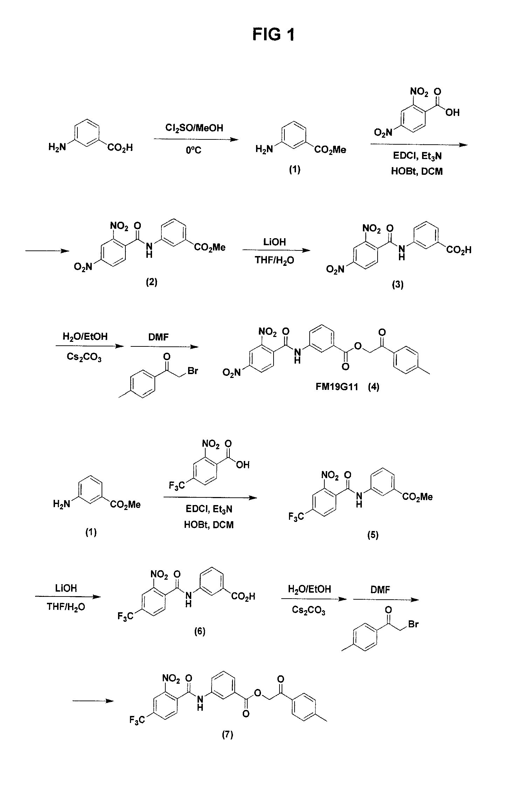

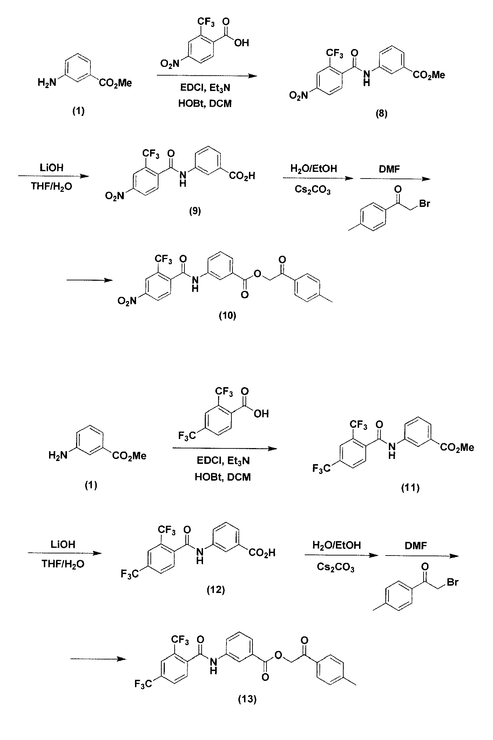

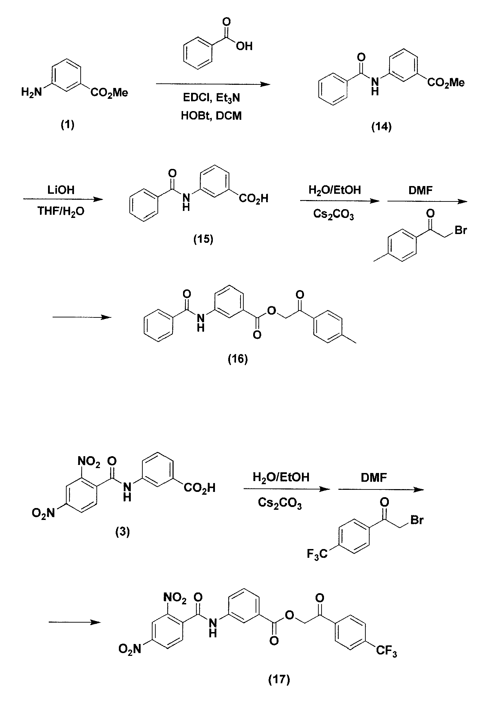

Figure 1: Chemical synthesis of the FM19G11 compound and analogs

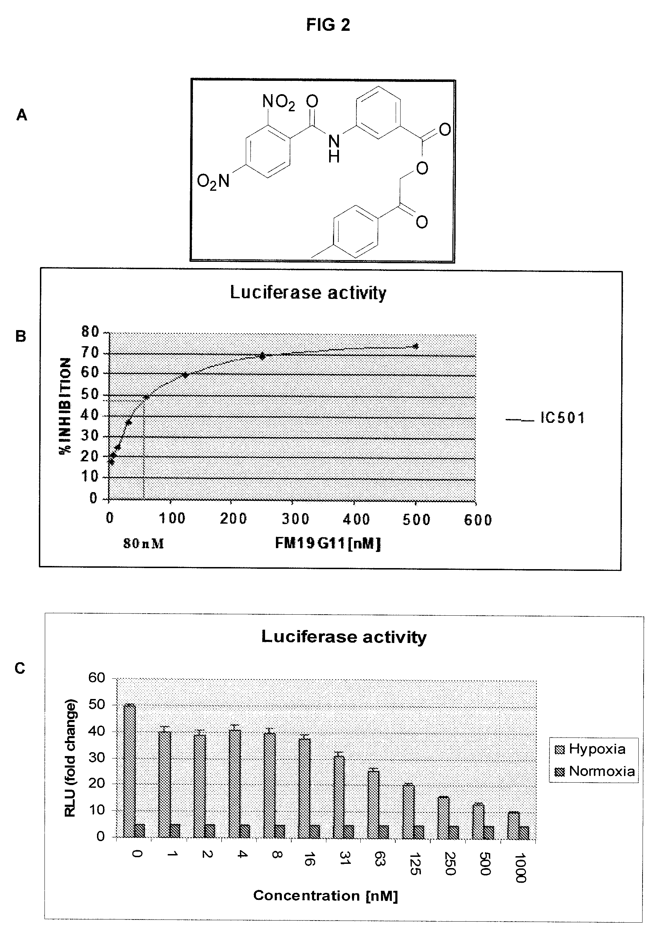

Figure 2: FM19G11, a novel inhibitor of the transcriptional activity of HIF. A) Chemical structure of FM19G11; B) Study of the transcriptional activity of HIF using the luciferase reporter gene, activated in response to the HIF proteins by binding to their HRE response elements, present in the promoter region thereof. The culture of HeLa cells, which constitutively overexpress the luciferase reporter gene fused to 9 HRE repeats, under hypoxic (1% O₂, blue bars) or normoxic (20% O₂, red bars) conditions, was treated with increasing concentrations of FM19G11 (0-1 µM) for 6 hours. The luciferase activity units are represented in absolute values; C) Calculation of the inhibition of the transcriptional activity of HIF from the luciferase activity assay shown in B: 100-(((RLU_{Hx+FM19G11}-RLU_{Hx+DMSO})*100/RLU_{Hx}-RLU_{Hx+DMSO}))). The experiments were performed in triplicate.

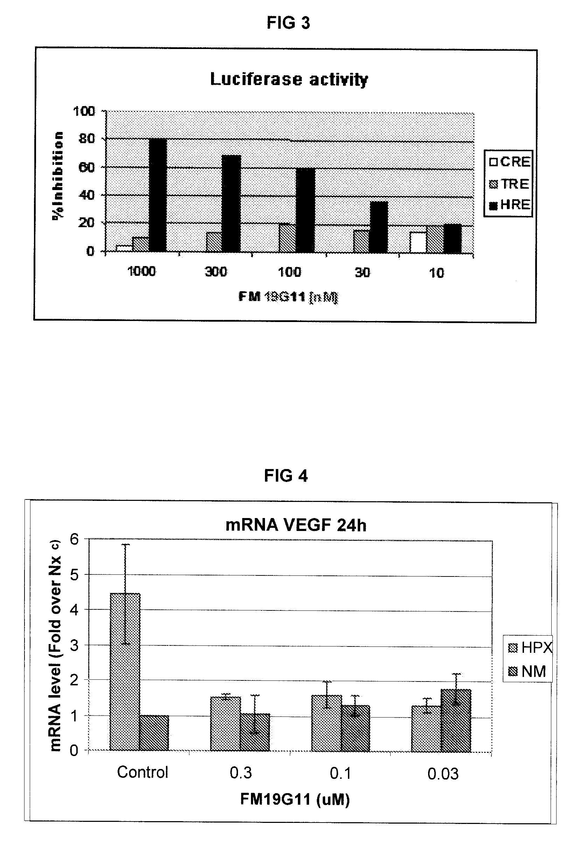

Figure 3: Study of specificity of FM19G11 in the regulation of the transcriptional activity of HIF. HeLa cells which constitutively overexpress the luciferase reporter gene, fused in its promoter area to the sequences which respond to the HIF proteins (HRE), or to the complex formed by c-jun/c-fos (TRE) or to the complex formed by ATF2/JunB (CRE), were cultured in the presence of each of their stimuli for 6 hours: hypoxia (1% O₂), for HRE; co-expression of c-fos and c-jun for TRE; co-expression of ATF2 and JunB for CRE. The activity of FM19G11, at increasing concentrations (0-1 µM), in the inhibition of the transcriptional activity was determined by measuring the luciferase activity and applying the following formula: 100-(((RLU_{Hx+FM19G11}-RLU_{Hx+DMSO})*100/RLU_{Hx}-RLU_{Hx+DMSO}))). The experiments were performed in triplicate.
Figure 4: Real Time PCR for the quantification of the expression levels of the messenger RNA of VEGF in HeLA cells. HeLa cells were cultured under hypoxic (1% 0₂, HPX) or under normoxic (20% O₂, NM) conditions for 24 hours in the presence of increasing concentrations of FM19G11 (0.03-0.3 µM) or of equivalent volumes of the carrier (DMSO, Control). The changes of expression due to changes in the amount of total RNA were corrected by the constitutive expression of the GAPDH gene. The value of the VEGF/GAPDH expression ratio in each of the samples was relativized with respect to the expression level obtained in control normoxia (=1). The experiments were performed in triplicate.

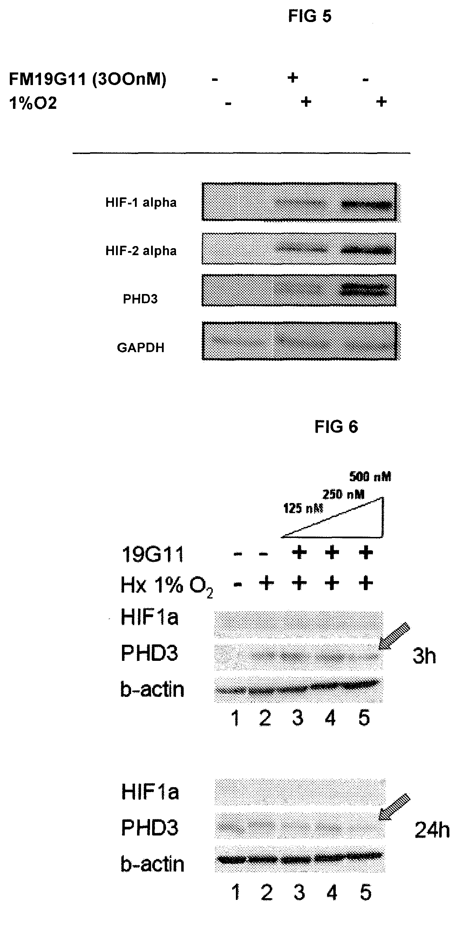

Figure 5: Analysis of expression of proteins by Western blotting using antibodies which recognize the two alpha isoforms of HIF and PHD3 (as a positive control, since it is a target gene of HIF1α), in HeLa 9XHRE cells. GAPDH was included as a protein loading control. The first lane corresponds to untreated cells. The second lane corresponds to cells treated with FM19G11 under hypoxic conditions, the third lane corresponds to cells cultured in hypoxia. The experiments were performed in triplicate. The stimulation time was 9 hours. An experiment representative of three independently performed experiments is shown.
Figure 6: Analysis of expression of proteins by Western blotting using antibodies which recognize HIF1α and PHD3 (as a positive control since it is a target gene of HIF1α), in C17.2 cells. B-ACTIN was included as a protein loading control. The first lane corresponds to untreated cells. The second lane corresponds to cells cultured under hypoxic conditions, the third, fourth and fifth lanes correspond to cells treated with increasing concentrations of FM19G11 (125, 250, 500 respectively) and cultured under hypoxic conditions. The experiments were performed in triplicate. The stimulation times were 3 and 24 hours. The marking with a red arrow indicates the considerable decrease of the expression of PHD3 (target gene of HIF). An experiment representative of three independently performed experiments is shown.

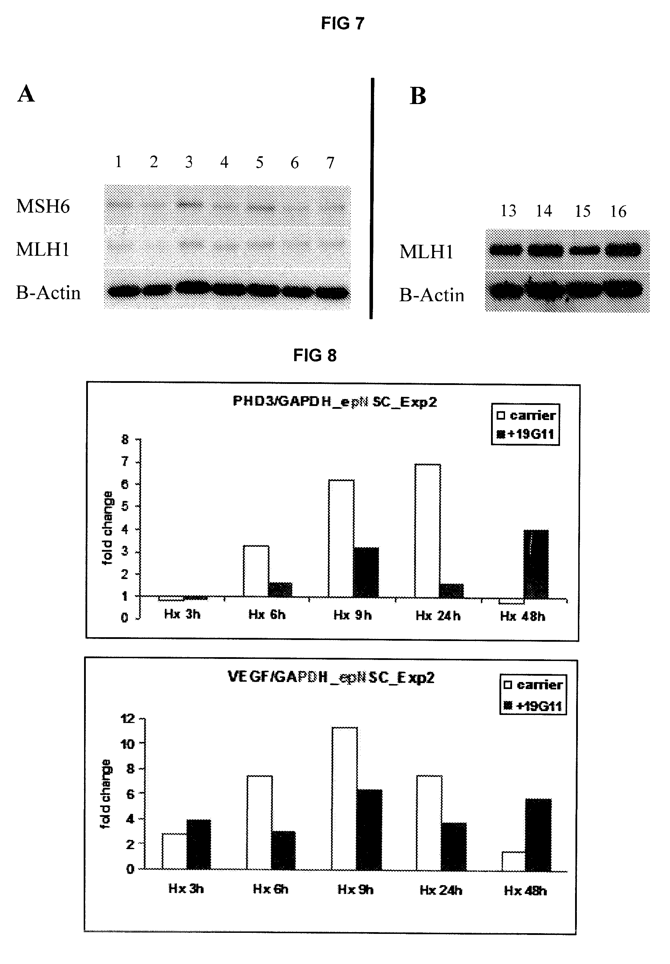

Figure 7: Western blotting performed with nuclear protein extracts of mesenchymal stem cells cultured in normoxia or hypoxia in the presence of FM19G11 (500 nM). The monoclonal anti-MLH1 and anti-MSH6 antibodies were used in the analysis. A- Results obtained in bone marrow mesenchymal stem cell. 1-Nx 3h; 2-Hx 3h; 3-Hx 3h+19G11; 4-Hx 18h; 5-Hx 18h+19G11;6- Hx 24h; 7-Hx 24h+19G11 B-Results obtained in dental pulp mesenchymal stem cell. 13-Nx 72h; 14-Nx 72h+19G11; 15-Hx 72h; 16-Hx 72h+19G11. The experiments were performed in triplicate. The stimulation time was 3 hours. An experiment representative of three independently performed experiments is shown.
Figure 8: Real Time PCR for the quantification of the expression levels of the messenger RNA of PHD3 and VEGF in epNPCs. The epNPCs were cultured under hypoxic conditions (1% O₂), in the presence of 500 nM FM19G11 or equivalent volumes of the carrier (DMSO), from 0 (baseline condition, 1) to 48 hours. The changes of expression due to changes in the amount of total RNA were corrected by the constitutive expression of the GAPDH gene. The value of the PHD3 or VEGF/GAPDH expression ratio in each of the samples was relativized with respect to the baseline expression level (normoxia or hypoxia at time 0= 1). The experiments were performed in triplicate.

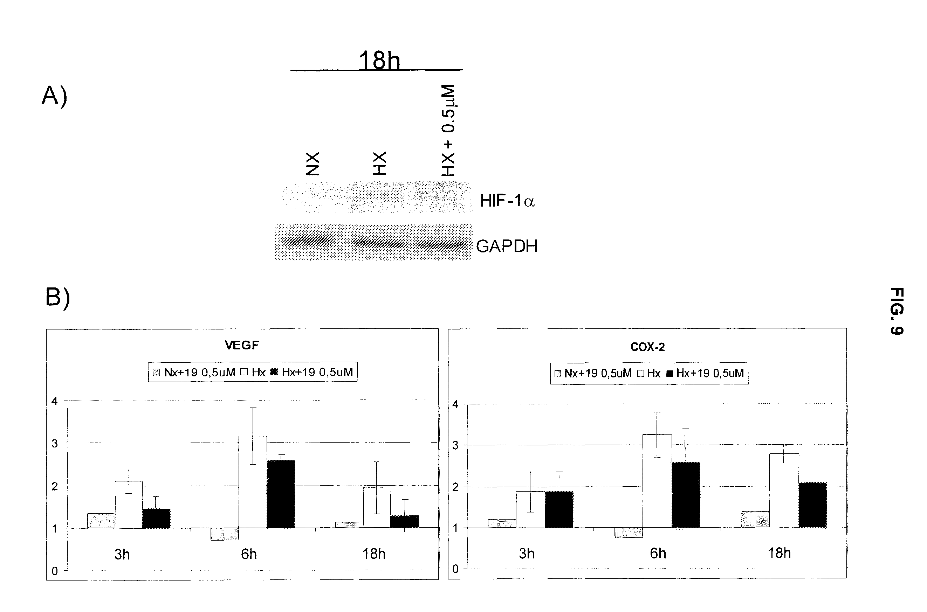

Figure 9: Effect of the compound FM19G11 on the expression of HIF-1 and its target genes VEGF and COX-2 in human dental pulp stem cells.

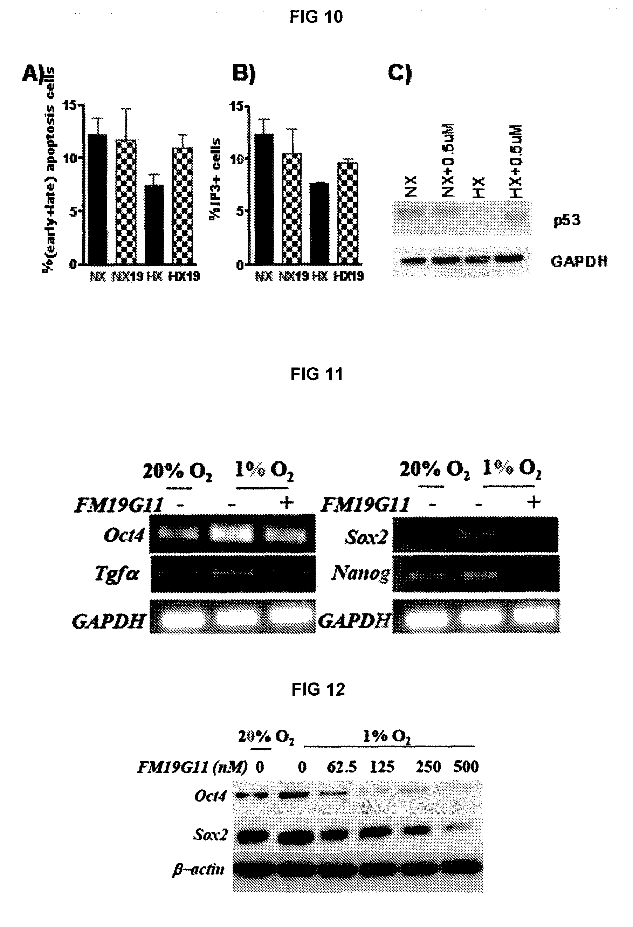

Figure 10: Effect of the compound FM19G11 on apoptosis and viability in human dental pulp stem cells.
Figure 11: Semiquantitative RT-PCR for the study of the expression of the messenger RNA of the undifferentiated state markers Oct3/4, Sox2 and their target genes TGFα and Nanog respectively. The epNPCs were cultured under normoxic (20% O₂) or hypoxic (1% O₂) conditions in the presence (+) of 500 nM FM19G11 or its carrier, DMSO (-) for 48 hours. The expression of the constitutive GAPDH gene shows there are no significant changes in the amount of sample assayed in all the conditions. The experiments were performed in triplicate.
Figure 12: Western Blot for the study of the protein expression levels of Oct3/4, Sox2 and ß-actin (loading control) of epNPCs cultured under normoxic (20% O₂) or hypoxic (1% O₂) conditions, for 48 hours, in the presence of increasing dose of FM19G11. The experiments were performed in triplicate.

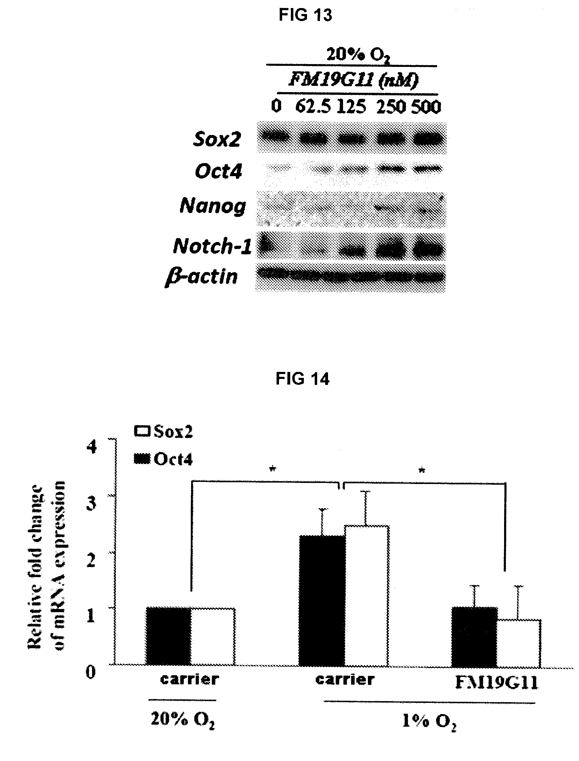

Figure 13: Western Blot for the study of the protein expression levels of Sox2, Oct3/4, Nanog, Notch-1 and ß-actin (loading control) of epNPCs cultured under normoxic conditions (20% O₂) for 48 hours in the presence of increasing doses of FM19G11. The experiments were performed in triplicate.
Figure 14: Real Time PCR for the quantification of the expression levels of the messenger RNA of Sox2 and OCt3/4 in hESCs. Colonies of hESCs, cultured on Matrigel, were treated under normoxic (20% O₂) or hypoxic (1% O₂) conditions, in the presence of 500 nM FM19G11 or equivalent volumes of its carrier (DMSO) for 48 hours. The changes of expression due to changes in the amount of total RNA were corrected by the constitutive expression of the GAPDH gene. The value of the Sox2 or Oct3/4/GAPDH expression ratio in each of the samples was relativized with respect to the baseline expression level (normoxia-carrier= 1). The experiments were performed in triplicate.

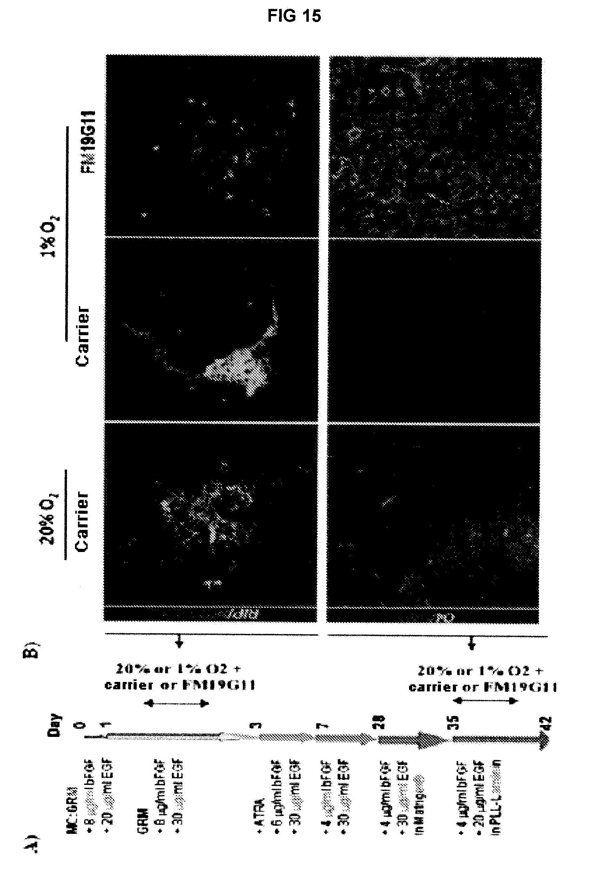

Figure 15: Targeted differentiation of epNPCs into oligodendrocytes. A) Scheme of the differentiation protocol. The epNPCs were treated with FM19G11 or its carrier (DMSO) in early stages of the process of differentiation, between days 1-3, and at the end of the protocol, between days 35-38, under normoxic (20% O₂) or hypoxic (1 % O₂) conditions. The expression of the markers for oligodendrocytes, NG2, RIP and 04 was determined in all the conditions by immunohistochemistry.

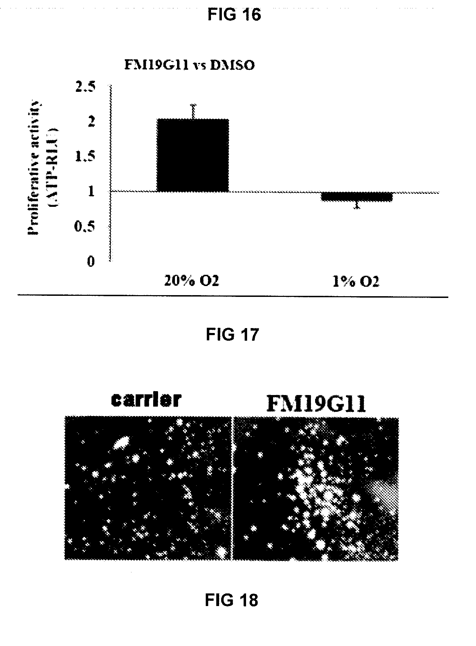

Figure 16: Quantification of the proliferative activity of epNPCs in the presence of FM19G11. epNPCs were cultured in an identical number in the presence of 500 nM FM19G11 or its carrier for 3 days under normoxic (20% O₂) or hypoxic (1% O₂) conditions. The proliferative activity was determined by quantifying the amount of ATP produced as an indication of the metabolic activity of the cell culture. The results obtained in the presence of FM19G11 were relativized with respect to the values obtained in the presence of the carrier, DMSO (=1).
Figure 17: Effect of FM19G11 on the formation/amplification of neurospheres from a culture of epNPCs. Phase-contrast photograph of a culture of epNPCs (16,000 cells) in the presence of 500 nM FM19G11 or its carrier (DMSO) for 48 hours.
Figure 18: Toxicity of FM19G11 against a cell panel. The cytotoxicity studies were performed in the HeLa 9x-HRE-Luc, PRC3, HeLa, MCF-7 and MDA-MB 435-S cell lines. The cell lines were cultured in DMEM medium supplemented with 10% inactivated fetal bovine serum, penicillin (50 IU/ml), streptomycin (50 µg/ml) (Invitrogen-Life Technologies, Inc., Carlsbad, CA) in the presence of increasing concentrations of FM19G11 (0-30 µM) for 72 hours under normoxic conditions. The percentage of viability of the culture was determined using the commercial kit Cell Titer 96® AQueous Non-Radioactive Cell Proliferation Assay (Promega Corporation).

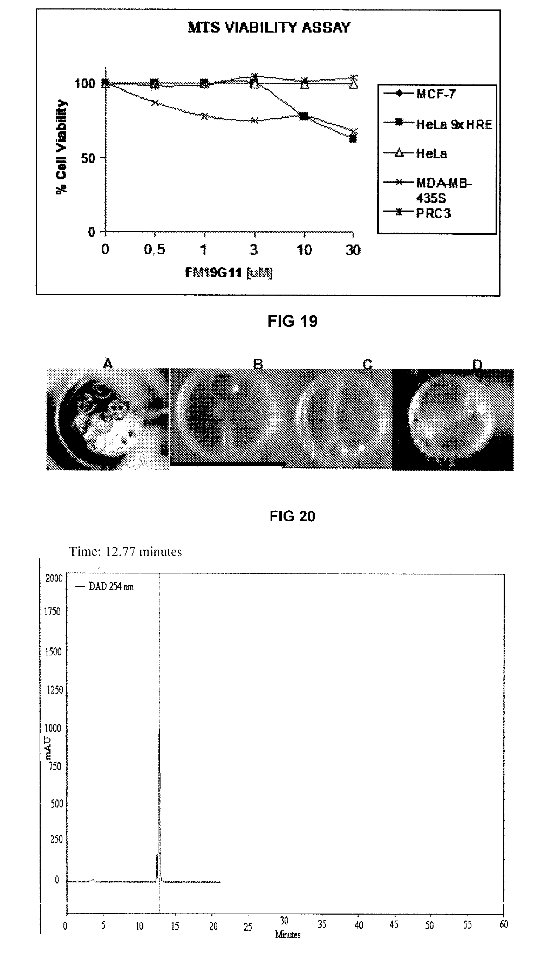

Figure 19: Assay of toxicity on fish (medaka) embryos of the compound FM19G11
Figure 20: Analytical HPLC chromatogram of the compound FM19G11

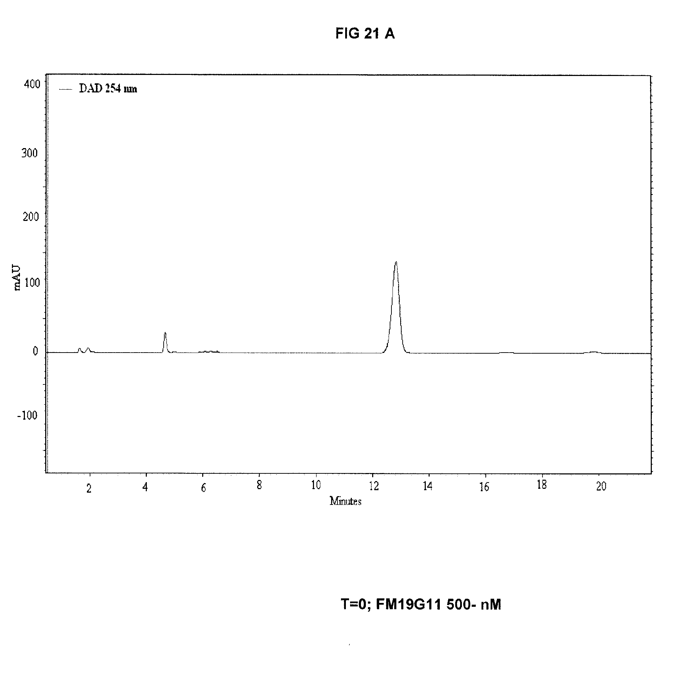

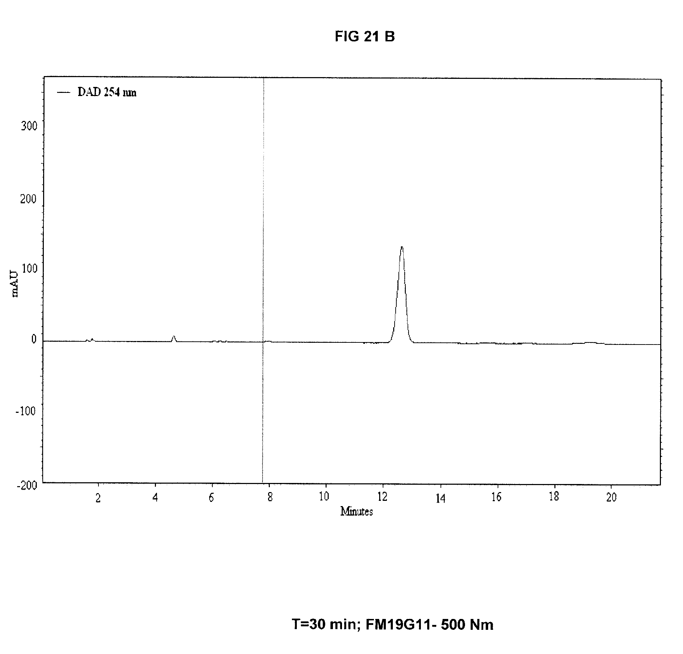

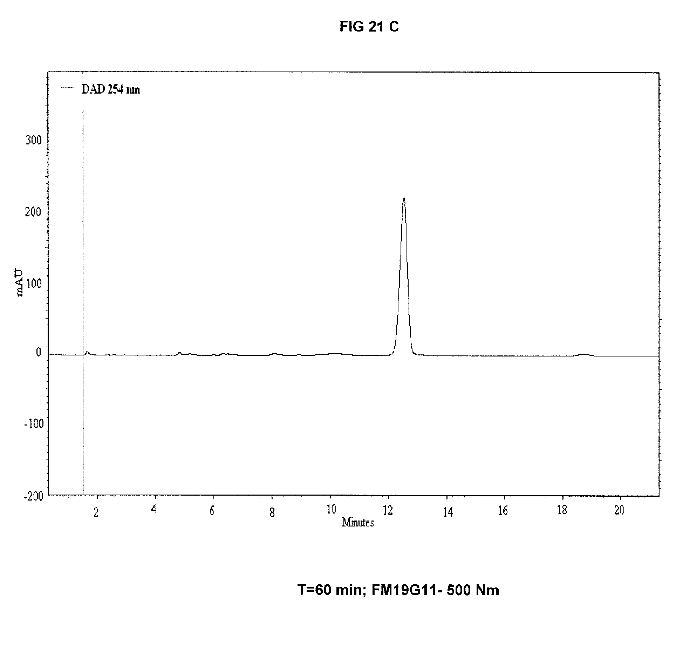

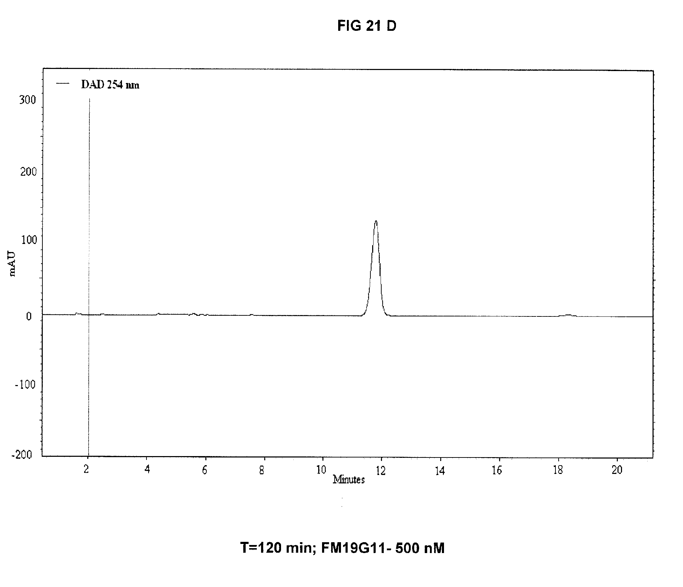

Figure 21: Chromatograms of the incubated extract of FM19G11 in bovine serum at different times.

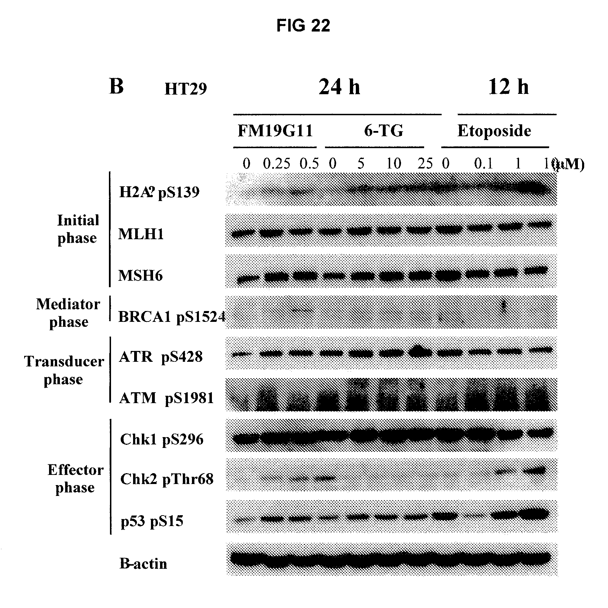

Figure 22: FM19G11 triggers a DNA damage response in human colon cancer cells (HT29). An increase in the activation by greater phosphorylation of kinases included in the key signaling pathways in DNA damage response, specifically the pathway known as ATM/ATR, that of DNA damage repair (MMR, BRCA1, H2AX), as well as at the points of control of the cell cycle (CHK1, CHK2), were detected in the HT29 tumor cells treated with FM19G11 (0.5 µM). The treatment with 6-TG (5, 10, 25 µM) or etoposide (0.1, 1, 10 µM) was included as a control as well as for the comparison of proteins which are activated in response to damage in the single strand (ATR-CHK1) or double strand (ATM-CHK2) of DNA respectively.

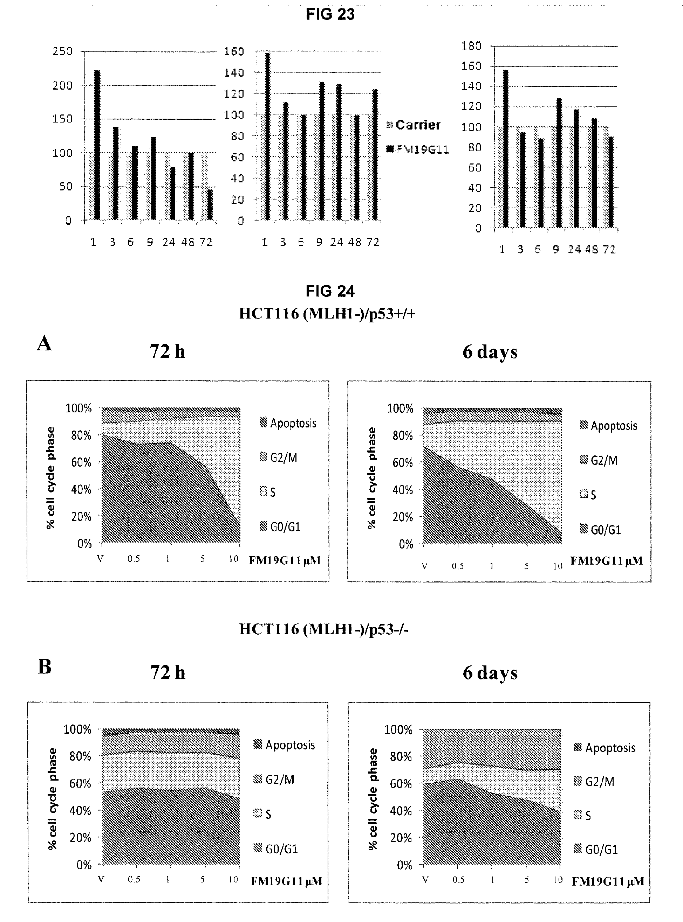

Figure 23. Kinetic studies show that a quick activation of ATR, associated to damage in the single strand of DNA in both HCT116 cell lines. After an hour of treatment with 0.5 uM FM19G11, an increase was detected in the phosphorylation or activation of ATR and p53 in p53 cells in HCT116/p53+/+, as well as of ATR in HCT116 p53-/- cells. The results shown above were obtained after subjecting a representative western blotting experiment to densitometric analysis, although similar results were obtained in three independent experiments.
Figure 24. Effect of p53 on the FM19G11-induced cycle arrest. A- The HCT116/p53+/+ cells and B-HCT116/p53-/- cells were exposed to different concentrations of FM19G11 (0.5, 1, 5, 10 µM) or carrier for 3 or 6 days to analyze the distribution of cells in the different cell cycle phases by means of flow cytometry. The graphs show the effects caused by different concentrations of FM19G11 on the distribution of percentages of cells in each cell cycle phase. The results shown are representative of an experiment, although very similar percentages were obtained in three independent experiments.

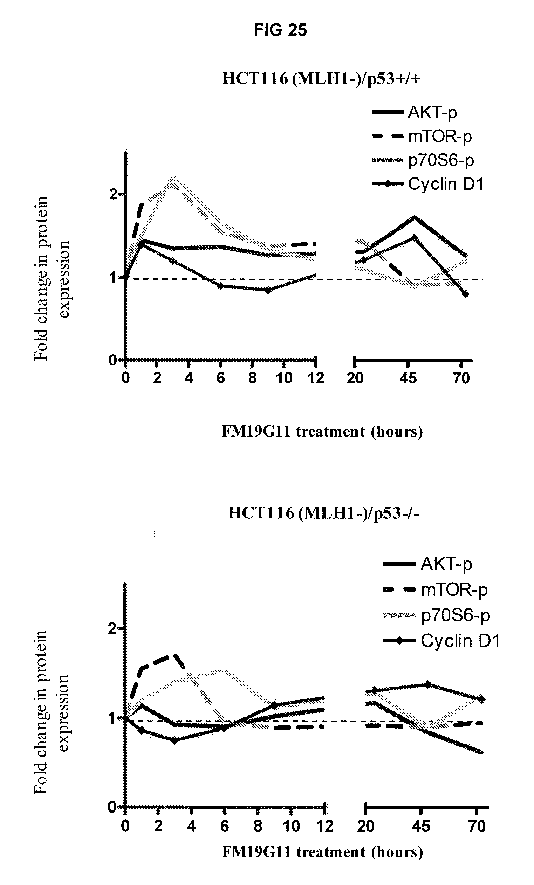

Figure 25. Kinetic studies show a quick and efficient activation (fold change ≥2) of the AKT/mTOR/p70S6/Cyclin D1 signaling pathway in HCT116/p53+/+ cells treated with FM19G11 (0.5 µM). mTOR was also phosphorylated quickly (but with an increase of fold change slightly ≤ 2) in HCT116/p53-/- cells after the treatment with FM19G11.

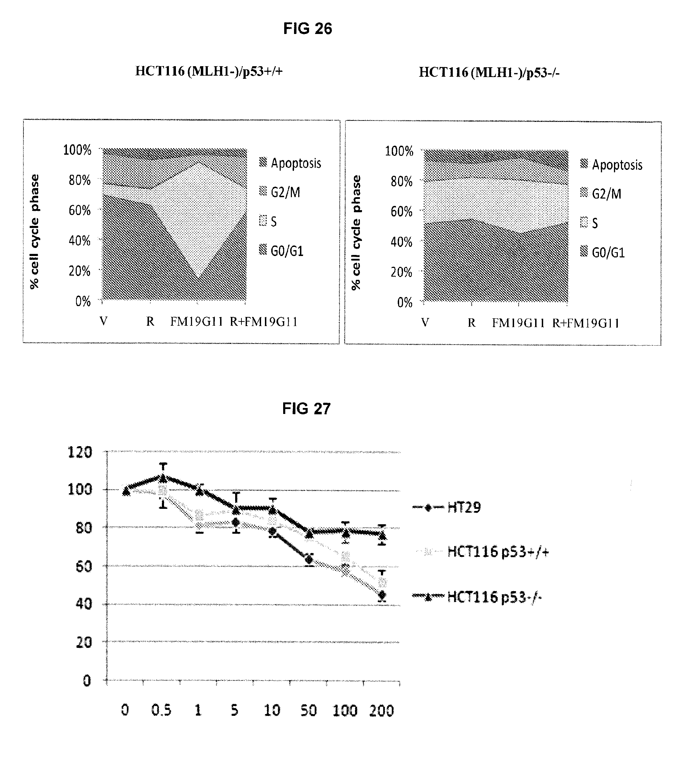

Figure 26. The HCT116/p53+/+ and HCT116/p53-/- cells were exposed to FM19G11 (10 µM), rapamycin (R) (a mTOR inhibitor) (100 pmol) alone or a combination of both drugs at the mentioned concentrations. The S phase cycle arrest occurring in HCT116/p53+/+ cells by treatment with FM19G11 was prevented when a simultaneous incubation of both drugs (100 pmol) was used.
Figure 27. The total ATP content of the cells is correlated with the cell viability, which was quantified as percentage of human colon cancer cells treated with FM19G11 and relative to the corresponding cell lines treated with DMSO for 48 hours.

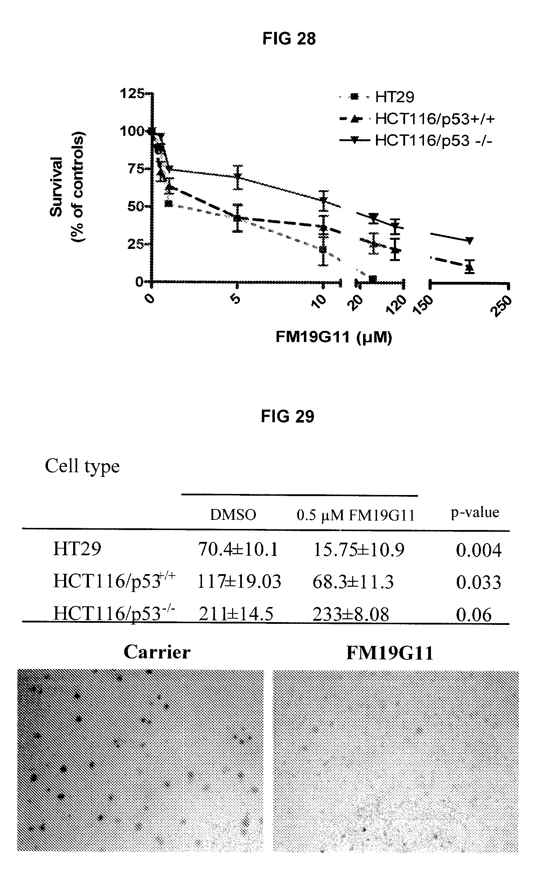

Figure 28. Results of the clonicity assays of HT29 human colon cancer cells, HCT116 cells treated with FM19G11 were monitored up to 10 after the treatment. The IC₅₀ was determined by extrapolating the percentage with the concentration. FM19G11 was used at concentrations: 0.5, 1, 5, 10, 50, 100, 200 µM
Figure 29. Formation of colonies of HT29, HCT116/p53+/+ and HCT116/p53-/- cells in semisolid agar medium. The number of colonies of each cell type which grew in noble agar was quantified 10 days after the seeding. The table shows the means of the different counts of the number of colonies of each of the mentioned cell lines (±SD) by five different fields, with a 4x lens magnification. The results represent counts of three independent experiments in which cells seeded and treated with carrier (DMSO) or FM19G11 (0.5 µM) were counted. Photographs representative of the formation of colonies in the HT29 tumor cell line were taken 10 days after the seeding in semisolid medium.

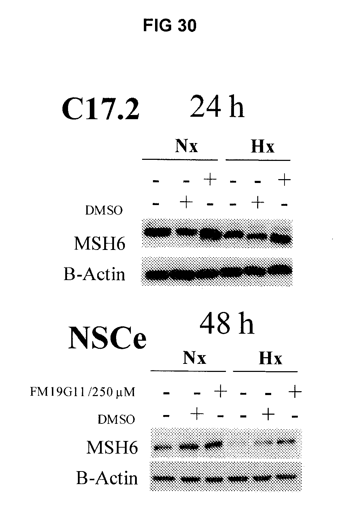

Figure 30. C17.2 neural stem cells and mouse neurospheres experience a decrease of the levels of the MSH6 repair protein under hypoxic conditions which are recovered in the presence of FM19G11. Under normoxic conditions, a decrease of the MSH6 protein by treatment with DMSO which is rescued by FM19G11 is observed in C17.2 cells.

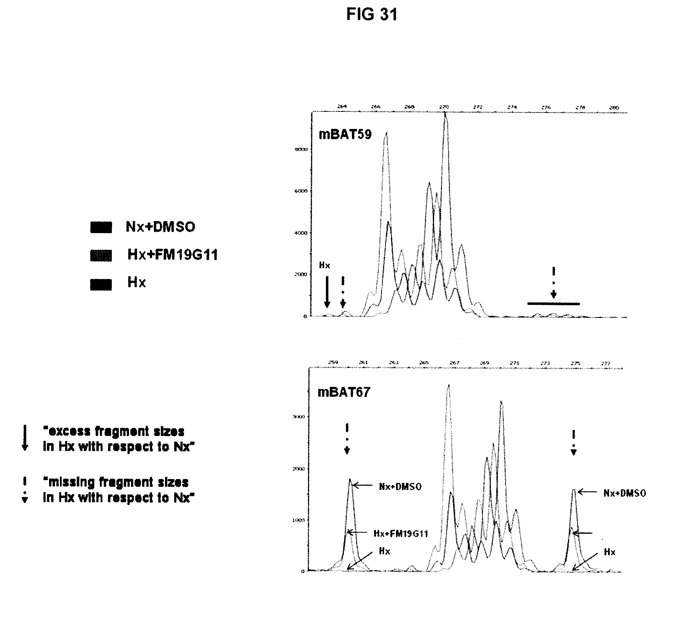

Figure 31. The genomic instability in mouse neurospheres (neural stem cells) caused by hypoxia is prevented by treatment with FM19G11. By using specific fluorescence-labeled primers for microsatellite sequence markers, the genomic stability of neurospheres in normoxia (Nx), hypoxia (Hx) or hypoxia+FM19G11 (Hx+19G11) was analyzed. In the figure it can be observed how two of the markers used (*mBAT59* and *mBAT67*) showed deletions or insertions in the DNA due to hypoxic conditions. The treatment with FM19G11 enabled the recovery of the normoxia profiles (used as a reference or control), thus preventing the genomic instability caused by hypoxia.

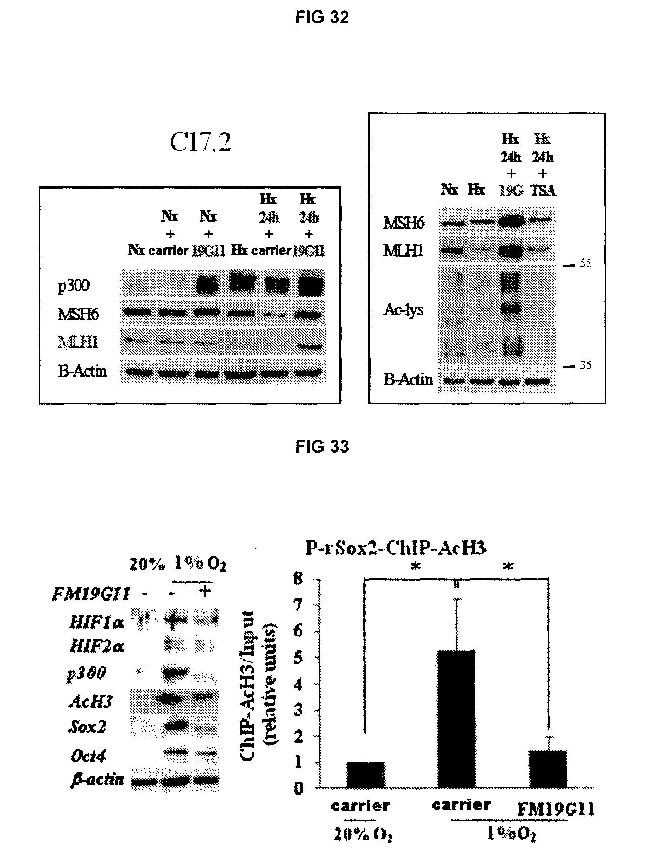

Figure 32. FM19G11 causes changes of expression of p300 (a histone acetyltransferase) and of DNA repair proteins (MLH1 and MSH6) under normoxic and hypoxic conditions in C17.2 neural stem cells. FM19G11 affects the degree of acetylation of global proteins. TSA was included as a positive control for an agent forcing histone acetylation.
Figure 33. Effects caused by FM19G11 on the expression of proteins of adaptation to oxygen changes (HIFα), undifferentiation markers (Sox2, Oct3/4), histone acetyltransferase (p300) and acetylated histone H3, under normoxic or hypoxic conditions (left panel). Influence of FM19G11 on the state of acetylation of histone H3 in normoxia and hypoxia determined by means of immunoprecipitation of chromatin using to that end an anti-AcH3 antibody (acetylated histone H3) and quantifying the DNA by means of quantitative PCR (right graph).

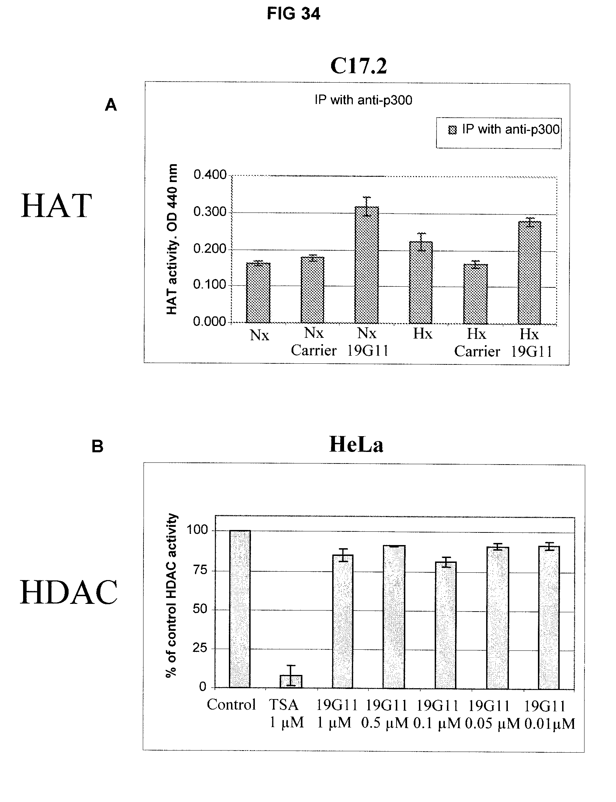

Figure 34. Evaluation of the acetyltransferase or histone deacetylase activity of FM19G11. The upper graph shows the acetyltransferase (HAT) activity of the protein extracts of cells which are untreated, treated with carrier or FM19G11, cultured under normoxic or hypoxic conditions. The protein extracts were previously immunoprecipitated with an anti-p300 antibody and the acetyltransferase activity measured by the detection of NADH. The evaluation of the histone deacetylase (HDAC) activity was carried out with protein extracts of HeLa cells treated with different concentrations of FM19G11. As a positive control, a fixed concentration of TSA (1 µM) was included and extracts of untreated cells were used as a control. The experiments were carried out following the instructions of the Biomol kit for detecting histone deacetylase activity (HDAC Fluorimetric Cellular Activity Assay, Biomol, Cat. No. AK-503).

### Object of the Invention

An object of the present invention relates to compounds represented by general formula (I), especially the one called FM19G11, which are capable of modulating, regulating and/or inhibiting the HIF transcription factor. Such compounds are useful for the treatment of diseases related to cancer, inflammation, autoimmune diseases and regenerative therapy.

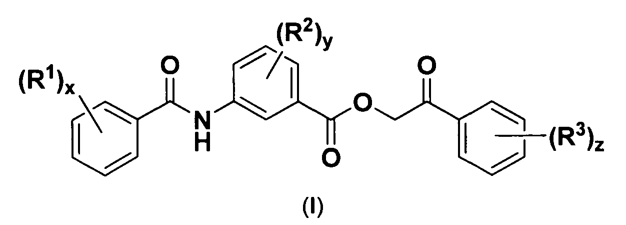

Another object of the invention relates to a method for the synthesis of molecules of general formula (I) or a salt thereof.

Another object of the present invention are pharmaceutical compositions containing a therapeutically effective amount of at least one of the molecules of general formula (I) for the treatment of diseases related to angiogenesis. The modulators and/or inhibitors object of the present invention may be present in the same or in a different pharmaceutical composition.

Another object of invention relates to the use of one or more compounds of general formula (I) or a pharmaceutically acceptable salt thereof for preparing a medicament for regulating the transcription of genes modulated by the hypoxia-inducible transcription factor. In a particular embodiment, said medicament regulates the transcription of one or more genes selected from the group: HIF, VEGF, PHD3, MMRs Sox2, Oct3/4, Nanog, Notch1, p53 and p300. In another particular embodiment, said medicament is applicable in the treatment of cancer. In another particular embodiment, said medicament is applicable in the treatment of tumors. In another embodiment, the medicament is applicable in pathologies which cause inflammation. In another embodiment, the medicament is applicable in regenerative medicine, specifically in stem cell differentiation. In another embodiment, the medicament is applicable in the treatment of pathologies which cause tissue degeneration.

Another object of the invention relates to the kit comprising the pharmaceutical composition.

### Detailed Description of the Invention

The selection of natural and synthetic compounds which are potentially active as therapeutic agents has been during recent decades the way to obtain candidate molecules with pharmacological activity. Before the end of the 20^{th} century, these compounds were started to be selected based on "combinatorial libraries" (John Nielsen et al., "Synthetic Methods for the Implementation of Encoded Combinatorial Chemistry" J. Am. Chem. Soc. 1993; 115, 9812-9813) and now in the 21^{st} century the convergence of pharmaceutical science and information technology is providing opportunities for therapy and the treatment of diseases without precedents. Computer science has penetrated all the spheres of the discovery, development and manufacture of drugs. Combinatorial libraries or chemical libraries are large collections of chemical compounds which are selected to identify new potential drugs following a process for identifying small individual molecules from a combinatorial library with pharmacological activity (EP 0751950) or the preparation of a general formula, for example, of amides developed as protease inhibitor drugs (EP 0950045) or new therapeutic targets and the use thereof for analgesic and/or anti-inflammatory identification (ES 2265201 A1). The arrival of the combinatorial library has revolutionized the process of finding new drugs. New technology allows studying 50,000-100,000 samples per day, whereby the information in the world of biology and medicine has been revolutionized (Ashis K. Mukherjee and Anil C. Ghosh. "The challenges in information technology based drug discovery" Int. J. Inf. Man. 2002, (IJITM), vol. 1, No. 4, 345-356).

The selection of a molecule from a database can be carried out, for example, from about 2 million compounds obtained from 15 chemical libraries (Mozziconacci J.C. *et al.,* 9^{th} Electronic Computational Chemistry Conference (ECCC) 03-2003, Internet and World Wide Web). Thus, structural analyses were carried out to assess the singularity and diversity of the libraries. The similarity of these compounds with the drugs was investigated using common chemical characteristics such as flexibility, the types of atoms, the functional groups and "the rule of five". In 1997, Lipinski et al.,("Experimental and computational approaches to estimate solubility and permeability in drug discovery and development settings" .Adv. Drug Delivery Rev. 1997; 23 (1-3), 3-25) described a method for measuring the absorption or the permeability of a compound, called the "rule of five", based on the four parameters specified in the table below:

| **PARAMETERS** | **VALUES** (For good absorption or prediction of permeability) |
|---|---|
| Log P | ≤ 5 |
| HB donors | ≤ 5 |
| HB acceptors | ≤ 10 |
| Molecular weight | ≤ 500 |

Specifically, the active molecule of general formula (I) of the present invention was identified from the deconvolution subsequent to the massive screening of the following commercial chemical libraries: Key organics, ChemStar; InterbioScreen, Microsource Discovery Systems, Tim Tec, Tripos, Speccs, Sigma-Aldrich, Otava, Saines, ChemBridge, Prestwick Chemical Library, from among which Myrianscreen (Sigma-Aldrich) and Prestwick, which entail a good representation of structural diversity and of properties of compounds with drug potential, were selected. Thus, the selection criteria of the compounds which make up the chemical libraries of the Centro de Investigación Principe Felipe (CIPF) are internal criteria based on:
- The structural diversity, coverage of the chemical space based on estimating the Tanimoto coefficient.
- Lipinski's rule of 5
- clogP ≤ 5
- Molecular weight, between 300 and 900.
- Reactivity. HB donors and acceptors
- Non-evident cell toxicity.
- Ease of synthesis. Between 3-10 steps of described synthesis or the structural complexity of which entails- according to the opinion of the synthesis chemists-, a similar process.

In a first aspect, the invention relates to a compound selected from general formula (I): 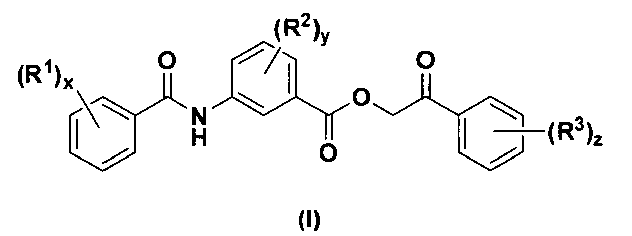 wherein: R¹, R² and R³ can independently be:
(i) hydrogen;
(ii) a branched or linear -(CH₂)ₙ-H alkyl group, optionally with an unsaturation, wherein n= 1-10, preferably methyl or ethyl;
(iii) a -COOH carboxylic acid or an ester of formula -COOR, R being a branched or linear -(CH₂)ₙ-H alkyl group, optionally with an unsaturation, wherein n= 1-10, preferably methyl or ethyl;
(iv) -OH hydroxyl group, -NO₂ group;
(v) halogen; -CHa₃ group, wherein Ha= halogen, preferably CF₃;
(vi) -NHCOR, R being a branched or linear -(CH₂)ₙ-H alkyl group, optionally with an unsaturation, wherein n= 1-10, preferably methyl or ethyl;
   x, y, z being= 1 - 4;
or a pharmaceutically acceptable salt thereof, except for 2-4-(methoxy)phenyl)-2-oxoethyl 3-(2,4-(dinitro)benzamido)benzoate (R¹= 2,4-di-NO₂, R²= H, R³= 4-OMe), and 2-4-(nitro)phenyl)-2-oxoethyl 3-(4-bromo)benzamido)benzoate (R¹= 4-Br, R²= H, R³= 4-NO₂).

The compound selected from among those comprised by general formula (I) as representative of the invention is the one called FM19G11 wherein R¹= 2,4-di-NO₂, R²= H, and R³= 4-CH₃.

The term "pharmaceutically acceptable salt" indicates a formulation of a compound which does not cause significant irritation in an organism and does not reduce the biological activity or the properties of the compound. Pharmaceutical salts can be obtained by the reaction of a compound of the invention with inorganic acids, for example hydrochloric acid, hydrobromic acid, sulfuric acid, nitric acid, phosphoric acid, methanesulfonic acid, ethanesulfonic acid, p-toluenesulfonic acid, salicylic acid and the like.

The invention also relates to a method for the synthesis of compounds comprised within general formula (I), generally consisting of three steps. Figure 1 shows the summary of the synthesis of the compounds which are obtained as detailed below in the examples.

Another aspect of the invention relates to pharmaceutical compositions containing an effective amount of one or more compounds of formula (I) and one or more carriers or diluents and optionally one or more physiologically acceptable adjuvants.

Generally, it is desirable to administer a dose of each compound of at least 10 micromolar and, preferably of at least 100 nanomolar, the administration is preferably in the range of 100 to 500 nanomolar, although smaller doses can be used. The dose can be a single dose and can be administered in powder form or solid form. The administration period can vary, being able to be a continuous administration or an administration of periodic or continuous release during days, weeks or months, although a daily periodicity or even a periodicity of several times a day can be recommendable in certain cases.

The effective dose for the administration of the pharmaceutical composition (or inhibitors) depends on the effect of the compounds and on their potency as inhibitors. The term "effective dose" relates to the sufficient amount of active compound to cause the desired effect in which angiogenesis and the symptoms of the disease are attenuated. The dose must not be used in proportions causing unwanted side effects, the clinical assessment of which makes them adverse and non-treatable therapeutically, such as hyperviscosity syndromes, pulmonary edema, congestive heart failure, and the like. Generally, the dose will vary with the age, condition, sex and extent of the disease in the patient and can be determined in each case.

The administration route of the active compound in the form of pharmaceutical composition can be intravenous (IV), intramuscular (IM), subcutaneous (SC), intradermal (ID), intraperitoneal (IP), intrathecal, i.e., in the space surrounding the spinal cord (IT), intraocular (IO), intrapleural (IPI), intrauterine (IU), rectal, vaginal, topical, intratumoral, or the like, depending on the problem to be solved and on the patient.

If necessary, the compound of the invention can be administered as a prodrug. The term "prodrug" indicates an agent which "in vivo" turns into the original drug. In certain situations, prodrugs can be more easy to administer than the original drug. For example, the prodrug can be bioavailable by oral administration, but not the original compound, or the prodrug can have a better solubility allowing its intravenous administration.

The compounds object of the present invention can be mixed with pharmaceutically acceptable excipients which act as diluents or carriers, compatible with the active ingredient, in amounts suitable for being used in therapeutically acceptable methodologies. Suitable excipients are, for example although not limited thereto, water, saline solution, dextrose, glycerol, ethanol or the like and combinations thereof. Furthermore, if desired, the composition can contain substances such as wetting agents or emulsifiers, pH buffers and the like which stabilize or improve the effectiveness of the active ingredients.

Generally, the modulator and/or inhibitor compounds of the invention must be formulated in a buffered solution at a pH between 5.5 and 7.0, more preferably between 5.5-6.0 and more preferably adjusted to 5.5. The pH can be adjusted by adding a suitable acid, such as hydrochloric acid. Furthermore, the aqueous "carriers" can contain more than one saline buffer, as well as salts such as sodium and potassium chloride, dextrose, polyethylene glycol, and other solutes. The liquid compositions can also contain other liquid solutions in addition to or instead of water, such as for example, liquid solutions of glycerin, vegetable oils and the like.

The compounds of the invention can contain salts of the components which are pharmaceutically acceptable. Pharmaceutically acceptable salts include salts formed by acid addition (formed with the amino group of the structure of the inhibitor) which are formed with inorganic acids such as for example hydrochloric acid or phosphoric acid or such as organic acids such as acetic acid, tartaric acid, mandelic acid and the like. The salts formed with radicals of carboxylic groups can be derived from inorganic bases such as for example, sodium, potassium, ammonium, calcium or ferric hydroxides and from organic bases such as isopropylamine, trimethylamine, 2-ethylaminoethanol, histidine, procaine and the like.

Another aspect of the invention is a kit comprising the pharmaceutical composition with one or more compounds of general formula (I), a carrier or diluent and/or one or more adjuvants, which can be together or separate in one or more containers depending on the administration route of the composition in each case.

The selection criteria of the compounds which make up the chemical libraries are internal criteria based on the structural diversity, the representativeness of pharmacophores, the number of chiral centers, the solubility and the ease of the chemical synthesis thereof. The collection of the CIPF was selected through the assay of the HIF response elements (HRE) (Example 1), the active compounds being selected by their potency against the inhibition of the activity and by their selectivity against cell assays based on the response elements of two different transcription factors, Jun B (CRE) and c-Fos (TRE), (Example 2). The selection of the best inhibitor obtained according to these potency and selectivity criteria was identified as FM19G11, which involved the need to obtain a sufficient amount of said active compound. Therefore, the present invention also relates to a method for obtaining by chemical synthesis the compound FM19G11 (Figure 1). Methods for obtaining by chemical synthesis intermediates as well as structural analogs of the compound FM19G11 are also set forth. These analogs are compared in Structure vs. Activity Relationship (SAR) studies (Examples 3-31).

The work developed show how the compounds of the invention inhibit the transcription of target genes of HIF, such as prolyl-hydrolase 3 (PHD 3), HIF itself in its autocrine regulation and the vascular endothelial growth factor (VEGF) in a tumor cell model (Example 32). This inhibitory activity is also shown on neural progenitor cell models (Example 33) as well as on a primary culture of adult spinal cord ependymal neural progenitors (epNSC) (Examples 34 and 35) and, finally, on a dental pulp mesenchymal cell model (Example 36 and 37).

The present invention demonstrates the low cell toxicity of FM19G11 against a panel of mammalian cell lines with the representation of different tissues (Example 39) as well as against whole vertebrate organisms in fish embryo models (Example 45).

The invention shows how the compound of the invention, FM19G11, allows the progress of the process of differentiation of epSPCs into oligodendrocytes under hypoxic conditions (Example 42), and how under normoxic conditions it allows self-renewal and maintains its undifferentiated state (Example 43).

The invention characterizes FM19G11 by means of HPLC by calculating its retention time (Example 46), this analytical method serving to establish the stability of FM19G11 in bovine serum (Example 47).

The present work also develops how FM19G11 triggers a DNA damage response in HT29 human colon cancer cells (Example 48) and the activation kinetics involved in said process (Example 49).

The compound of the present invention induces the G1/S phase cell cycle arrest in human colon cancer tumor cells in a p53-dependent manner (Example 20), and the connection existing between the mTOR signaling pathway and the S phase cell cycle arrest is detailed in Example 51.

Example 56 of the present invention shows how FM19G11 has the capacity to remove chromatin by epigenetic events.

All these features present the compounds of the invention as possible modulators and/or inhibitors of pathological processes of angiogenesis, inflammation, apoptosis and cell therapy.

### Examples

The following specific examples which are provided herein serve to illustrate the nature of the present invention. These examples are included solely for illustrative purposes and must not be interpreted as limitations to the invention claimed herein.

### Example 1: Assay of HIF 1 and selection of the compound FM19G11

In order to identify direct inhibitors of the activity of HIF, a stable recombinant line was first constructed from the cervical tumor line, known in scientific literature as HeLa, which contained the deoxyribonucleic acid (DNA) sequences of the region located between positions -985 and -951 of the human vascular endothelial growth factor (hVEGF), repeated nine times (9X) and fused to the luciferase reporter gene (LUC). This plasmid, provided by Dr. Manuel Ortiz de Landazuri from the Hospital de La Princesa of Madrid, called 9XHRE-LUC, was prepared using a commercial kit (Endofree Maxi-Prep; Qiagen, Inc., Valencia, CA). The transfections were performed with FuGene6 (Roche, cat.1814443) according to the manufacturer's instructions, the µg DNA: µg FuGene ratio was 1:3. The transfected cell lines were generated by cotransfection with the vector pCMV-Hygrom which expressed a hygromycin resistance gene in mammalian cells, allowing the selection of the transfected clones. After 48 hours, the growth medium was substituted with fresh medium containing 100 µg/ml hygromycin (Sigma), the clones resistant to the antibiotic were collected and analyzed by means of RT-PCR to select the clone with the desired construct. Routinely, the HeLa 9XHRE-LUC cell line was maintained in exponential growth by means of performing subcultures in DMEM (Invitrogen-Life Technologies, Inc., Carlsbad, CA), supplemented with 10% inactivated fetal bovine serum (Whittacker Bioproducts), penicillin (50 IU/ml), streptomycin (50 µg/ml) (Invitrogen-Life Technologies, Inc., Carlsbad, CA). The cells were cultured at 37°C with a humidified atmosphere containing 5% CO₂ and 300 nM Desferoxamine (DFX; Sigma St. Louis, MO).

The compounds of the Collection of the CIPF, coming from a selection of commercial collections of Sigma-Aldrich, were solubilized in DMSO at a final concentration of 5 mM and preserved at -80°C. The concentration of these compounds in the luciferase assay was 1 mcM finally in the well of the multi-well plate in which the reaction was carried out. The luciferase assays were performed in white 96-well plates *(Valtecknova ref. 3917*), using the kit Bright-Glo™ Luciferase Assay System (Promega, Inc., Madison, WI) following the instructions supplied by the manufacturer. From the 12,250 compounds analyzed the compound FM19G11 was selected, which had an IC50 of 80 nM under chemical hypoxic conditions (300 nM DFX), an activity which was subsequently corroborated under physical hypoxic conditions (1% O₂) in a hypoxia chamber (IN VIVO₂ 400 RUSKINN).

Figure 2 A shows the chemical structure of FM19G11 and Figure 2 B shows the dose response curve with FM19G11 with the corresponding IC50 obtained of 80 nM. Figure 2 C shows the relationship of inhibition of the activity of HIF1α by FM19G11 expressed in relative luciferase units (RLU) under hypoxic (1% O₂) and normoxic (20% O₂) conditions.

### Example 2: FM19G11 selectivity studies

The selectivity assays with the active compound FM19G11 were performed in white 96-well plates using the recombinant strains Hek 293-TRE-LUC and Hek 293-CRE-LUC which represent the transcriptional activity of transcription factors of the AP-1 complex. The luciferase assay for measuring transcriptional activity was performed using the kit Bright-Glo™ Luciferase Assay System (Promega, Inc., Madison, WI), following the instructions supplied by the manufacturer and the Hek 293-TRE-LUC and Hek 293-CRE-LUC lines contain the response elements of transcription factors of the AP-1 complex, specifically of c-FOS and JUNB, respectively. The Hek 293-TRE-LUC and Hek 293-CRE-LUC lines were provided by Dr. Rosa Farrás.

Figure 3 shows the selective activity of FM19G11 by inhibition of the expression of luciferase observed in the assays performed against three cell lines. The HeLa 9XHRE-LUC line (black bars) has an inhibition dose-dependent on the concentration of the inhibitor whereas the transcriptional levels of Hek 293-TRE-LUC (gray bars) and Hek 293-CRE-LUC (white bars), measured by the luminescent emission of the LUC reporter gene, are not affected in any case under the experimental conditions performed.

Figure 1 summarizes the preparation of the compounds of the invention.

### Example 3. Synthesis of methyl 3-aminobenzoate.

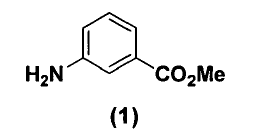

Thionyl chloride (0.37 mL, 3.27 mmol) was slowly added to a solution of 3-aminobenzoic acid (0.3 g, 2.18 mmol) in dry MeOH (3.26 mL) at 0°C. The reaction was stirred for 16 hours and then hydrolyzed with a saturated aqueous solution of NaHCO₃. The crude product was extracted with CH₂Cl₂, dried on Na₂SO₄ and concentrated under reduced pressure. The product was used in the following phases without purification (yield: 90%).

### Example 4. Synthesis of methyl 3-((2,4-dinitro)benzamido)benzoate.

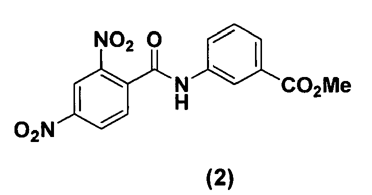

**1** (0.11 g, 0.82 mmol), EDCI (0.23 g, 1.24 mmol), HOBt (0.25 g, 1.65 mmol) and Et₃N (0.23 mL, 1.65 mmol) were added to a solution of 2,4-dinitrobenzoic acid (0.17 g, 0.82 mmol) in dry CH₂Cl₂ (4 mL). After stirring for 3 hours, the reaction was hydrolyzed with a saturated aqueous solution of NH₄Cl. The crude product was extracted with CH₂Cl₂, dried on Na₂SO₄ and concentrated under reduced pressure. The product was purified by flash chromatography with hexane-EtOAc (2:1) as an eluent (yield: 61%). mp: 140-143°C. ¹H NMR (300 MHz, CD₃COCD₃) δ 3.94 (s, 3H), 7.59 (t, *J* = 8.0 Hz, 1 H), 7.86 (dt, *J*₁ = 7.8 Hz, *J*₂ = 1.0 Hz, 1H), 8.02 (dc, *J*₁ = 9.0 Hz, *J*₂ = 1.2 Hz, 1 H), 8.25 (d, *J* = 8.5 Hz, 1 H), 8.45 (t, *J* = 1.8 Hz, 1 H), 8.76 (dd, *J*₁ = 8.4 Hz, *J*₂ = 2.5 Hz, 1 H), 8.94 (d, *J* = 2.4 Hz, 1 H), 10.2 (broad s, 1 H). ¹³C NMR (75.5 MHz, CD₃COCD₃) δ 51.6, 119.8, 120.5, 124.1, 125.2, 128.3, 129.2, 130.9, 137.5, 138.8, 147.0, 148.6, 162.7, 166.0. HRMS (EI) calculated for C₁₅H₁₁N₃O₇ (M⁺): 345.0597, experimental: 345.0570.

### Example 5. Synthesis of 3-((2,4-dinitro)benzamido)benzoic acid.

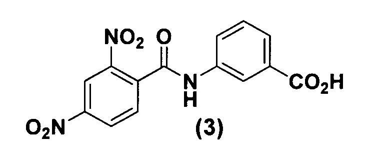

LiOH•H₂O (0.036 g, 0.86 mmol) was added to a solution of 2 (0.1 g, 0.28 mmol) in 4:1 THF:H₂O (15 ml). The reaction was stirred for 16 hours and then hydrolyzed with 1 M HCl. The crude product was extracted with EtOAc, dried on Na₂SO₄ and concentrated under reduced pressure. The product was used without subsequent purification (yield: 99%). mp: 260-263°C. ¹H NMR (300 MHz, CD₃COCD₃) δ 2.90 (broad s, 1H), 7.58 (t, *J* = 8.3 Hz, 1H), 7.90 (dt, *J*₁ = 7.8 Hz, *J*₂ = 1.1 Hz, 1H), 8.0 (dt, *J*₁ = 8.2 Hz, *J*₂ = 1.0 Hz, 1H), 8.2 (d, *J* = 8.2 Hz, 1 H), 8.4 (t, *J* = 1.4 Hz, 1H), 8.7 (dd, *J*₁ = 8.6 Hz, *J*₂ = 2.3 Hz, 1H), 8.9 (d, *J* = 2.1 Hz, 1H), 10.2 (broad s, 1H). ¹³C NMR (75.5 MHz, CDCl₃) δ 121.5, 122.7, 125.8, 127.2, 130.1, 130.8, 132.5, 133.1, 139.3, 140.5, 148.7, 150.2, 164.4, 168.0. HRMS (EI) calculated for C₁₄H₉N₃O₇ (M⁺): 331.0440, experimental: 331.0430.

### Example 6. Synthesis of 2-oxo-2-(p-tolyl)ethyl 3-((2,4-dinitro)benzamido)benzoate.

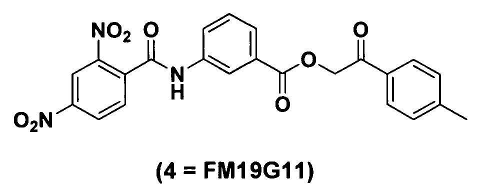

An aqueous solution of Cs₂CO₃ was added to a solution of acid 3 (0.2 g, 0.6 mmol) in 1:1 H₂O/EtOH (6 mL) until reaching a pH of 6.5 in order to obtain cesium salt. This salt was concentrated under reduced pressure, redissolved in DMF (3.2 mL), and bromomethyl-*p-*tolyl ketone (0.141 g, 0.66 mmol) was added. After stirring for 3 hours, the reaction was hydrolyzed with a saturated aqueous solution of NaCl. The crude product was extracted with EtOAc, dried on Na₂SO₄ and concentrated under reduced pressure. The product was purified by flash chromatography with hexane-EtOAc (3:1) as an eluent (yield: 54%). mp: 168-170°C. ¹H NMR (300 MHz, CDCl₃) δ 2.37 (s, 3H), 5.41 (s, 2H), 7.18-7.25 (m, 3H), 7.59 (d, *J* = 8.6 Hz, 2H), 7.69 (t, *J* = 6.8 Hz, 2H), 7.85 (d, *J* = 8.3 Hz, 1H), 8.01 (s, 1 H), 8.30 (dd, *J*₁ = 8.3 Hz, *J*₂ = 2.0 Hz, 1H), 8.64 (d, *J* = 2.0 Hz, 1H), 8.95 (s, 1H). ¹³C NMR (75.5 MHz, CDCl₃) δ 21.9, 66.4, 120.0, 121.3, 125.2, 126.6, 127.8, 128.2, 129.4, 129.7, 130.6, 131.1, 137.3, 137.4, 145.7, 146.3, 148.1, 162.6, 165.3, 173.9, 192.8. HMRS (EI) calculated for C₂₃H₁₇N₃O₈ (M⁺): 463.1015, experimental: 463.0999.

Example 7 Synthesis of methyl 3-(2-nitro-4-(trifluoromethyl)benzamido)benzoate.

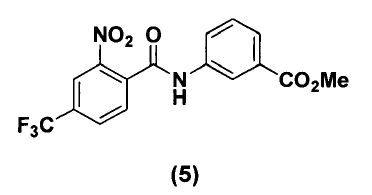

**1** (0.08 g, 0.62 mmol), EDCI (0.17 g, 0.93 mmol), HOBt (0.19 g, 1.24 mmol) and Et₃N (0.17 mL, 1.24 mmol) were added to a solution of 2-nitro-4-(trifluoromethyl)benzoic acid (0.14 g, 0.62 mmol) in dry CH₂Cl₂ (3 mL). After stirring for 16 hours, the reaction was hydrolyzed with a saturated aqueous solution of NH₄Cl. The crude product was extracted with CH₂Cl₂, dried on Na₂SO₄ and concentrated under reduced pressure. The product was purified by flash chromatography with hexane-EtOAc (4:1) as an eluent (yield: 90%). mp: 133-136°C. ¹H NMR (300 MHz, CDCl₃) δ 3.75 (s, 3H), 7.30 (t, *J* = 7.6 Hz, 1H), 7.66 (s, 1H), 7.69 (s, 1H), 7.79 (d, *J* = 7.7 Hz, 1 H), 7.81 (d, *J* = 8.9 Hz, 1H), 7.91 (s, 1H), 8.17 (s, 1H), 8.46 (s, 1H). ¹³C NMR (75.5 MHz, CDCl₃) δ 52.6, 121.5, 122.0 (c, ³*J*_{CF} = 3.7 Hz), 122.5 (c, ¹*J*_{CF} = 275.4 Hz), 125.3, 126.5, 129.5, 130.0, 130.8 (c, ³*J*_{CF} = 3.2 Hz), 131.0, 133.4 (c, ²*J*_{CF} = 34.8 Hz), 135.4, 137.5, 146.4, 163.9, 167.0. ¹⁹F NMR (282.4 MHz, CDCl₃) δ -63.1 (s, 3F). HRMS (EI) calculated for C₁₆H₁₁F₃N₂O₅ (M⁺): 368.0620, experimental: 368.0620.

### Example 8. Synthesis of 3-(2-nitro-4-(trifluoromethyl)benzamido)benzoic acid.

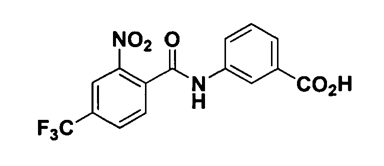

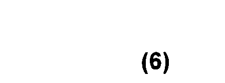

LiOH•H₂O (0.56 g, 1.6 mmol) was added to a solution of **5** (0.1 g, 0.33 mmol) in 4:1 THF:H₂O (20 mL). The reaction was stirred for 16 hours and then hydrolyzed with 1 M HCl. The crude product was extracted with EtOAc, dried on Na₂SO₄ and concentrated under reduced pressure. The product was used without subsequent purification (yield: 83%). mp: 264-267°C. ¹H NMR (300 MHz, CD₃COCD₃) δ 7.40 (t, *J* = 7.7 Hz, 1 H), 7.71 (dt, *J*₁ = 7.8 Hz, *J*₂ = 1.3 Hz, 1H), 7.88 (dc, *J*₁ = 7.8 Hz, *J*₂ = 1.1 Hz), 8.01 (d, *J* = 8.1 Hz, 1 H), 8.14 (dd, *J*₁ = 7.8 Hz, *J*₂ = 0.9 Hz, 1 H), 8.30 (t, *J* = 1.8 Hz, 1 H), 8.34 (s, 1 H), 9.97 (broad s, 1 H). ¹³C NMR (75.5 MHz, CD₃COCD₃) δ 121.8, 122.5 (c, ³*J*_{CF} = 4.1 Hz), 124.9, 126.3, 123.8 (c, ¹*J*_{CF} = 272.7 Hz), 130.0, 131.5, 132.3, 133.0 (c, ²*J*_{CF} = 35.3 Hz), 136.9, 139.7, 148.0, 163.9, 167.2. ¹⁹F NMR (282.4 MHz, CD₃COCD₃) δ -111.6 (s, 3F). HRMS (EI) calculated for C₁₅H₉F₃N₂O₅ (M⁺): 354.0463, experimental: 354.0473.

### Example 9. Synthesis of 2-oxo-2-(p-tolyl)ethyl 3-(2-nitro-4-(trifluoromethyl)benzamido)benzoate.

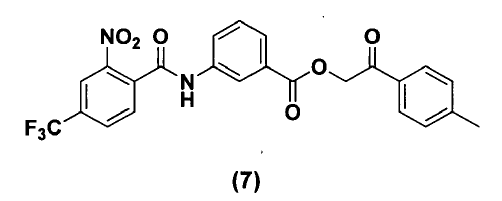

An aqueous solution of Cs₂CO₃ was added to a solution of acid **6** (0.06 g, 0.2 mmol) in 1:1 H₂O/EtOH (2 mL) until reaching a pH of 6.5 in order to obtain the cesium salt. This salt was concentrated under reduced pressure, redissolved in DMF (1.0 mL), and bromomethyl-*p*-tolyl ketone (0.03 g, 0.18 mmol) was added. After stirring for 4 hours, the reaction was hydrolyzed with a saturated aqueous solution of NaCl. The crude product was extracted with EtOAc, dried on Na₂SO₄ and concentrated under reduced pressure. The product was purified by flash chromatography with hexane-EtOAc (4:1) as an eluent (yield: 32%). mp: 179-181°C. ¹H NMR (300 MHz, CD₃COCD₃) δ 2.29 (s, 3H), 5.58 (s, 2H), 7.24 (s, 1H), 7.26 (s, 1H), 7.44 (t, *J* = 7.8 Hz, 1H), 7.77 (dt, *J*₁ = 8.0 Hz, *J*₂ = 1.3 Hz, 1H), 7.80 (s, 1 H), 7.83 (s, 1H), 7.91 (dc, *J*₁ = 8.1Hz, *J*₂ = 1.1 Hz, 1 H), 8.02 (d, *J* = 7.8 Hz, 1 H), 8.14 (d, *J* = 8.4 Hz, 1H), 8.34 (s, 1H), 8.37 (t, *J* = 1.4 Hz, 1H), 10.04 (s, 1 H). ¹³C NMR (75.5 MHz, CD₃COCD₃) δ 20.2, 66.2, 120.2, 122.4 (c, ¹*J*_{CF} = 272.1 Hz), 121.1 (c, ³*J*_{CF} = 3.9 Hz), 123.9, 124.9, 127.3, 128.8, 128.9, 130.1, 130.2, 131.3, 131.5, 135.6, 138.5, 144.2, 146.6, 162.7, 164.7, 191.0. ¹⁹F NMR (282.4 MHz, CD₃COCD₃) δ -63.06 (s, 3F). HRMS (EI) calculated for C₂₄H₁₇F₃N₂O₆ (M⁺): 486.1038, experimental: 486.1033.

### Example 10. Synthesis of methyl 3-(4-nitro-2-(trifluoromethyl)benzamido)benzoate.

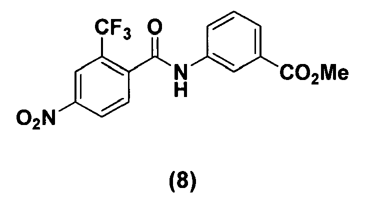

**1** (0.1 g, 0.74 mmol), EDCI (0.21 g, 1.11 mmol), HOBt (0.22 g, 1.48 mmol) and Et₃N (0.20 mL, 1.48 mmol) were added to a solution of 4-nitro-2-(trifluoromethyl)benzoic acid (0.17 g, 0.74 mmol) in dry CH₂Cl₂ (2 mL). After stirring for 16 hours, the reaction was hydrolyzed with a saturated aqueous solution of NH₄Cl. The crude product was extracted with CH₂Cl₂, dried on Na₂SO₄ and concentrated under reduced pressure. The product was purified by flash chromatography with hexane-EtOAc (5:1) as an eluent (yield: 69%). mp: 102-104°C. ¹H NMR (300 MHz, CDCl₃) δ 3.74 (s, 3H), 7.27 (t, *J* = 7.9 Hz, 1 H), 7.63-7.72 (m, 3H), 7.96 (t, *J* = 1.5 Hz, 1 H), 8.21 (dd, *J*₁ = 8.4 Hz, *J*₂ = 2.4 Hz, 1 H), 8.31 (d, *J* = 2.2 Hz, 1 H), 8.80 (s, 1 H). ¹³C NMR (75.5 MHz, CDCl₃) δ 52.5, 121.4, 122.3 (c, ³*J*_{CF} = 4.9 Hz), 122.5 (c, ¹*J*_{CF} = 273.6 Hz), 125.1, 126.6, 127.1, 129.3 (c, ²*J*_{CF} = 25.1 Hz), 129.5, 130.3, 131.2, 137.4, 140.8, 148.3, 164.1, 166.8. ¹⁹F NMR (282.4 MHz, CDCl₃) δ -59.6 (s, 3F). HRMS (EI) calculated for C₁₆H₁₁F₃N₂O₅ (M⁺): 368.0620, experimental: 368.0618.

### Example 11. Synthesis of 3-(4-nitro-2-(trifluoromethyl)benzamido)benzoic acid.

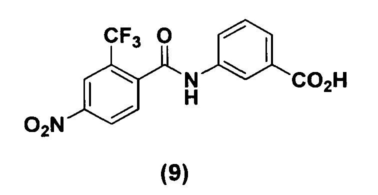

LiOH•H₂O (0.039 g, 0.94 mmol) was added to a solution of **8** (0.08 g, 0.23 mmol) in 4:1 THF:H₂O (13 mL). The reaction was stirred for 16 hours and then hydrolyzed with 1 M HCl. The crude product was extracted with EtOAc, dried on Na₂SO₄ and concentrated under reduced pressure. The product was used without subsequent purification (yield: 99%). mp: 250-255°C. ¹H NMR (300 MHz, CD₃COCD₃) δ 7.41 (t, *J* = 7.9 Hz, 1H), 7.72 (dt, *J*₁ = 7.5 Hz, *J*₂ = 1.4 Hz, 1H), 7.89 (dc, *J*₁ = 9.0 Hz, *J*₂ = 1.1 Hz, 1 H), 8.02 (d, *J* = 8.7 Hz, 1H), 8.32 (t, *J* = 2.1 Hz, 1H), 8.47-8.52 (m, 2H), 9.96 (s, 1H). ¹³C NMR (75.5 MHz, CD₃COCD₃) δ 122.6, 123.4 (c, ³*J*_{CF} = 5.3 Hz), 125.8, 127.1, 129.0, 130.0 (c, ²*J*_{CF} = 32.9 Hz), 130.7, 132.2, 133.1, 140.5, 143.1, 149.9, 165.6, 168.1. ¹⁹F NMR (282.4 MHz, CDCl₃) δ -59.3 (s, 3F). HRMS (EI) calculated for C₁₅H₉F₃N₂O₅ (M⁺): 354.0463, experimental: 354.0475.

### Example 12. Synthesis of 2-oxo-2-(p-tolyl)ethyl 3-(4-nitro-2-(trifluoromethyl)benzamido)benzoate.

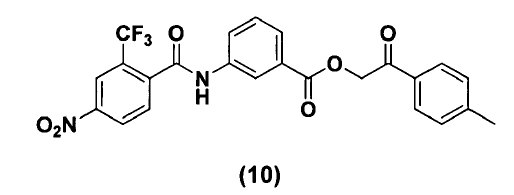

An aqueous solution of Cs₂CO₃ was added to a solution of acid **9** (0.07 g, 0.2 mmol) in 1:1 H₂O/EtOH (2 mL) until reaching a pH of 6.5 in order to obtain the cesium salt. This salt was concentrated under reduced pressure, redissolved in DMF (1.0 mL), and bromomethyl-*p-*tolyl ketone (0.04 g, 0.20 mmol) was added. After stirring for 48 hours, the reaction was hydrolyzed with a saturated aqueous solution of NaCl. The crude product was extracted with EtOAc, dried on Na₂SO₄ and concentrated under reduced pressure. The product was purified by flash chromatography with hexane-EtOAc (4:1) as an eluent (yield: 28%). mp: 165-166°C. ¹H NMR (300 MHz, CD₃COCD₃) δ 2.28 (s, 3H), 5.59 (s, 2H), 7.24 (s, 1H), 7.27 (s, 1H), 7.45 (t, *J* = 8.2 Hz, 1H), 7.77 (d, *J* = 7.7 Hz, 1H), 7.81 (s, 1H), 7.83 (s, 1 H), 7.93 (dd, *J*₁ = 7.2 Hz, *J*₂ = 2.0 Hz, 1 H), 8.04 (d, *J* = 8.7 Hz, 1 H), 8.39 (t, *J* = 1.9 Hz, 1H), 8.48 (broad s, 1H), 8.50-8.53 (m, 1 H), 10.0 (broad s, 1H). ¹³C NMR (75.5 MHz, CD₃COCD₃) δ 20.2, 66.3, 120.3, 121.4 (c, ³*J*_{CF} = 4.6 Hz), 123.9, 125.0, 126.9, 127.3, 128.0 (c, ³*J*_{CF} = 6.3 Hz), 128.8, 128.9, 129.5 (c, ¹*J*_{CF} = 285.8 Hz), 130.0, 130.2, 138.4, 140.8, 144.1, 147.9, 163.5, 164.6, 190.9. ¹⁹F NMR (282.4 MHz, CDCl₃) δ -59.60 (s, 3F). HRMS (EI) calculated for C₂₄H₁₇F₃N₂O₆ (M⁺): 486.1039, experimental: 486.1026.

### Example 13. Synthesis of methyl 3-(2,4-bis-(trifluoromethyl)benzamido)benzoate.

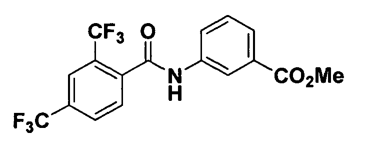

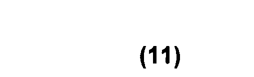

**1** (0.16 g, 1.25 mmol), EDCI (0.35 g, 1.87 mmol), HOBt (0.38 g, 2.50 mmol) and Et₃N (0.34 mL, 2.50 mmol) were added to a solution of 2,4-bis-(trifluoromethyl)benzoic acid (0.32 g, 1.25 mmol) in dry CH₂Cl₂ (5 mL). After stirring for 16 hours, the reaction was hydrolyzed with a saturated aqueous solution of NH₄Cl. The crude product was extracted with CH₂Cl₂, dried on Na₂SO₄ and concentrated under reduced pressure. The product was purified by flash chromatography with hexane-EtOAc (3:1) as an eluent (yield: 50%). mp: 130-132°C. ¹H NMR (300 MHz, CDCl₃) δ 3.82 (s, 3H), 7.41 (t, *J* = 7.5 Hz, 1H), 7.61 (broad s, 1H), 7.73-7.81 (m, 2H), 7.85-7.94 (m, 3H), 8.02 (broad s, 1H). ¹³C NMR (75.5 MHz, CDCl₃) δ 52.5, 121.2 (c, ³*J*_{CF} = 5.1 Hz), 121.4, 124.0, 124.8, 125.1, 126.5, 128.6 (c, ²*J*_{CF} = 34.5 Hz), 129.3, 129.6, 131.2, 132.8 (c, ²*J*_{CF} = 32.1 Hz), 137.6, 138.8. ¹⁹F NMR (282.4 MHz, CDCl₃) δ -59.2 (s, 3F), -63.1 (s, 3F). HRMS (EI) calculated for C₁₇H₁₁F₆NO₃ (M⁺): 391.0643, experimental: 391.0645.

### Example 14. Synthesis of 3-(2,4-bis-(trifluoromethyl)benzamido)benzoic acid.

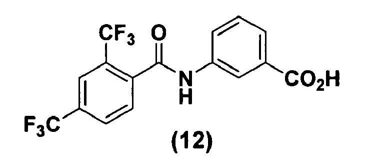

LiOH•H₂O (0.085 g, 2.04 mmol) was added to a solution of **11** (0.2 g, 0.51 mmol) in 4:1 THF:H₂O (29 ml). The reaction was stirred for 16 hours and then hydrolyzed with 1 M HCl. The crude product was extracted with EtOAc, dried on Na₂SO₄ and concentrated under reduced pressure. The product was used without subsequent purification (yield: 97%). mp: 238-241°C. ¹H NMR (300 MHz, CD₃COCD₃) δ 7.41 (t, *J* = 7.9 Hz, 1H), 7.72 (dt, *J*₁ = 7.8 Hz, J₂ = 1.2 Hz, 1 H), 7.90 (dc, *J*₁ = 8.1 Hz, *J*₂ = 0.9 Hz, 1H), 7.94 (d, *J* = 7.8 Hz, 1H), 8.04 (m, 2H), 8.33 (t, *J* = 1.7, 1H), 9.84 (broad s, 1H). ¹³C NMR (75.5 MHz, CD₃COCD₃) δ 120.4, 123.0, 123.5, 124.9, 127.4 (c, ²*J*_{CF} = 31.9 Hz), 128.6, 129.0, 129.3, 130.8, 130.9 (c, ²*J*_{CF} = 33.5 Hz), 138.4, 139.2, 163.9, 165.9. ¹⁹F NMR (282.4 MHz, CDCl₃) δ -59.2 (s, 3F), -63.1 (s, 3F).

### Example 15. Synthesis of 2-oxo-2-(p-tolyl)ethyl 3-(2,4-bis(trifluoromethyl)benzamido)benzoate.

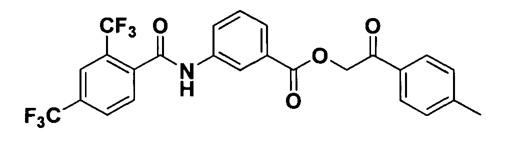

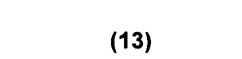

An aqueous solution of Cs₂CO₃ was added to a solution of acid **12** (0.07 g, 0.2 mmol) in 1:1 H₂O/EtOH (2 mL) until reaching a pH of 6.5 in order to obtain the cesium salt. This salt was concentrated under reduced pressure, redissolved in DMF (1.0 mL), and bromomethyl-*p-*tolyl ketone (0.04 g, 0.20 mmol) was added. After stirring for 12 hours, the reaction was hydrolyzed with a saturated aqueous solution of NaCl. The crude product was extracted with EtOAc, dried on Na₂SO₄ and concentrated under reduced pressure. The product was purified by flash chromatography with hexane-EtOAc (3:1) as an eluent (yield: 31%). mp: 161-163°C. ¹H NMR (300 MHz, CDCl₃) δ 2.36 (s, 3H), 5.46 (s, 2H), 7.20-7.23 (m, 2H), 7.38 (t, *J* = 8.2 Hz, 1 H), 7.73 (d, *J* = 8.7 Hz, 2H), 7.77-7.85 (m, 4H), 7.89-7.94 (m, 2H), 8.11 (s, 1H). ¹³C NMR (75.5 MHz, CDCl₃) δ 21.8, 66.5, 121.3, 123.7, 125.1, 126.6, 127.8, 128.1, 128.9 (c, ²*J*_{CF} = 40.7 Hz), 129.4, 129.5, 129.6, 130.2, 131.4, 137.4, 138.7, 142.5, 145.1, 164.3, 165.4, 191.8. ¹⁹F NMR (282.4 MHz, CDCl₃) δ -59.2 (s, 3F), -63.1 (s, 3F). HRMS (EI) calculated for C₂₅H₁₁F₆NO₄ (M⁺): 509.1061, experimental: 509.1062.

### Example 16. Synthesis of methyl 3-(benzamido)benzoate.

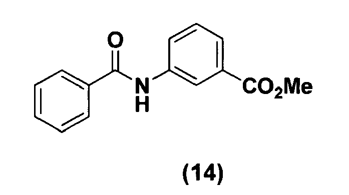

**1** (0.2 g, 1.8 mmol), EDCI (0.5 g, 2.7 mmol), HOBt (0.5 g, 3.7 mmol) and Et₃N (0.5 mL, 3.7 mmol) were added to a solution of benzoic acid (0.4 g, 3.6 mmol) in dry CH₂Cl₂ (8 mL). After stirring for 16 hours, the reaction was hydrolyzed with a saturated aqueous solution of NH₄Cl. The crude product was extracted with CH₂Cl₂, dried on Na₂SO₄ and concentrated under reduced pressure. The product was purified by flash chromatography with hexane-EtOAc (5:1) as an eluent (yield: 20%).

### Example 17. Synthesis of 3-(benzamido)benzoic acid.

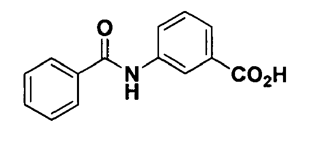

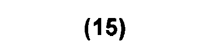

LiOH•H₂O (0.03 g, 0.8 mmol) was added to a solution of **14** (0.05 g, 0.2 mmol) in 4:1 THF:H₂O (14 ml). The reaction was stirred for 16 hours and then hydrolyzed with 1 M HCl. The crude product was extracted with EtOAc, dried on Na₂SO₄ and concentrated under reduced pressure. The product was used without subsequent purification (yield: 90%).

### Example 18. Synthesis of 2-oxo-2-(p-tolyl)ethyl 3-(benzamido)benzoate.

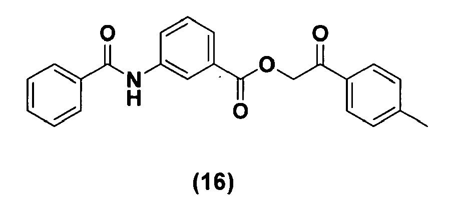

An aqueous solution of Cs₂CO₃ was added to a solution of acid **15** (0.04 g, 0.2 mmol) in 1:1 H₂O/EtOH (2 mL) until reaching a pH of 6.5 in order to obtain the cesium salt. This salt was concentrated under reduced pressure, redissolved in DMF (1.0 mL), and bromomethyl-*p-*tolyl ketone (0.04 g, 0.2 mmol) was added. After stirring for 12 hours, the reaction was hydrolyzed with a saturated aqueous solution of NaCl. The crude product was extracted with EtOAc, dried on Na₂SO₄ and concentrated under reduced pressure. The product was purified by flash chromatography with hexane-EtOAc (3:1) as an eluent (yield: 52%). mp: 158-160°C. ¹H NMR (300 MHz, CDCl₃) δ 2.40 (s, 3H), 5.51 (s, 2H), 7.27 (d, *J* = 8.2 Hz, 2H), 7.41-7.55 (m, 4H); 7.81 (s, 1H); 7.84-7.88 (m, 4H); 8.02 (s, 1H); 8.11-8.14 (m, 2H). ¹³C NMR (75.5 MHz, CDCl₃) δ 22.1, 66.9, 121.6, 125.5, 126.2, 127.4, 128.2, 129.1, 129.6, 129.9, 132.0, 132.3, 134.9, 138.6, 145.3, 165.9, 166.2, 192.1. HRMS (EI) calculated for C₂₃H₁₉NO₄ (M⁺): 373.1314, experimental: 373.1299.

### Example 19. Synthesis of 2-oxo-2-(4-trifluoromethyl) ethyl 3-(2,4-(dinitro)benzamido)benzoate.

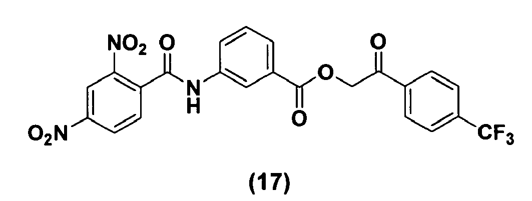

An aqueous solution of Cs₂CO₃ was added to a solution of acid **3** (0.2 g, 0.6 mmol) in 1:1 H₂O/EtOH (2 mL) until reaching a pH of 6.5 in order to obtain the cesium salt. This salt was concentrated under reduced pressure, redissolved in DMF (1.0 mL), and 4-(trifluoromethyl)phenacyl bromide (0.06 g, 0.2 mmol) was added. After stirring for 12 hours, the reaction was hydrolyzed with a saturated aqueous solution of NaCl. The crude product was extracted with EtOAc, dried on Na₂SO₄ and concentrated under reduced pressure. The product was purified by flash chromatography with hexane-EtOAc (1:1) as an eluent (yield: 34%). mp: 198-200°C. ¹H NMR (300 MHz, CDCl₃) δ 5.53 (s, 2H), 7.41-7.47 (m, 1H), 7.74 (d, *J* = 9 Hz, 2H), 7.85-7.93 (m, 3H), 8.01 (d, *J* = 6.0 Hz, 2H), 8.10-8.18 (m, 2H), 8.50 (dd, *J*, = 8.1 Hz, *J*₂ = 2.7 Hz, 1H), 8.87 (s, 1 H). ¹³C NMR (75.5 MHz, CD₃COCD₃) δ 67.9 120.8, 121.8, 125.5, 126.5, 126.8 (c, ¹*J*_{CF} = 3.5 Hz), 129.3, 129.5, 130.3, 131.3, 131.8, 135.0 (c, ²*J*_{CF} = 32.4 Hz), 138.4, 139.9, 147.9, 149.5, 163.7, 166.0, 192.6. ¹⁹F NMR (282.4 MHz, CDCl₃) δ -63.30 (s, 3F). HRMS (EI) calculated for C₂₃H₁₄F₃N₃O₈ (M⁺): 517.0733, experimental: 517.0759.

### Example 20. Synthesis of 2-phenyl-2-oxoethyl 3-(2,4-(dinitro)benzamido)benzoate.

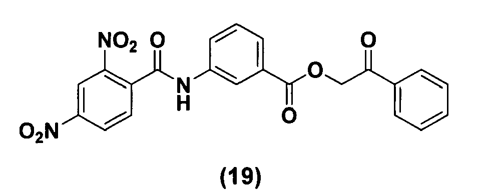

An aqueous solution of Cs₂CO₃ was added to a solution of acid **3** (0.1 g, 0.3 mmol) in 1:1 H₂O/EtO (4 mL) until reaching a pH of 6.5 in order to obtain the cesium salt. This salt was concentrated under reduced pressure, redissolved in DMF (2 mL), and 2-bromoacetophenone was added (0.07 g, 0.35 mmol). After stirring for 12 hours, the reaction was hydrolyzed with a saturated aqueous solution of NaCl. The crude product was extracted with EtOAc, dried on Na₂SO₄ and concentrated under reduced pressure. The product was purified by flash chromatography with hexane-EtOAc (2:1) as an eluent (yield: 63%). mp: 124-126°C. ¹H NMR (300 MHz, CDCl₃) δ 5.41 (s, 2H), 7.25 (d, *J* = 7.7 Hz, 1H), 7.42 (t, *J* = 7.5 Hz, 2H), 7.57 (t, *J* = 6.6 Hz, 1H), 7.72 (m, 4H), 7.85 (d, *J* = 8.7 Hz, 1H), 7.97 (s, 1H), 8.33 (t, *J* = 7.2 Hz, 1H), 8.64 (s, 1H), 8.86 (broad s, 1H). ¹³C NMR (75.5 MHz, CDCl₃) δ 61.0, 66.9, 120.2, 121.5, 125.5, 126.7, 128.1, 128.6, 129.4, 129.7, 129.9, 131.0, 133.8, 134.9, 137.6, 137.7, 146.5, 148.3, 163.2, 165.8, 193.8. HRMS (EI) calculated for C₂₂H₁₅N₃O₈ (M⁺): 449.0859, experimental: 449.0871.

### Example 21. Synthesis of methyl 3-(4-nitrobenzamido)benzoate.

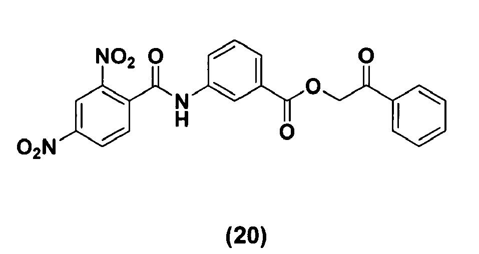

**1** (0.27 g, 2.0 mmol), EDCI (0.57 g, 3.0 mmol), HOBt (0.61 g, 4.0 mmol) and Et₃N (0.55 mL, 4.0 mmol) were added to a solution of 4-nitrobenzoic acid (0.33 g, 2.0 mmol) in dry CH₂Cl₂ (9 mL). After stirring for 16 hours, the reaction was hydrolyzed with a saturated aqueous solution of NH₄Cl. The crude product was extracted with CH₂Cl₂, dried on Na₂SO₄ and concentrated under reduced pressure. The product was purified by flash chromatography with hexane-EtOAc (4:1) as an eluent (yield: 37%).Example

### 22. Synthesis of 3-(4-nitrobenzamido)benzoic acid.

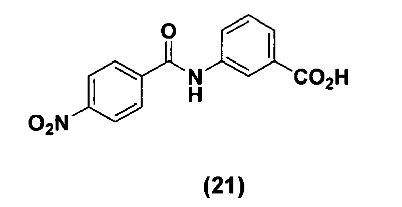

LiOH•H₂O (0.092 g, 2.19 mmol) was added to a solution of **20** (0.16 g, 0.55 mmol) in 4:1 THF:H₂O (12 mL). The reaction was stirred for 16 hours and then hydrolyzed with 1 M HCl. The crude product was extracted with EtOAc, dried on Na₂SO₄ and concentrated under reduced pressure. The product was used without subsequent purification (yield: 80%).

### Example 23. Synthesis of 2-oxo-2-(p-tolyl)ethyl 3-(4-nitrobenzamido)benzoate.

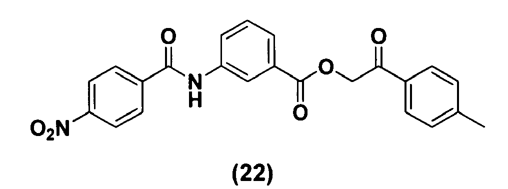

An aqueous solution of Cs₂CO₃ was added to a solution of acid **21** (0.1 g, 0.3 mmol) in 1:1 H₂O/EtOH (2 mL) until reaching a pH of 6.5 in order to obtain the cesium salt. This salt was concentrated under reduced pressure, redissolved in DMF (1.0 mL), and bromomethyl-p-tolyl ketone was added (0.7 g, 0.35 mmol). After stirring for 12 hours, the reaction was hydrolyzed with a saturated aqueous solution of NaCl. The crude product was extracted with EtOAc, dried on Na₂SO₄ and concentrated under reduced pressure. The product was purified by flash chromatography with hexane-EtOAc (4:1) as an eluent (yield: 31%). mp: 172-173°C. ¹H NMR (300 MHz, CD₃COCD₃) δ 2.29 (s, 3H), 5.58 (s, 2H), 7.25 (d, *J* = 7.8 Hz, 2H), 7.43 (t, *J* = 7.8 Hz, 1 H), 7.75 (d, *J* = 7.3 Hz, 1 H), 7.82 (d, *J* = 7.3 Hz, 2H), 8.07 (d, *J* = 8.6 Hz, 1 H), 8.16 (d, *J* = 8.3 Hz, 2H), 8.24 (d, *J* = 8.3 Hz, 2H), 8.42 (broad s, 1 H). ¹³C NMR (75.5 MHz, CD₃COCD₃) δ 22.4, 68.4, 122.9, 125.2, 126.5, 126.9, 129.5, 130.6, 130.7, 131.1, 132.1, 133.6, 141.1, 142.2, 146.3, 151.5, 165.6, 166.9, 193.1. HRMS (EI) calculated for C₂₃H₁₈N₂O₆ (M⁺): 418.1179, experimental: 418.1164.

### Example 24. Synthesis of methyl 3-(2-nitrobenzamido)benzoate.

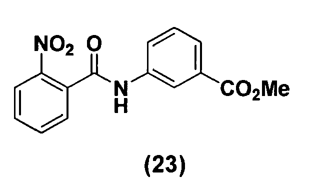

**1** (0.27 g, 2.0 mmol), EDCI (0.57 g, 3.0 mmol), HOBt (0.61 g, 4.0 mmol) and Et₃N (0.55 mL, 4.0 mmol) were added to a solution of 2-nitrobenzoic acid (0.33 g, 2.0 mmol) in dry CH₂Cl₂ (9 mL). After stirring for 16 hours, the reaction was hydrolyzed with a saturated aqueous solution of NH₄Cl. The crude product was extracted with CH₂Cl₂, dried on Na₂SO₄ and concentrated under reduced pressure. The product was purified by flash chromatography with hexane-EtOAc (4:1) as an eluent (yield: 25%). mp: 175-176°C. ¹H NMR (300 MHz, CD₃COCD₃) δ 3.90 (s, 3H), 7.53 (t, *J* = 7.7 Hz, 1H), 7.77-7.85 (m, 2H), 7.87-7.93 (m, 2H), 8.02 (d, *J* = 8.7 Hz, 1 H), 8.16 (d, *J* = 7.6 Hz, 1 H), 8.45 (broad s, 1 H). ¹³C NMR (75.5 MHz, CD₃COCD₃) δ 53.6, 122.2, 125.6, 125.9, 126.5, 130.7, 130.7, 132.6, 134.5, 135.4, 141.1, 148.6, 166.1, 167.7. HRMS (EI) calculated for C₁₅H₁₂N₂O₅ (M⁺): 300.0746, experimental: 300.0754.

### Example 25. Synthesis of 3-(2-nitrobenzamido)benzoic acid.

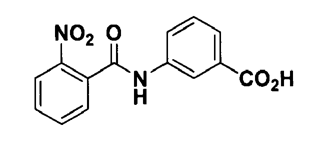

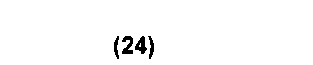

LiOH·H₂O (0.072 g, 1.72 mmol) was added to a solution of **23** (0.12 g, 0.43 mmol) in 4:1 THF:H₂O (9.5 mL). The reaction was stirred for 16 hours and then treated with 1 M HCl. The crude product was extracted with EtOAc, dried on Na₂SO₄ and concentrated under reduced pressure. The product was used without subsequent purification (yield: 75% yield). mp: 262-265°C. ¹H NMR (300 MHz, CD₃COCD₃) δ 7.39 (t, *J* = 6.9 Hz, 1 H), 7.63-7.78 (m, 4H), 7.90 (d, *J*= 8.1 Hz, 1 H), 8.01 (d, *J* = 8.7 Hz, 1 H), 8.32 (broad s, 1H). ¹³C NMR (75.5 MHz, CD₃COCD₃) δ 121.7, 124.8, 125.2, 126.1, 129.9, 131.8, 132.2, 133.8, 134.8, 140.2, 147.9, 165.3, 167.3.

### Example 26. Synthesis of 2-oxo-2-(p-tolyl)ethyl 3-(2-nitrobenzamido)benzoate.

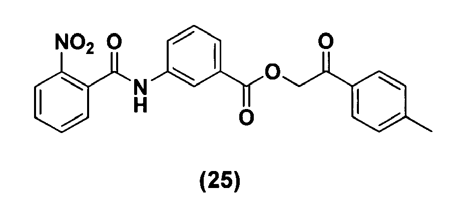

An aqueous solution of Cs₂CO₃ was added to a solution of acid **24** (0.04 g, 0.1 mmol) in 1:1 H₂O/EtOH (2 mL) until reaching a pH of 6.5 in order to obtain the cesium salt. This salt was concentrated under reduced pressure, redissolved in DMF (1.0 mL), and bromomethyl-p-tolyl ketone (0.03 g, 0.1 mmol) was added. After stirring for 12 hours, the reaction was hydrolyzed with a saturated aqueous solution of NaCl. The crude product was extracted with EtOAc, dried on Na₂SO₄ and concentrated under reduced pressure. The product was purified by flash chromatography with hexane-EtOAc (4:1) as an eluent (yield: 34%). mp: 166-168°C. ¹H NMR (300 MHz, CDCl₃) δ 2.35 (s, 3H), 5.41 (s, 2H), 7.21 (d, *J* = 7.9 Hz, 2H), 7.31 (t, *J* = 8.0 Hz, 1H), 7.41-7.47 (m, 1 H), 7.56-7.63 (m, 2H), 7.71-7.77 (m, 2H), 7.88 (d, *J*=7.9Hz, 1 H), 7.94 (d, *J* = 7.9 Hz, 1H), 8.07 (broad s, 1 H), 8.58 (broad s, 1 H). ¹³C NMR (75.5 MHz, CDCl₃) δ 21.8, 53.5, 66.5, 121.3, 124.5, 125.1, 126.2, 128.0, 128.8, 129.2, 129.6, 129.9, 130.6, 131.4, 132.6, 133.9, 137.9, 145.2, 146.1, 164.7, 165.4, 192.1. HRMS (EI) calculated for C₂₃H₁₈N₂O₆ (M⁺): 418.1169, experimental: 418.1164.

### Example 27. Synthesis of 2-(4-hydroxyphenyl)-2-oxoethyl 3-(2,4-(dinitro)benzamido)benzoate.

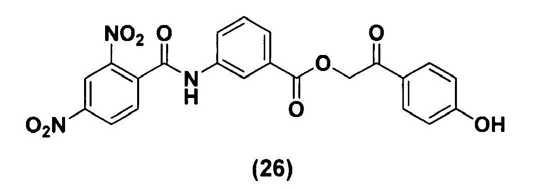

An aqueous solution of Cs₂CO₃ was added to a solution of acid 3 (0.2 g, 0.6 mmol) in 1:1 H₂O/EtOH (12 mL) until reaching a pH of 6.5 in order to obtain the cesium salt. This salt was concentrated under reduced pressure, redissolved in DMF (6 mL), and 4-(((*tert*-butyl)dimethyl)silyloxy)phenacyl bromide (0.2 g, 0.6 mmol) was added. After stirring for 12 hours, the reaction was hydrolyzed with a saturated aqueous solution of NaCl. The crude product was extracted with EtOAc, dried on Na₂SO₄ and concentrated under reduced pressure. The product was purified by flash chromatography with hexane-EtOAc (4:1) as an eluent (yield: 59%). mp 229-230°C. ¹H NMR (300 MHz, CD₃COCD₃) δ 5.53 (s, 2H), 6.85 (d, *J* = 7.9 Hz, 2H), 7.45 (t, *J* = 8.2 Hz, 1 H), 7.77 (d, *J* = 7.8 Hz, 1 H), 7.83 (d, *J* = 8.1 Hz, 1 H), 7.92 (d, *J* = 8.2 Hz, 1 H), 8.10 (d, *J*=8.8Hz, 1 H), 8.34 (s, 1 H), 8.61 (d, *J*=8.2, 1 H), 8.77 (broad s, 1 H), 9.22 (s, 1 H), 10.11 (s, 1 H). ¹³C NMR (75.5 MHz, CD₃COCD₃) δ 68.2, 117.2, 121.6, 122.4, 122.5, 125.9, 126.1, 127.2, 128.2, 130.0, 131.0, 131.9, 132.6, 139.1, 140.6, 148.8, 150.2, 164.0, 164.4, 166.7, 191.7. HRMS (EI) calculated for C₂₂H₁₅N₃O₈ (M⁺): 465.0808, experimental: 465.0814.

### Example 28. Synthesis of 2-((trimethyl)silyl)ethyl 4-(2-(bromo)acetyl)benzoate.

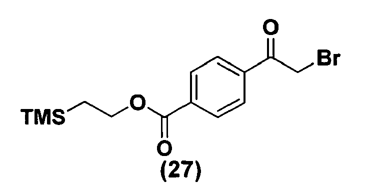

DMAP (0.01 g, 0.12 mmol), DCC (0.14 g, 0.68 mmol) and 2-trimethylsilylethanol (0.11 mL, 0.80 mmol) were added to a solution of 4-(2-(bromo)acetyl)benzoic acid (0.15 g, 0.62 mmol) in CH₂Cl₂ (5 mL) at 0°C. The mixture was stirred at room temperature for 4 hours, and then concentrated under reduced pressure. The residue was purified by flash chromatography with hexane-EtOAc (3:1) as an eluent (yield: 47%). mp 60-61°C. ¹H NMR (300 MHz, CDCl₃) δ 0.02 (s, 9H), 1.05-1.11 (m, 2H), 4.35-4.41 (m, 4H), 7.95 (d, *J* = 9.0 Hz, 1 H), 8.07 (d, *J* = 8.7 Hz, 1 H). ¹³C NMR (75.5 MHz, CDCl₃) δ -1.5, 17.3, 30.6, 63.9, 128.7, 129.7, 135.1, 137.1, 165.5, 190.7. HRMS (EI) calculated for C₁₄H₁₉BrO₃Si (M⁺): 342.0286, experimental: 342.0206.

### Example 29. Synthesis of 2-oxo-2-(4-(((2-(trimethyl)silyl)ethoxy)carbonyl)phenyl)ethyl 3-(2,4-(dinitro)benzamido)benzoate.

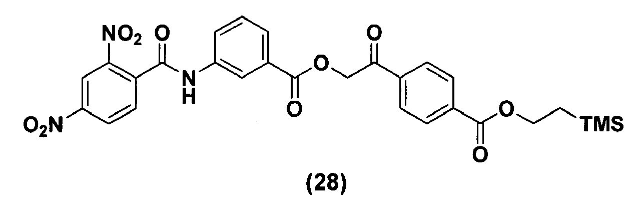

An aqueous solution of Cs₂CO₃ was added to a solution of acid 3 (0.07 g, 0.21 mmol) in 1:1 H₂O/EtOH (4 mL) until reaching a pH of 6.5 in order to obtain the cesium salt. This salt was concentrated under reduced pressure, redissolved in DMF (2 mL), and **27** (0.07 g, 0.2 mmol) was added. After stirring for 12 hours, the reaction was hydrolyzed with a saturated aqueous solution of NaCl. The crude product was extracted with EtOAc, dried on Na₂SO₄ and concentrated under reduced pressure. The product was purified by flash chromatography with hexane-EtOAc (4:1) as an eluent (yield: 56%). mp: 86-87°C. ¹H NMR (300 MHz, CD₃COCD₃) δ -0.6 (s, 9H), 1.05-1.10 (m, 2H), 4.33-4.39 (m, 2H), 5.68 (s, 2H), 7.48 (t, *J* = 7.7 Hz, 1 H), 7.78-7.82 (m, 1 H), 7.91-7.95 (m, 1H), 8.06 (s, 4H), 8.11 (d, *J* = 8.1 Hz, 1 H), 8.39 (t, *J* = 1.8 Hz, 1H), 8.61 (dd, *J₁* = 8.3 Hz, *J₂* = 2.1 Hz, 1 H), 8.79 (d, *J* = 2.3 Hz, 1 H), 10.53 (broad s, 1 H). ¹³C NMR (75.5 MHz, CD₃COCD₃) δ - 0.6, 18.6, 64.9, 68.6, 121.5, 122.5, 126.2, 127.2, 129.6, 130.0, 131.0, 131.2, 132.1, 132.6, 136.6, 139.1, 139.2, 140.6, 148.7, 150.2, 164.6, 166.7, 193.7.

### Example 30. Synthesis of 4-(2-(3-(2,4-dinitrobenzamido)benzoyloxy)-acetyl)benzoic acid.

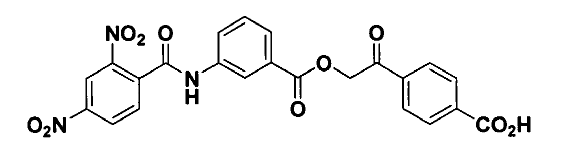

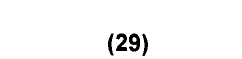

CsF (0.06 g, 0.4 mmol) was added to a solution of **28** (0.04 g, 0.07 mmol) in DMF (1 mL). The reaction mixture was stirred at room temperature for 6 hours, and then hydrolyzed with 1 M HCl. The crude product was extracted with Et₂O, dried on Na₂SO₄ and concentrated under reduced pressure. The product was purified by flash chromatography with 3% AcOH in EtOAc as an eluent (yield: 77%). mp: 284-286°C. ¹H NMR (300 MHz, MeOD) δ 5.65 (s, 2H), 7.48 (t, *J* = 8.6 Hz, 1 H), 7.84-7.88 (m, 1 H), 7.91 (s, 1 H), 7.96 (d, *J* = 8.0 Hz, 1 H), 8.02-8.11 (m, 4H), 8.33 (t, *J* = 1.8 Hz, 1 H), 8.60 (dd, *J₁* = 8.3 Hz, *J₂* = 2.2 Hz, 1 H), 8.90 (d, *J* = 2.2 Hz, 1 H). ¹³C NMR (75.5 MHz, CD₃COCD₃) δ 66.9, 119.8, 121.1, 121.3, 124.7, 125.5, 127.9, 128.3, 129.3, 129.9, 130.6, 130.9, 136.0, 137.6, 137.8, 139.0, 147.2, 148.8, 155.0, 162.7, 165.1, 192.1.

### Example 31. Inhibition of the activity of HIF by FM19G11 and its structural analogs.

According to the methodology of the cell assay based on the HIF response elements (Example 1), the intermediate compounds of the synthesis and several synthesized analogs were tested to obtain the SAR of FM19G11. The results obtained are shown in Table 2:

**TABLE 2**

| | Structure | IC50 HRE (µM) |
|---|---|---|
| **2** | 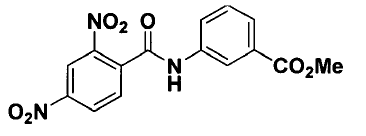 | >1 |
| **3** | 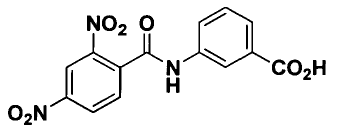 | >1 |
| **4** (FM19G11) | 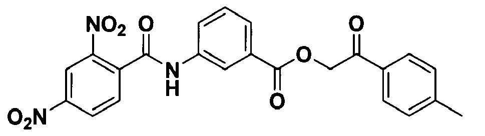 | 0.08 |
| **5** | 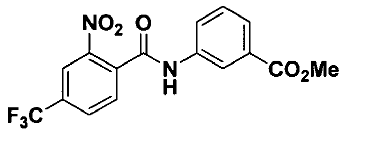 | >1 |
| **6** | 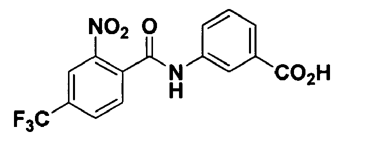 | >1 |
| **7** | 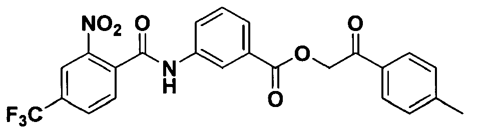 | 0.084 |
| **8** | 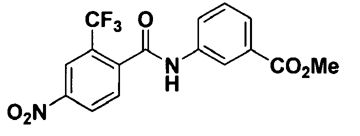 | >1 |
| **9** | 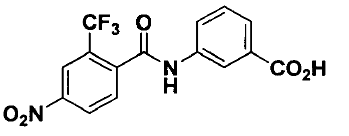 | >1 |
| **10** | 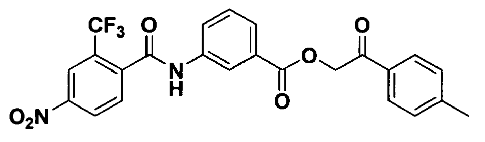 | 0.14 |
| **12** | 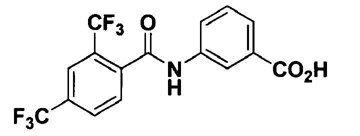 | >1 |
| **13** | 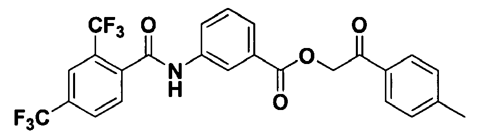 | 0.3 |
| **16** | 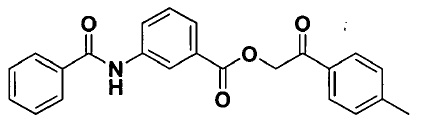 | >1 |
| **17** | 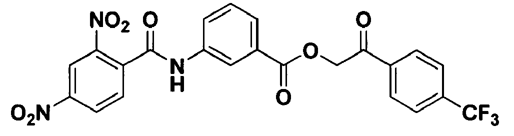 | 1 |
| **19** | 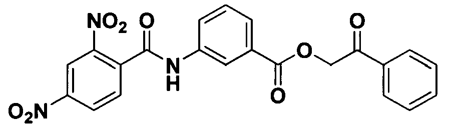 | >1 |
| **22** | 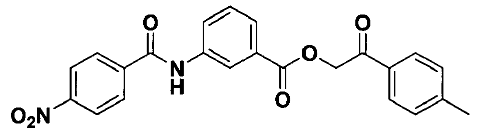 | 2.5 |
| **23** | 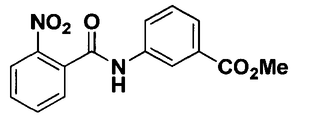 | >1 |
| **24** | 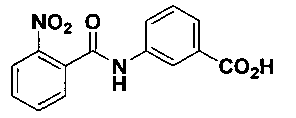 | 1.4 |
| **25** | 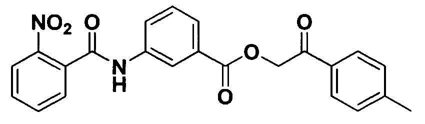 | 2.8 |
| **26** | 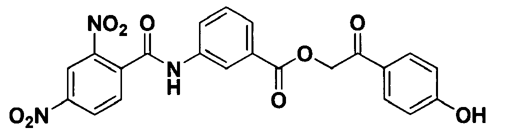 | 0.36 |
| **29** | 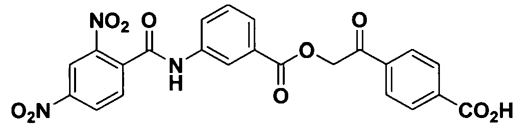 | 1.4 |

### Example 32. Inhibition of the target genes of HIF by FM19G11 in the HeLa tumor line

### 32.1 Analysis of the gene expression of the target genes of HIF-1 by real-time quantitative PCR (qPCR)

The qPCR reaction was carried out in a final volume of 20 µl containing: 1 µl of cDNA; 1 µl of 300 nM FW primer; 1 µl of 300 nM Rev primer; 10 µl of SYBR Green I and 7 µl of H₂O. It was performed in the GeneAmp 5700 Sequence Detection System of Applied BioSystems, with the following steps:
50°C 2 minutes 1 cycle
95°C 10 minutes 1 cycle
95°C 15 seconds
60°C 1 minute- 40 cycles

The quantification by means of qPCR is based on the determination of the threshold cycle (Ct) for each cDNA in each qPCR experiment. The Ct for a sample is defined as the number of minimum PCR cycles from which the fluorescence signal is greater than the minimum level of detection of the apparatus. Thus, the Ct values of different samples are used to calculate the abundance of template cDNA in each sample, since the Ct values are directly proportional to the initial amount of cDNA and are therefore the basis for calculating the levels of mRNA.

The relative expression levels of the genes are expressed by the increase of Ct (ΔCt)=Ct (Gene)-Ct(GAPDH). Due to the exponential nature of the PCR reaction, the increase of Ct is converted into linear form by means of the equation 2^{-(ΔCt)}. From the results obtained the threshold cycle (Ct) was calculated for the gene VEGF and GAPDH as an internal control. The software was Gene Amp 5700 SDS.

The analysis of the expression levels of the VEGF and GAPDH genes was carried out. The primers were designed with the Primer3 program and the sequences are:

| **Name of the primer** | **SEQ ID No** | **Sequence** |
|---|---|---|
| VEGF FW | 1 | 5' CCTCCGAAACCATGAACTTT 3' |
| VEGF Rev | 2 | 5' ATCTGCATGGTGATGTTGGAC 3' |
| GAPDH FW ^{a} | 3 | 5' CATCTTCCAGGAGCGAGATC 3' |
| GAPDH Rev ^{a} | 4 | 5' GTTCACACCCATGACGAACAT 3' |

| | | |
|---|---|---|
| a.- The GAPDH gene, used as an internal control. | | |

The levels of mRNA of VEGF were measured by qPRC in HeLa 9xHRE cells under hypoxic or normoxic conditions for 24 hours for VEGF and in the presence or absence of the compound FM19G11 at 0.3, 0.1 and 0.03 µM using GAPDH as an internal control. As observed in Figure 4, there is a decrease of the levels of mRNA of VEGF in the presence of the compound FM19G11 at the concentrations used.

### 32.2. Analysis of the protein expression of the target genes of HIF-1 by immunological analysis (Western Blot).

The HeLa9XHRE/LUC cells were lysed with a hypotonic buffer (10 mM Tris-HCl, pH 7.5, 1.5 mM MgCl₂, I mM KCIO, 2 mM DTT, 1 mM Pefabloc, 2 mM sodium vanadate, 4 µg/ml pepstatin, 4 µg/ml leupeptin, and 4 µg/ml aprotinin), preserved 20 minutes in ice and subsequently centrifuged for 10 minutes at 1,200 rpm, collecting the supernatant and preserving it at -20°C until its use. The quantification of the proteins was performed with colorimetric Coomassie blue method described by Bradford. A commercial system was used following the manufacturer's instructions (PIERCE Chem Co., Rockford III. USA). Bovine serum albumin (A-7906, SIGMA Chem Co., St. Louis MO, USA) was used to generate a standard curve (0-200 µg of protein). Five microliters of each sample were pipetted in a 96-well plate and 150 mL of the Coomassie solution were added and it was maintained in darkness for 5 minutes at room temperature and the absorbance at OD 595 nm was measured in a spectrophotometer. By means of the Graph-Pad statistical program, the standard line was calculated and the values were extrapolated for each of the samples. The final values were expressed as mcg/mcl of protein. The electrophoresis of proteins of the extract was carried out in polyacrylamide gels under denaturing conditions (SDS-PAGE). A commercial mixture of proteins for low molecular mass polypeptides (Amersham) was used as a molecular size standard. The transfer of the proteins from the polyacrylamide gels to a nitrocellulose membrane (Millipore) was carried out using a Trans-Blot SD transfer system (Bio-Rad). The second antibody is coupled to the peroxidase enzyme, the activity of which was used to detect the antigen-antibody binding. The high-sensitivity chemiluminescence system (ECL by western blot analysis, Amersham-Pharmacia) was used for developing. The membranes were incubated for 2 minutes in a recently prepared mixture of detection solutions 1 and 2 of the ECL immunodetection system in a 1:1 ratio, according to the manufacturer's recommendations. The primary antibodies used were: Monoclonal anti-HIF-1α (BD Biosciences) Monoclonal anti-HIF-1α (BD Biosciences), monoclonal anti-HIF-2α (Abcam, Cambridge Science Park) 1:500 dilution, monoclonal anti-PHD-3 1:1000 dilution (Abcam, Cambridge Science Park), monoclonal anti-VEGF (R&D systems) and GAPDH 1:5000 dilution as a housekeeping protein. The secondary antibodies used were monoclonal anti-mouse IgG antibody for HIF-1α, donkey anti-rabbit IgG for HIF-2□ and PHD-3 and GAPDH, donkey anti-goat IgG for VEGF, all of them at a 1:5000 dilution.

Figure 5 shows the inhibition of the target proteins PHD3, HIF1α and HIF2α by FM19G11 in the HeLa9XHRE-LUC. A. cell line, by Western blot analysis. The HeLa 9xHRE cells were subjected under hypoxic or normoxic conditions for 9 hours, in the presence and absence of the compound FM19G11 (0.3 mM). The anti-HIF-1α, anti-HIF-2α and anti-PHD3 antibodies were used. GAPDH was used as an internal control. A decrease is observed in the expression of HIF 1α and 2α, as well as of PHD3 in the presence of the compound FM19G11.

### Example 33. Inhibition of the activity of HIF in the C17.2 stem cell

C17.2 were cultured in an undifferentiated state using a high-glucose Dulbecco's modified medium (DMEM) supplemented with 10% fetal calf serum, 5% horse serum, 1% glutamine (2 mM), 1% penicillin/streptomycin/fungizone. The cells were seeded at 1500 cells/cm², both for normoxic conditions and for hypoxic conditions. The culture of cells in normoxia was carried out in an incubator with 20% O₂, 5% CO₂, 37°C and the standard percentage of humidity (94%). The conditions of low concentration of O₂ were created by using a hypoxia chamber (Invivo₂ 400, Ruskinn Life Sciences), with the mixture of gases 1% O₂, 5% CO₂, and 94% N₂ at 37°C. The cells were maintained under the normoxic and hypoxic conditions at different times 3, 6, 9, 24, 48 hours. They were subsequently collected and the proteins and the mRNA were extracted using a commercial kit, NucleoSpin RNA/protein (Macherey Nagel) according to the manufacturers' instructions. The concentrations of 19G11 used during the studies conducted are 125, 250 and 500 nM. The concentrations were used according to the results obtained after evaluating the toxicity of C17.2 due to the presence of the compound, since IC₅₀ >1 µM.

Figure 6 shows the inhibition of the target genes of HIF1α by FM19G11 in the C.17.2 neural stem cell line. The compound FM19G11 was used at different nanomolar (nM) concentrations both under hypoxic (Hx refers to hypoxic conditions at 1% O₂) and normoxic (20% O₂) conditions.

### Example 34. Restoration of the repair (Mismatch Repair-MMRs) proteins in murine and human stem cell models.

According to the results obtained, the C17.2 neural stem cell subjected to hypoxia experienced repression in the DNA repair (mismatch repair, MMR) system. Figure 32 shows the western blotting performed with nuclear protein extracts of C17.2 neural stem cells cultured under normoxic or hypoxic conditions in the presence of 19G11 (500 nM) or carrier (DMSO) in which 19G11 was dissolved. The monoclonal anti MLH1 and MSH6 antibodies were used in the analysis, Nx referring to normoxia and Hx to hypoxia.

Figure 32 shows how 19G11 is capable of reestablishing the expression of these repair proteins which could prevent stem cells proposed in cell therapy from experiencing an unwanted onco-transformation. The recovery of the expression of MMRs is not due to the carrier (DMSO), since even 19G11 rescues from the possible repression caused by the latter. The increase of the expression of the repair genes not only takes place in hypoxia but also under normoxic conditions.

These studies were extrapolated to other human stem cells such as bone marrow or dental pulp mesenchymal cells. Thus, Figure 7 shows the Western blotting performed with nuclear protein extracts of mesenchymal stem cells cultured in normoxia or hypoxia in the presence of 19G11 (500 nM). The monoclonal anti MLH1 and MSH6 antibodies were used in the analysis. Figure 7A refers to the results obtained in bone marrow mesenchymal stem cells, the conditions of the experiment being 1-Nx 3h; 2-Hx 3h; 3-Hx 3h+19G11; 4-Hx 18h; 5-Hx 18h+19G11;6- Hx 24h; 7-Hx 24h+19G11, wherein Nx refers to normoxia and Hx refers to hypoxia. Panel B refers to the results obtained in dental pulp mesenchymal stem cells, the conditions of the experiment being 13-Nx 72h; 14-Nx 72h+19G11; 15-Hx 72h; 16-Hx 72h+19G11; Nx.-normoxia; Hx.- hypoxia

Thus, a recovery such as the one described for C17.2 was also observed in this case.

Considering the previously published scientific results, it is known that after repression, the expression of the repair genes can be recovered by a histone deacetylase inhibitor known as Trichostatin A (TSA) (Cameron EE, et al. "Synergy of demethylation and histone deacetylase inhibition in the re-expression of genes silenced in cancer" Nat Genet. 1999 Jan;21(1):103-107). This drug is capable of increasing the degree of acetylation of the histones located in the promoter region of those genes, this confers to chromatin a conformation which is accessible for the transcription factors regulating these genes. Thus, the results obtained by means of using an anti-acetyllysine antibody show that 19G11 increases the overall degree of acetylation of different proteins and rescues the expression of MMRs even more effectively than TSA itself. The fact that a drug recovers genes which slow down or prevent cancer makes it a possible candidate for an antitumor agent; in this sense, TSA is a drug which is currently in a preclinical phase for breast cancer treatment (Vigushin DM, et al. "Trichostatin A is a histone deacetylase inhibitor with potent antitumor activity against breast cancer in vivo", Clin Cancer Res. 2001 Apr;7(4):971-6.).

Figure 32 shows the Western blotting performed with nuclear protein extracts of C17.2 or neural stem cells cultured in normoxia or hypoxia in the presence of 19G11 (500 nM) or carrier (DMSO) in which 19G11. The monoclonal antibodies anti-acetyllysine, anti-MLH1 and MSH6 as well as against beta-actin used as a loading control were used in the analysis; wherein Nx refers to Normoxia and Hx to hypoxia. The results show that FM19G11 is capable of recovering the expression of the MMRs more effectively than TSA.

### Example 35. Regulation by FM19G11 of the expression levels of target genes of HIF in a primary culture of ependymal adult stem cells (epNSCs)

### 35.1 Primary culture of adult spinal cord ependymal neural progenitors (epNSC):

Adult Wistar rats of between 2 to 3 months of age were used (the animals were handled following the mode of action which complies with section 2.1 (acceptable methods of euthanasia) of the document prepared for the DGXI of the European Commission regarding the protection of animals used for experimentation and other scientific purposes (No. L358, ISSN 0378-6978) and after the approval of the protocols by the Committee for Animal Care and Wellbeing of the Centro de Investigación Principe Felipe, Valencia, SPAIN). Once the animals were sacrificed, the dorsal part of the spinal cord was dissected and the tissue was maintained, at 4°C, in washing medium (DPBS-glucose) for its processing. To reduce the percentage of connective tissue cells the meninges as well as the most superficial blood vessels were removed. Under sterility conditions, the medullary tissue was cut into portions close to 1 mm³, incubated for 10 minutes at 37°C in washing medium, and washed twice, using the same medium, by mild centrifugation (1000 rpm, 10 min). The tissue was finally homogenized in complete medium [DMEM/F12 (Invitrogen) supplemented with 5 mM HEPES, 3 mM NaHCO_{3,} 0.6% glucose, 20 nM progesterone, 3 mM Na selenite, 100 µg/ml apotransferrin, 10 µM putrescine, penicillin-streptomycin, L-glutamine, 25 µg/m insulin, EGF (20 ng/ml), FGF (20 ng/ml), 2 g of BSA, heparin 357 U/ml]. The tissue suspension was repeatedly passed through siliconized glass pipettes, reducing the diameter thereof with a Bunsen burner, until obtaining a cell suspension. Seven days later, the neural progenitors formed neurospheres (NSCs). The NSCs floating in the primary culture were collected, separating them from the cell remains, dead cells and other cell types adhered to the culture plate (e.g., fibroblasts, microglia, astroglia). The NSCs were then cultured in low attach plates (Nunc), which prevents their adhesion to the plastic and consequently their differentiation, and were maintained at 37°C in an incubator with 94% humidity and 5% CO₂ (normoxic conditions). The low O₂ concentration conditions (hypoxic conditions) were created using a hypoxia chamber (Invivo₂ 400, Ruskinn Life Sciences), with the mixture of gases 1% O₂, 5% CO₂, and 94% N₂ at 37°C. The NSCs were maintained under the normoxic and hypoxic conditions at different times 3, 6, 9, 24, 48 hours in the presence or absence of the compound 19G11, at a final concentration of 500 nM (dose below its IC₅₀ >1 µM and more effective in the C17.2 cell line).

### 35.2. Detection of the transcriptional expression levels of VEGF, PHD3 and GAPDH:

Total RNA was isolated using the commercial kit RNAeasy (Qiagen) and following the manufacturer's instructions. 1 µg of total RNA of each sample was retrotranscribed using the commercial kit Reverse Transcription Reagent (Applied Biosystem). The quantitative polymerase chain reaction (qPCR) was carried out using Taqman probes and primers designed by Applied Biosystem for rVEGF (Ref Rn00582935_m1), rPHD3 (Ref Rn00571341_m1) and rGAPDH (Ref Rn01775763_g1). The qPCR was developed in duplicate in the 5700 Sequence Detection System (Applied Biosystems) in a final volume of 20 µl, using TaqMan Universal PCR Master Mix (Applied Biosystems) 1x, 40 ng of cDNA (equivalent amount of total RNA), and 250 nM of each probe and primer. The program chosen for the amplification was: 50°C, 2 min x 1, 95°C, 10 min x 1, 95°C 15 seconds and 60°C 1 minute x 40. The expression levels of VEGF and PHD3 were expressed as the increase of Ct with respect to the expression levels of GAPDH (constitutive expression gene): Ct (ΔCt)=Ct (VEGF or PHD3)-Ct (GAPDH). Each ΔCt under hypoxic conditions (Hx) was normalized with its value under normoxic conditions (Nx): ΔΔCt=Ct(hx)-Ct(Nx). Due to the exponential nature of the PCR reaction, the increase of Ct was converted into linear form by means of the equation 2^{-ΔΔCt}.

Figure 8 shows the study of the expression levels of PHD3 and VEGF (by quantitative RT-PCR), in the primary culture of epNSCs, under hypoxic conditions (white bars), it shows a time-dependent increase from 3 to 24 hours in the first case and, up to 9 hours in the second, which reverts after said times up to 48 hours with respect to the levels in normoxia. In the presence of 500 nM FM19G11 (black bars) the increase of both PHD3 and VEGF is significantly inhibited from 6 to 24 hours of exposure in hypoxia..

### Example 36. Regulation by FM19G11 of the expression levels of target genes of HIF in a primary culture of adult human dental pulp stem cells.

### - Primary culture conditions.

The culture medium used was Low-Glucose DMEM supplemented with 10% fetal bovine serum. The human dental pulp mesenchymal stem line [Gronthos S, et al., "Postnatal human dental pulp stem cells (DPSCs) in vitro and in vivo" Proc Natl Acad Sci U S A, 2000 Dec 5; 97(25):13625-30.; Gronthos S et al.,." Stem cell properties of human dental pulp stem cells" J Dent Res, 2002 Aug; 81(8):531-5], obtained from human third molars from adults (19-29 years of age), was provided by the Cardioregeneration Dept. of the CIPF. The cells were seeded at a density of 2000 cells/cm², and when they reached a 90% confluence they were subcultured again by means of trypsinization.

### -Study of the activity of FM19G11 on the expression of HIF-1alpha.

The dental pulp cells were seeded at 2000 cells/cm² in 21 cm² plates. When the cultures reached subconfluence, the compound FM19G11 was added at the concentration of 0.5 µM, for 24 hours, in parallel assays under physical normoxic and hypoxic (1% O₂, 94% N₂ and 5% CO₂ conditions. The proteins were then extracted in a lysis buffer (50 mM tris-HCL pH8, 150 mM NaCl, 0.02% NaN₃, 0.1% NP40, 0.5% DOC, 1x protease inhibitor (Complete, Sigma-Aldrich)), they were quantified by the Bradford technique, and 30 µg were loaded in a 12% SDS-PAGE gel to determine the expression of HIF-1alpha (120 KDa). For the detection, primary anti-HIF-1α (BD Biosciences) and anti-GAPDH (Trevigen) antibodies, followed by secondary goat anti-mouse (for HIF-1α) and donkey anti-rabbit (for GAPDH) antibodies conjugated with HRP (Amersham, 1:5000 dilution) and the chemiluminescent detection system (ECL; Amersham, 1:5000 dilution) and autoradiography film (Kodak) (see Figure 9 A) were used.

### -Detection of the transcriptional expression levels of VEGF, COX-2 and GAPDH:

Total RNA was isolated using the commercial kit RNAeasy (Qiagen) and following the manufacturer's instructions. 1 µg of total RNA of each sample was retrotranscribed using the commercial kit Reverse Transcription Reagent (Applied Biosystem). The quantitative polymerase chain reaction (qPCR) was carried out using Taqman probes and primers designed by Applied Biosystem for hsVEGF (Ref HS00173626_m1), hsCOX-2 (Ref Hs00153133_m1) and hsGAPDH (Ref HS 99999905_m1). The qPCR was developed in duplicate in the 5700 Sequence Detection System (Applied Biosystems) in a final volume of 20 µl, using TaqMan Universal PCR Master Mix (Applied Biosystems) 1x, 40 ng of cDNA (equivalent amount of total RNA), and 250 nM of each probe and primers. The program chosen for the amplification was: 50°C, 2 min x 1, 95°C, 10 min x 1, 95°C 15 seconds and 60°C 1 minute x 40. The expression levels of VEGF and COX-2 were expressed as the increase of Ct with respect to the expression levels of GAPDH (constitutive expression gene): Ct (ΔCt)=Ct (VEGF or PHD3)-Ct (GAPDH). Each ΔCt under hypoxic conditions (Hx) was normalized with its value under normoxic conditions (Nx): ΔΔCt=Ct(hx)-Ct(Nx). Due to the exponential nature of the PCR reaction, the increase of Ct was converted into linear form by means of the equation 2^{-ΔΔct}.

Figure 9 shows the induction of the expression of HIF-1α after 18 hours of exposure to physical hypoxia. As can be observed in Figure 9 A, the compound FM19G11 at the concentration of 0.5 µM inhibited the expression of HIF-1α, GAPDH was used as an internal loading control. As shown in Figure 11 B the study of the expression levels of VEGF and COX-2 (by quantitative RT-PCR) at 3, 6 and 18 hours, in the culture of dental pulp stem cells, under hypoxic conditions (white bars), showed a maximum expression at 6 hours for both target genes of HIF with respect to the levels in normoxia. In the presence of FM19G11 at 0.5 µM under hypoxic conditions (black bars) the increase of both VEGF and COX-2 is inhibited at 6 and at 18 hours (Figure 9B). However, the compound did not cause any effect under normoxic conditions (gray bars).

### Example 37: Regulation by FM19G11 of the decrease of death and apoptosis, occurring in hypoxia in a primary culture of adult human dental pulp stem cells.

The apoptosis studies were conducted from cell cultures subjected to normoxic and hypoxic conditions in the presence and absence of the compound at 0.5 µM. The presence of phosphatidylserine in the outer part of the plasma membrane, characteristic of apoptotic processes, was detected using Annexin V (fluorescent dye Annexin V-FITC Apoptosis Detection Kit (BD Pharmingen, BD Biosciences) according to the manufacturer's instructions. The cells subjected to the different conditions were incubated with Annexin V for 30 minutes at 4°C. The cells were then incubated with propidium iodide (PI) and analyzed in a FACScalibur flow cytometer (Beckman Coulter). PI-/AnnexinV+ cells were defined as early apoptotic cells, PI+/AnnexinV+ cells as late apoptotic cells and PI+/AnnexinV- cells as dead cells. Therefore, the number of total apoptotic cells will be the sum of early and late apoptotic cells.

As can be observed in Figure 10, the hypoxia reduces the number of total apoptotic cells at 72 hours and the compound FM19G11 at 0.5 µM is capable of reverting said effect (Figure 10 A). Furthermore, the compound also reverts the decrease in death occurring in hypoxia (Figure 12 B). Finally, Figure 10 C shows, in hypoxia, the decrease of expression of the tumor suppressor p53 involved in cell death and apoptosis, and the recovery of p53 when treating cells in hypoxia with FM19G11.

### Example 39: Regulation by FM19G11 of the expression of undifferentiated state markers in a primary culture of adult ependymal stem cells (epSPC) in hypoxic and normoxic conditions

The primary culture of adult spinal cord ependymal neural progenitors (epSPC) was performed according to the conditions already set forth in Example 35.1.

Detection of the transcriptional and transductional expression levels of genes responsible for maintaining the undifferentiated state, Sox2, Oct4, Nanog, TGF-alpha: Total RNA was isolated using the commercial kit RNAeasy (Qiagen) and following the manufacturer's instructions. 1 µg of total RNA of each sample was retrotranscribed using the commercial kit Reverse Transcription Reagent (Applied Biosystem). The semiquantitative polymerase chain reaction (sqPCR) was carried out using the primers detailed below, for the amplification of the messenger RNAs of Sox2, Oct4, Nanog, TGF-alpha and GAPDH. The sqPCR was developed in an Eppendorf thermal cycler in a final volume of 50 µl, using 1 U of Taq Polymerase, 40 ng of cDNA (equivalent amount of total RNA), and 300 nM of each primer. The program chosen for the amplification was: [95°C, 3 min] x1, [55°C, 30 sec, 72°C, 5 min, 95°C 30 sec] x 35 and 72°C 10 min. The expression levels were viewed in a 1% agarose gel in 1x TAE buffer stained with ethidium bromide. The changes of expression due to the loading difference were corrected by the differential expression of the GAPDH constitutive expression gene. The experiments were performed in triplicate (with cells from three different rats).

In relation to the oct3/4 and oct 4 nomenclature, it is emphasized that, taking into account that the isoform 3 does not exist in rats and that, however, in humans the antibodies or determination of the expression thereof is difficult to clarify for each of the isoforms, the correct name for this gene in humans is oct ¾, and in rats Oct 4.

| **Name of the primer** | **SEQ ID No** | **Sequence** |
|---|---|---|
| rSox2 sense | 5 | 5' GGATGGTTGTCTATTAACTT 3' |
| rSox2 antisense | 6 | 5' CTCCATCATGTTATACATGC 3' |
| rOct4 sense | 7 | 5'GCTGGACACCTGGCTTCAGA 3' |
| rOct4 antisense | 8 | 5'TCTGAAGCCAGGTGTCCAGC 3' |
| rGAPDH sense | 9 | 5' AGCGGCATCTTCTTGTGCAG 3' |
| rGAPDH antisense | 10 | 5' TTGCCGTGGGTAGAGTCATA 3' |
| rNanog sense | 11 | 5'TAGCCCTGATTCTTCTAGCA 3' |
| rNanog antisense | 12 | 5'TACACCAGGACTGAGTGCTC 3' |
| rTGF-α sense | 13 | 5'TCGCTCTGCTAGCGCTGGGTATCCT |
| rTGF-α antisense | 14 | 5'- ACTGTCTCAGAGTGGCAGCAGGCAG |

The levels of proteins of the undifferentiated state markers Sox2, Oct4, Nanog and Notch1 were determined by immunological analysis (Western Blot) following the methodology described in Example 35.1. The primary antibodies for the detection of Sox2, Oct4, Nanog and Notch1 were acquired from Abcam (UK) and the expression of beta-actin (antibody acquired in Sigma Aldrich Co., UK) served as a loading control.

The presence for 48 hours of the compound FM19G11 at a concentration of 500 nM (Figure 11) in a culture exposed to a low level of oxygen, 1% O₂, shows a down-regulation of the expression at the level of the messenger RNA of Sox2 and Oct4 induced by hypoxia, as well as of their target genes, Nanog and TGF-α respectively.

Increasing concentrations of FM19G11 in low oxygen conditions (Figure 12), 1% O₂, inhibits in a dose-dependent manner the expression of both proteins, Sox2 and Oct4:
Under normoxic or high oxygen level conditions (~20%), however, the presence of FM19G11 (500 nM) for 48 hours (Figure 13) significantly induces the expression of both Sox2 and Oct4 and of other undifferentiated state markers of epSPCs, such as Nanog and Notch1.

### Example 40: Regulation by FM19G11 of the expression levels of Sox2 and Oct3/4, the genes responsible for maintaining the undifferentiated state in human embryonic stem cells (hESCs)

Culture of the H9 human embryonic cell (hESC) line: To maintain and propagate the hESC line (up to no more than 70 passes), the hESCs are cultured on a monolayer of mouse fibroblasts inactivated with mitomycin C (Sigma Aldrich, Sweden) (Thomson *et al.,* [1998]). To study the transcriptional changes of SOx2 and Oct3/4, the undifferentiated colonies of hESCs were transferred, by mechanical dissociation, to a culture plate coated with a thin layer of Matrigel® and are cultured in the presence of conditioned medium. The conditioned medium is obtained from the culture of confluent and inactivated mouse fibroblasts, seeded on plates coated with gelatin (0.1 %; Sigma).

### Detection of the transcriptional expression levels of Sox2, Oct3/4 and GAPDH:

Using the experimental conditions described in Example 35, the procedure was the same including in this case the TaqMan probes for human Sox2, Oct3/4 and GAPDH (Sox2: Hs01053049_s1; Oct3/4:Hs01895061_u1; GAPDH: Hs99999905_m1). As shown in Figure 14, the presence of the compound FM19G11 at a concentration 500 nM, after 48 hours of treatment and in low oxygen culture conditions, hypoxia (1% O₂), significantly inhibits the expression of both Sox2 and Oct3/4. The expression levels of both messenger RNAs are induced under hypoxic conditions versus normoxic conditions (high oxygen culture conditions ~ 20% O₂),

### Example 42: The presence of FM19G11 allows the progress of the process of differentiation of epSPCs into oligodendrocytes under hypoxic conditions

The hypoxic condition blocks stem cell differentiation, and it is well acknowledged that this process is due to the activity of the HIF proteins. The exposure of ependymal stem cells to a low concentration of oxygen (1% O₂) prevents the progress of differentiation aimed at the oligodendrocyte glial strain, clearly shown by both the morphological characteristics of the culture and by the low expression of typical markers of this cell strain (NG2, RIP, 04). The presence of the compound FM19G11 (500 nM), inhibitor of both the HIF-1 and -2 alpha proteins, recovers the expression levels of the oligodendrocyte markers present under normoxic conditions as well as the phenotype thereof.

The analysis of the expression of the cell markers Rip, NG2 and 04 in the presence or absence of FM19G11 under normoxic and/or hypoxic conditions was carried out by immunocytochemical techniques (Figure 15). After fixing the cell culture with paraformaldehyde (4%), it was permeabilized with Triton (0.05%) for 10 minutes, and after blocking non-specific bindings with fetal bovine serum (1%), it was incubated with the corresponding antibodies (acquired from Chemicon, USA). Secondary antibodies conjugated with different fluorochromes, rhodamine in rabbit IgG and fluorescein in mouse IgG, reveal the specific labeling of the primary antibodies. DAPI (Sigma Aldrich Co, UK) was used for the counterstaining of the nuclei.

### Example 43: The presence of FM19G11 improves the self-renewal of ependymal stem cells, epSPCs, and maintains the undifferentiated state thereof under normoxic conditions

Under standard oxygen concentrations, ~20%, the presence of the compound FM19G11 (500 nM, 48 hours) increases the rates of proliferation of ependymal stem cells, epSPCs (Figure 16). However, this situation is not reproduced under hypoxic conditions (1% O₂). The accumulation of ATP, a metabolite directly related to the increase of metabolic activity and therefore with the proliferative activity, was determined with the commercial CellTiter-Glo® luminescent cell viability kit (Promega, Madison, WI).

The presence of the compound FM19G11 under normoxic conditions (20% O₂) favors the formation of neurospheres from a population of ependymal stem cells (epSPC) disaggregated in an individualized manner (Figure 16)

### Example 44. Toxicity studies of FM19G11 against a cell panel.

The cytotoxicity studies were conducted in the HeLa 9x-HRE-Luc, PRC3, HeLa, MCF-7 and MDA-MB 435-S cell lines. The cell lines were cultured in DMEM medium supplemented with 10% inactivated fetal bovine serum, penicillin (50 IU/ml), streptomycin (50 µg/ml) (Invitrogen-Life Technologies, Inc., Carlsbad, CA). The cells were cultured at 37°C with a humidified atmosphere containing 5% CO₂. The cytotoxic effect of the compounds was assayed in the dose range between 0.5 to 30 µM. The cells were seeded at 5,000 cells/well in the case of HeLa 9x HRE and in transparent 96-well plates, 50 µl per well, they were cultured for 72 hours at 37°C with a humidified atmosphere containing 5% CO₂ subsequently carrying out the colorimetric MTS assay according to the supplier's instructions (Cell Titer 96^{®} AQᵤₑₒᵤₛ Non-Radioactive Cell Proliferation Assay Technical Bulletin TB169, Promega Corporation). The live and metabolically active cells were capable of reducing MTS to formazan, a product soluble in the culture medium. The absorbance of the formazan can be measured at 490 nm and is directly proportional to the number of live cells in the culture. As can be seen in Figure 18, the lethal dose 50 at concentrations greater than 500 times the IC50 - in the range of 100 nM- of FM19G11 was not reached in any of the cell lines used in the assay of the HIF response elements.

### EXAMPLE 45. Toxicity study of FM19G11 against zebrafish (Danio rerio) and medaka (Oryzias latipes) embryos.

Fish embryos have a series of characteristics which make them suitable models for assays of evaluation of drugs in vertebrate animals (Zon, L.I. & Peterson, R (2005). "In vivo drug discovery in the zebrafish". Nature Rev Drug Disc, 4, 35-44; Medaka, Oryzias latipes. Development of test methods and Suitability of Medaka as Test Organism for Detection of Endocrine Disrupting Chemicals, February 2003, Ministry of Environment, Japan Chemicals Evaluation and Research Institute, Japan). On one hand, they are transparent and have an *ex utero* development, therefore the induced phenotypes can be easily observed, they are much more inexpensive and easier to maintain, the results furthermore being obtained more quickly, than mice which are the most used model in toxicity assays. The toxicity of the compound has been tested in two fish models, zebrafish and medaka, firmly established as animal models for studying development in vertebrates.

In the first place, for the zebrafish embryos, various experiments were performed with 10, 6 and 4 embryos per well in a volume of 100 microl, in early (between 1 and 8) and late (more than 20) stages of 1 x Yamamoto buffer, culturing the embryos at 26°C for 24 hours, and the compound FM19G11 was subsequently added at concentrations of 10 microM and 50 microM with negative controls without additives to analyze the endogenous mortality of the embryos and with 1% and 5% DMSO (solvent of the compound) to analyze the mortality of the solvent. In all the cases the solvent induced a high lethality between 60% and 90%.

The same experiments were performed using medaka embryos for which DMSO has not lethal effects at concentrations less than 5%. Six embryos per well, a final volume of 100 µl and independent experiments with embryos in blastula and neurula stages were used. At the used concentrations of 10 microM and 50 microM no significant lethality (greater than 10%) was observed. The results obtained showed that FM19G11 does not affect the viability of the embryos- all of them remained viable - visible morphological alterations which could be interpreted as organotoxicity for the development of the embryo were not observed.

To prevent the possibility that FM19G11 may not be traversing the protective layer of the embryo (chorion), the compound at a concentration of 0.5 mM was microinjected into the chorionic space (between the embryo and the chorion). Experiment 1: n=15, 20% induced mortality, DMSO control = 0%. The result was the same, no anomaly being observed in the development of the embryo, nor any effect on the viability of the injected embryos. The images of Figure 19 show medaka embryos subjected to the treatment with FM19G11 at 50 microM for 4 days (A) 10 medaka embryos in a well of a multi-well plate; B and C, photos of embryos treated with 50 microM FM19G11 for 1 day; and D a photo of a medaka embryo which was not treated with the compound.

### EXAMPLE 46. Analytical method for the detection of FM19G11

The analysis of the compound FM19G11 was conducted by HPLC using an RP18 Lichrocart 250-4 (5 µm) analytical column, with an isocratic method 59:41 MeCN/H₂O, and with a flow of 0.8 mL/min. The retention time of the compound is 12.7 minutes (Figure 20).

### EXAMPLE 47: Stability of FM19G11 in bovine serum

The stability of FM19G11 was determined by incubation in bovine serum (stabilized with 10% PBS) and subsequent analysis by HPLC. Samples with concentrations of 0.5 mM and 1.0 mM in the serum were prepared and aliquots of 100 µL were extracted at different incubation times (0, 0.5, 1, 2 and 3 hours). The samples were preserved at -80°C. To carry out the extraction in each sample, 800 µL of H₂O, 900 µL of CHCl₃ and a drop of isopropanol were added, the mixture was then centrifuged and the supernatant was removed. The extract was concentrated and the residue was suspended in the mobile phase used for the analysis by HPLC (59:41 MeCN/H₂O). 25 µL of each sample were injected (Figure 21).

### Example 48: FM19G11 triggers a DNA damage response in human colon cancer cells (HT29)

According to the results obtained by the Western blotting technique, FM19G11 triggers a DNA damage response (DDR) in human colon cancer cells (HT29). The treatment of the HT29 cells with different concentrations of FM19G11 (0.25 or 0.5 µM) after 24 hours of treatment efficiently increased the phosphorylation of proteins involved in the initial DDR phase (H2AX, MLH1, MSH6), damage signal mediators (BRCA1), translators (ATM, ATR) or effectors of the DNA damage response (CHK1, CHK2, p53) (Figure 22).

Figure 22 shows how FM19G11 triggers a DNA damage response in human colon cancer cells (HT29). An increase in the activation by greater phosphorylation of kinases included in the key signaling pathways in DNA damage response, specifically the pathway known as ATM/ATR, that of DNA damage repair (MMR, BRCA1, H2AX), as well as at the points of control of the cell cycle (CHK1, CHK2), were detected in the HT29 tumor cells treated with FM19G11 (0.5 µM). The treatment with 6-TG (5, 10, 25 µM) or etoposide (0.1, 1, 10 µM) was included as a control as well as for the comparison of proteins which are activated in response to damage in the single strand (ATR-CHK1) or double strand (ATM-CHK2) of DNA respectively.

### Example 49: Activation kinetics of proteins involved in DDR

In addition to the DNA damage observed for HT29, it was evaluated if FM19G11 triggers the previously described DDR in other colon cancer tumor cell lines, specifically in HCT116/p53^{+/+} and HCT116/p53^{-/-} cells. The p53 tumor suppressor gene is mutated in 50% of the human tumors and is a key element in the response to genotoxic stress since it activates the transcription of genes which are important for DNA repair or cell cycle arrest.

To determine the degree of involvement of p53 in the possible effects caused by FM19G11, a HCT116 colon cancer cell line with expression of p53 and the same HCT116 line in which p53 was inactivated by homologous recombination were used (Bunz, F, Dutriaux, A, Lengauer, C, Waldman, T, Zhou, S, Brown, JP et al., (1998) Requirement for p53 and p21 to sustain G2 arrest after DNA damage. Science 282: 1497-501; Bunz, F, Hwang, PM, Torrance, C, Waldman, T, Zhang, Y, Dillehay, L et al., (1999) Disruption of p53 in human cancer cells alters the responses to therapeutic agents. J Clin Invest 104: 263-9.). The HCT116/p53^{+/+} and HCT116/p53^{-/-} cells were exposed to FM19G11 (0.5 µM) at different times, the phosphorylation (activation) pattern of the ATR protein and of p53 which can be phosphorylated by ATR (Tibbetts, RS, Brumbaugh, KM, Williams, JM, Sarkaria, JN, Cliby, WA, Shieh, SY et al., (1999) A role for ATR in the DNA damage-induced phosphorylation of p53. Genes Dev 13: 152-7.), in p53-proficient HCT116 cells, as well as ATR in p53-deficient cells being analyzed (Figure 23). A quick increase (after 1 hour) of the phosphorylation of ATR in both HCT116 cell lines by treatment with FM19G11 (0.5 µM), with a subsequent decrease throughout the different stimulation times, was detected. This quick activation of ATR coincides with that of p53 in HCT116/p53^{+/+} cells after 1 hour of treatment with FM19G11 (0.5 µM), but the activation of p53 remains virtually on the expression of cells without drug, for the different times analyzed.

Thus, Figure 23 shows the kinetic studies showing that FM19G11 causes a quick activation of ATR, associated with damage in the single strand of DNA in both HCT116 cell lines. After an hour of treatment with 0.5 µM FM19G11, an increase in the phosphorylation or activation of ATR and p53 in p53 cells in HCT116/p53+/+, as well as of ATR in HCT116 p53-/- cells, was detected. The results shown above were obtained after subjecting a representative western blotting experiment to densitometric analysis, although similar results were obtained in three independent experiments.

### Example 50: FM19G11 induces a G1/S phase cycle arrest in human colon cancer tumor cells in a p53-dependent manner.

It was investigated if the activation of DDR by FM19G11 caused a delay in the cycle progress through the G1/S phases in HCT116 tumor cells, as has been described for other DNA-damaging agents (.Lim, DS, Kim, ST, Xu, B, Maser, RS, Lin, J, Petrini, JH et al., (2000) ATM phosphorylates p95/nbs1 in an S-phase checkpoint pathway. Nature 404: 613-7.; Jazayeri, A, Falck, J, Lukas, C, Bartek, J, Smith, GC, Lukas, J et al., (2006) ATM- and cell cycle-dependent regulation of ATR in response to DNA double-strand breaks. Nat Cell Biol 8: 37-45; Liu, JS, Kuo, SR, Beerman, TA and Melendy, T, (2003) Induction of DNA damage responses by adozelesin is S phase-specific and dependent on active replication forks. Mol Cancer Ther 2: 41-7; Ranjan, P and Heintz, NH, (2006) S-phase arrest by reactive nitrogen species is bypassed by okadaic acid, an inhibitor of protein phosphatases PP1/PP2A. Free Radic Biol Med 40: 247-59.). The HCT116 p53+/+ and HCT116 p53-/- cells were exposed for 3 or 6 days to the carrier (DMSO) or to FM19G11 (0.5, 1, 5, 10 µM). In p53-proficient cells, treatment with 5 or 10 µM FM19G11 caused G1/S phase cycle arrest after 72 hours of treatment, being more evident after 6 days. However, in p53-deficient cells, FM19G11 did not cause a G1/S phase cycle blocking, indicating that the S phase cycle arrest caused by FM19G11 is dependent on the p53 protein. (Figure 24).

Thus, Figure 24 shows the effect of p53 on the FM19G11-induced cycle arrest. A- The HCT116/p53+/+ cells and B-HCT116/p53-/- cells were exposed to different concentrations of FM19G11 (0.5, 1, 5, 10 µM) or carrier for 3 or 6 days to analyze the distribution of cells in the different cell cycle phases by means of flow cytometry. The graphs show the effects caused by different concentrations of FM19G11 on the distribution of percentages of cells in each cell cycle phase. The results shown are representative of an experiment, although very similar percentages were obtained in three independent experiments.

### Example 51: FM19G11 affects the activation kinetics of the mTOR signaling pathway in HCT116 cells. Connection between the mTOR signaling pathway and the S phase cycle arrest

Due to the crucial role of p53 as a tumor-suppressing agent, it was analyzed in HCT116 cells how FM19G11 affected the activation kinetics of proteins involved in favoring cell cycle progress, specifically the mTOR cell signaling pathway. An increase of cell progress can be due to replicative stress which generates DNA damage (Bartkova, J, Horejsi, Z, Koed, K, Kramer, A, Tort, F, Zieger, K et al., (2005) DNA damage response as a candidate anti-cancer barrier in early human tumorigenesis. Nature 434: 864-70; Bartkova, J, Rezaei, N, Liontos, M, Karakaidos, P, Kletsas, D, Issaeva, N et al., (2006) Oncogene-induced senescence is part of the tumorigenesis barrier imposed by DNA damage checkpoints. Nature 444: 633-7; Di Micco, R, Fumagalli, M, Cicalese, A, Piccinin, S, Gasparini, P, Luise, C et al., (2006) Oncogene-induced senescence is a DNA damage response triggered by DNA hyper-replication. Nature 444: 638-42), it was explored if the DDR triggered by FM19G11 was related to an increase of the cell cycle progress signal modulated by the mTOR signaling pathway. An increase of phosphorylation of AKT, mTOR, p70S6 or increase in the expression of cyclin D1, not only in HCT116/p53^{+/+} cells but also in HT29 or in stem cells, indicating the activation of this signaling pathway after treatment with FM19G11 (0.5 µM), was observed at short times. The increase of cycle activation in the HCT116 cells mediated by the presence of FM19G11 coincided with an activation of the pathway associated with DDR, in the same way as occurs with its activation mediated by oncogenes ((Bartkova, J, Horejsi, Z, Koed, K, Kramer, A, Tort, F, Zieger, K et al., (2005) DNA damage response as a candidate anti-cancer barrier in early human tumorigenesis. Nature 434: 864-70; Bartkova, J, Rezaei, N, Liontos, M, Karakaidos, P, Kletsas, D, Issaeva, N et al., (2006) Oncogene-induced senescence is part of the tumorigenesis barrier imposed by DNA damage checkpoints. Nature 444: 633-7; Di Micco, R, Fumagalli, M, Cicalese, A, Piccinin, S, Gasparini, P, Luise, C et al., (2006) Oncogene-induced senescence is a DNA damage response triggered by DNA hyper-replication. Nature 444: 638-42). The phosphorylation pattern of the AKT/mTOR/p70S6/Cyclin D1 pathway caused by treatment with FM19G11 differs in the HCT116/p53^{-/-} cells, although the quick activation of the ATR pathway, due to the treatment with FM19G11, is preserved. (Figure 25).

The kinetic studies of Figure 25 show a quick and efficient activation (fold change ≥2) of the AKT/mTOR/p70S6/Cyclin D1 signaling pathway in HCT116/p53+/+ cells treated with FM19G11 (0.5 µM). mTOR was also phosphorylated quickly (but with an increase of fold change slightly ≤ 2) in HCT116/p53-/- cells after the treatment with FM19G11.

To elucidate if the S phase cycle arrest is related to the induction of the AKT/mTOR/p70S6 pathway by FM19G11, the HCT116 cells were treated with FM19G11 (10 µM) in combination with the mTOR inhibitor, rapamycin (100 pmol) and the induced S phase arrest in the HCT116/p53^{+/+} cells was not observed when they were only treated with FM19G11 (10 µM). (Figure 26).

The HCT116/p53+/+ and HCT116/p53-/- cells were exposed to FM19G11 (10 µM), rapamycin (R) (an mTOR inhibitor) (100 pmol) alone or a combination of both drugs at the mentioned concentrations. The S phase cycle arrest occurring in HCT116/p53+/+ cells by treatment with FM19G11 was prevented when a simultaneous incubation of both drugs (100 pmol) was used.

In short, FM19G11 induces the activation of the mTOR pathway and cell cycle progress signals (increase of Cyclin D1), this causes a trigger of the DNA damage signal by replicative stress, similarly to an oncogene ((Bartkova, J, Horejsi, Z, Koed, K, Kramer, A, Tort, F, Zieger, K et al., (2005) DNA damage response as a candidate anti-cancer barrier in early human tumorigenesis. Nature 434: 864-70; Bartkova, J, Rezaei, N, Liontos, M, Karakaidos, P, Kletsas, D, Issaeva, N et al., (2006) Oncogene-induced senescence is part of the tumorigenesis barrier imposed by DNA damage checkpoints. Nature 444: 633-7; Di Micco, R, Fumagalli, M, Cicalese, A, Piccinin, S, Gasparini, P, Luise, C et al., (2006) Oncogene-induced senescence is a DNA damage response triggered by DNA hyper-replication. Nature 444: 638-42), and consequently causes an activation of p53 which is responsible for the G1/S phase cycle arrest. This makes the mode of action of the drug be novel compared to other drugs such as cisplatin which also depends, like FM19G11, on p53 being operative.

### Example 52: p53 sensitizes human colon cancer cells (HCT116 and HT29) to the drug FM19G11

To determine the effects of FM19G11 on human colon cancer cells (HCT116 and HT29), cell viability assays were carried out in HT29, HCT116/p53^{+/+} and HCT116/p53^{-/-} cells by means of quantifying the ATP which associates with metabolically active cells. The results shown in Figure 27 show that the HCT116/p53 ^{-/-} cells are less sensitive to the presence of FM19G11 after 48 hours of treatment (0.5, 1, 5, 10, 50, 100, 1000 µM) compared to the sensitivity shown by HCT116/p53^{+/+} or HT29 (p53-proficient cells).

The total ATP content of the cells (Figure 28) is correlated with the cell viability, which was quantified as percentage of human colon cancer cells treated with FM19G11 and relative to the corresponding cell lines treated with DMSO for 48 hours.

### Example 53: Effects of FM19G11 on the formation of colonies of human colon cancer cells

Clonicity assays were conducted to determine the toxicity of FM19G11 in p53-proficient (HT29 and HCT116/p53^{+/+}) and p53-deficient (HCT116/p53^{-/-}) tumor cells. Once the HT-29, HCT116/p53^{+/+} and HCT116/p53^{-/-} cells were adhered to the culture plate, they were stimulated with increasing concentrations of FM19G11 (0.5, 1, 5, 10, 20, 50 µM) for 72 hours. After 10 days of stimulation, the number of colonies formed in the plates of treated and untreated cells was determined. The evaluation of the number of colonies (Figure 28) showed that FM19G11 decreases the clonicity of the HT29, HCT116/p53^{+/+} and HCT116/p53^{-/-} cells, with a survival IC₅₀ of 1 µM, 3.5 µM, 16.5 µM, respectively. Therefore, the loss of function of p53 in HCT116 cells confers an increase of resistance to FM19G11 of 4.71 times, according to the quantification of ratios of the IC₅₀ values in HCT116.

Results of the clonicity assays of HT29 human colon cancer cells, HCT116 cells treated with FM19G11 were monitored up to 10 after the treatment (Figure 28). The IC₅₀ was determined by extrapolating the percentage with the concentration. FM19G11 was used at concentrations: 0.5, 1, 5, 10, 50, 100, 200 µM

### Example 54: Anchorage-independent cell growth

Anchorage-independent cell growth is a study which allows predicting the tumorigenicity of tumor cells *in vivo.* Anchorage-independent cell growth studies were performed for HT29, HCT116/p53^{+/+} and HCT116/p53^{-/-} cells in semisolid agar which were monitored daily to determine the efficiency of the formation of colonies in the presence of carrier (DMSO) or FM19G11. To that end, the starting material was 75 x 10³ cells suspended in semisolid agar, which were maintained until 10 days later. It was possible to observe (Figure 29) a significant decrease of the growth of colonies of p53-proficient cells, such as HT29 (p ≤ 0.005) and HCT116/p53^{+/+} (p ≤ 0.033) in semisolid agar medium when these cells were treated with FM19G11 (0.5 µM) compared to those treated with carrier (DMSO). A reduction of the formation of colonies in the HCT116/p53^{-/-} cells is not observed. The growth of colonies was quantified in three independent experiments by means of counting colonies in the 4 x magnification optical field of an inverted optical microscope.

Figure 29 shows the formation of colonies of HT29, HCT116/p53+/+ and HCT116/p53-/- cells in semisolid agar medium. The number of colonies of each cell type which grew in noble agar was quantified 10 days after the seeding. The table shows the means of the different counts of the number of colonies of each of the mentioned cell lines (±SD) by five different fields, with a 4x lens magnification. The results represent counts of three independent experiments in which cells seeded and treated with carrier (DMSO) or FM19G11 (0.5 µM) were counted. Photographs representative of the formation of colonies in the HT29 tumor cell line were taken 10 days after the seeding in semisolid medium.

### Example 55: FM19G11 recovers decrease of expression of MMR and prevents the genomic instability caused by hypoxic conditions.

The decrease of expression of the DNA mutation repair (MMR) genes caused by hypoxic conditions in stem cells can entail the onset of genomic instability (Rodriguez-Jimenez FJ, M-MV, Lucas-Dominguez R, Sanchez-Puelles JM. Hypoxia Causes Down-Regulation of Mismatch Repair System and Genomic Instability in Stem Cells. Stem Cells. May 29 2008). Genomic instability has been widely associated with the acquirement of tumor phenotype (Scherer, SJ, Avdievich, E and Edelmann, W, (2005) Functional consequences of DNA mismatch repair missense mutations in murine models and their impact on cancer predisposition. Biochem Soc Trans 33: 689-93; Fishel, R, Lescoe, MK, Rao, MR, Copeland, NG, Jenkins, NA, Garber, J et al., (1993) The human mutator gene homolog MSH2 and its association with hereditary nonpolyposis colon cancer. Cell 75: 1027-38; Aaltonen, THE, Peltomaki, P, Leach, FS, Sistonen, P, Pylkkanen, L, Mecklin, JP et al., (1993) Clues to the pathogenesis of familial colorectal cancer. Science 260: 812-6; Eckert, A, Kloor, M, Giersch, A, Ahmadi, R, Herold-Mende, C, Hampl, JA et al., (2007) Microsatellite instability in pediatric and adult high-grade gliomas. Brain Pathol 17: 146-50). FM19G11 is capable of recovering the expression of DNA repair proteins. This could prevent the onset of genomic instability in stem cells located in hypoxic niches, which would entail a compromise for the organism, since the stem cells could be self-renewed or differentiate into other more specialized cells which would continue with the genomic instability present in the predecessor cell. The possibility that FM19G11 increases the expression of repair proteins which are down-regulated under hypoxic conditions allows thinking of FM19G11 as a possible antitumor agent, not only due to the described properties of inhibition of the HIF tumor progression factor, trigger of DNA damage response or cell cycle arrest, but also as an agent which prevents an increase of genomic instability under hypoxic conditions.

Figure 30 shows how the C17.2 neural stem cells and mouse neurospheres experience a decrease of the levels of the MSH6 repair protein under hypoxic conditions which are recovered in the presence of FM19G11. Under normoxic conditions, a decrease of the MSH6 protein by treatment with DMSO which is rescued by FM19G11 is observed in C17.2 cells.

Figure 31 shows how the genomic instability in mouse neurospheres (neural stem cells) caused by hypoxia is prevented by treatment with FM19G11. By using specific fluorescence-labeled primers for microsatellite sequence markers, the genomic stability of neurospheres in normoxia (Nx), hypoxia (Hx) or hypoxia+FM19G11 (Hx+19G11) was analyzed. In said figure it can be observed how two of the markers used *(mBAT59* and *mBAT67)* showed deletions or insertions in the DNA due to hypoxic conditions. The treatment with FM19G11 enabled the recovery of the normoxia profiles (used as a reference or control), thus preventing the genomic instability caused by hypoxia.

### Example 56: FM19 G11 has the capacity to remodel chromatin by epigenetic events

The findings in the laboratory show that FM19G11 is capable of affecting the expression of p300, a histone acetyltransferase enzyme, i.e., it transfers acetyl groups to the histones on which the DNA (nucleosomes) is coiled and which form the chromatin. Depending on the cell type, FM19G11 favors changes in the expression of p300 and of the degree of acetylation of certain chromosomal regions. For example, according to previous findings (Rodriguez-Jimenez FJ, M-MV, Lucas-Dominguez R, Sanchez-Puelles JM. Hypoxia Causes Down-Regulation of Mismatch Repair System and Genomic Instability in Stem Cells. Stem Cells. May 29 2008), the hypoxia and the associated epigenetic events cause a decrease of the levels of repair proteins for DNA mutations (MSH6, MLH1). It was observed in the mentioned work that in hypoxia a closed conformation of chromatin was produced due to a decrease of the acetylation of the histones H3 in the promoter regions of the *MLH1 and MSH6* genes. However, in the presence of FM19G11 there was an increase of p300 and of the repair proteins with respect to the cells treated with carrier. It was possible to observe how the drug caused an increase of the overall acetylation of proteins, even to a greater extent when they were treated with TSA (histone deacetylase inhibitor) which prevents histones from being deacetylated.

TSA has been proposed as a possible antitumor drug in breast cancer and is in the preclinical phase (Vigushin, DM, Ali, S, Pace, PE, Mirsaidi, N, Ito, K, Adcock, I et al., (2001) Trichostatin A is a histone deacetylase inhibitor with potent antitumor activity against breast cancer in vivo. Clin Cancer Res 7: 971-6.). FM19G11 forces the acetylation of proteins as effectively or more than TSA, FM19G11 behaves like an agent which forces acetylation in this type of neural stem cell (C17.2) and which could be related to the observed recovery of the MLH1 and MSH6 repair proteins, since considering previous studies, these proteins are regulated by epigenetic processes (Rodriguez-Jimenez FJ, M-MV, Lucas-Dominguez R, Sanchez-Puelles JM. Hypoxia Causes Down-Regulation of Mismatch Repair System and Genomic Instability in Stem Cells. Stem Cells. May 29 2008).

Figure 32 shows how FM19G11 causes changes of expression of p300 (a histone acetyltransferase) and of DNA repair proteins (MLH1 and MSH6) under normoxic and hypoxic conditions in C17.2 neural stem cell. FM19G11 affects the degree of acetylation of global proteins. TSA was included as a positive control for an agent forcing histone acetylation.

Although the changes in the degree of acetylation are evident when the cells are treated with FM19G11, the genetic background of each cell type affects the epigenetic changes caused by FM19G11 and, consequently, it affects their increase or decrease of the expression. Thus, the ependymal cells obtained from spinal cord acquire a lower overall degree of acetylation of global histones H3, FM19G11 generating a more closed conformation of chromatin. Under physical hypoxic conditions, FM19G11 is capable of causing in this cell type a decrease of certain proteins such as the two isoforms of HIFα, the mentioned p300, as well as the undifferentiation markers: Sox2, Oct3/4. The decrease of these proteins is associated with a decrease of the acetylation of histone H3, which confers a conformation of heterochromatin (closed conformation) and which would prevent the access of transcription-activating factors.

Figure 33 shows how the effects caused by FM19G11 on the expression of proteins of adaptation to oxygen changes (HIFα), undifferentiation markers (Sox2, Oct3/4), histone acetyltransferase (p300) and acetylated histone H3, under normoxic or hypoxic conditions (left panel). Influence of FM19G11 on the state of acetylation of histone H3 in normoxia and hypoxia determined by means of immunoprecipitation of chromatin using to that end an anti-AcH3 antibody (acetylated histone H3) and quantifying the DNA by means of quantitative PCR (right graph).

Since FM19G11 was capable of affecting the degree of acetylation of histone H3 and therefore the conformation of chromatin, it was evaluated if FM19G11 favored the transfer of acetyl groups to histone H3 or, on the contrary, prevented histone H3 from being deacetylated. Both effects would cause an increase of the degree of acetylation of histone H3. Immunoprecipitation assays were performed with an antibody recognizing acetyltransferase p300 and then assays for measuring the acetyltransferase activity by means of using the kit "HAT Colorimetric Assay" (BioVision) were carried out. This method consists of detecting NADH since peptide acetylation causes the release of the free form of Coenzyme A, which serves as an essential coenzyme for producing NADH. It was observed that in C17.2 cells treated with FM19G11 there was an increase of the acetyltransferase activity of the immunoprecipitated fraction, regardless of the percentage of oxygen. However, according to the results obtained from the assays performed with the kit HDAC Fluorimetric Cellular Activity Assay (Biomol, AK-503), FM19G11 did not show activity as a histone deacetylase inhibitor.

Figure 34 A shows the acetyltransferase (HAT) activity of the protein extracts of cells which are untreated, treated with carrier or FM19G11, cultured under normoxic or hypoxic conditions. The protein extracts were previously immunoprecipitated with an anti-p300 antibody and the acetyltransferase activity measured by the detection of NADH. The evaluation of the histone deacetylase (HDAC) activity (Figure 34 B) was carried out with protein extracts of HeLa cells treated with different concentrations of FM19G11. As a positive control, a fixed concentration of TSA (1 µM) was included and extracts of untreated cells were used as a control. The experiments were carried out following the instructions of the Biomol kit for detecting histone deacetylase activity (HDAC Fluorimetric Cellular Activity Assay, Biomol, Cat. No. AK-503).

## Claims

1. A compound of general formula (I): 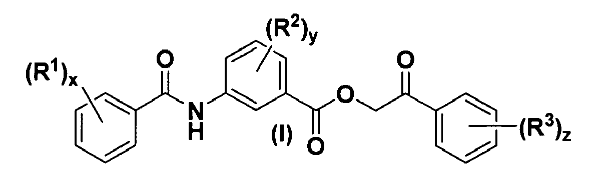 wherein R¹, R² and R³ can independently be:
(i) hydrogen;
(ii) a branched or linear -(CH₂)ₙ-H alkyl group, optionally with an unsaturation, wherein n= 1-10, preferably methyl or ethyl;
(iii) a -COOH carboxylic acid or an ester of formula -COOR, R being a branched or linear -(CH₂)ₙ-H alkyl group, optionally with an unsaturation, wherein n= 1-10, preferably methyl or ethyl;
(iv) -OH hydroxyl group, -NO₂ group;
(v) halogen; -CHa₃ group, wherein Ha= halogen, preferably CF₃;
(vi) -NHCOR, R being a branched or linear -(CH₂)ₙ-H alkyl group, optionally
with an unsaturation, wherein n= 1-10, preferably methyl or ethyl x, y, z being= 1-4;
or a pharmaceutically acceptable salt thereof, except for 2-(4-methoxyphenyl)-2-oxoethyl 3-(2,4-(dinitro)benzamido)benzoate (R¹= 2,4-di-NO₂, R²= H, R³= 4-OMe), and 2-4-(nitrophenyl)-2-oxoethyl 3-(4-bromo)benzamido)benzoate (R¹= 4-Br, R²= H, R³= 4-NO₂).

2. The compound of general formula (I) or a pharmaceutically acceptable salt thereof according to claim 1, called FM19G11, wherein R¹= 2,4-di-NO₂, R²= -H, and R³= -CH₃ (4).

3. The compound of general formula (I) or a pharmaceutically acceptable salt thereof according to claim 1, wherein R¹= 2-NO₂ 4-CF₃, R²= -H, and R³= -CH₃ (7).

4. The compound of general formula (I) or a pharmaceutically acceptable salt thereof according to claim 1, wherein R¹= 2-CF₃, 4-NO₂, R²= -H, and R³= -CH₃ (10).

5. The compound of general formula (I) or a pharmaceutically acceptable salt thereof according to claim 1, wherein R¹= 2-4-CF₃, R²= -H, and R³= -CH₃ (13).

6. The compound of general formula (I) or a pharmaceutically acceptable salt thereof according to claim 1, wherein R¹= -H, R²= -H, and R³= -CH₃ (16).

7. The compound of general formula (I) or a pharmaceutically acceptable salt thereof according to claim 1, wherein R¹= 2-4-NO₂, R²= -H, and R³= -CF₃ (17).

8. The compound of general formula (I) or a pharmaceutically acceptable salt thereof according to claim 1, wherein R¹= 2-4-NO₂, R²= -H, and R³= -H (19).

9. The compound of general formula (I) or a pharmaceutically acceptable salt thereof according to claim 1, wherein R¹= 4-NO₂, R²= -H, and R³= -CH₃ (22).

10. The compound of general formula (I) or a pharmaceutically acceptable salt thereof according to claim 1, wherein R¹= 2- NO₂, R²= -H, and R³= -CH₃ (25).

11. The compound of general formula (I) or a pharmaceutically acceptable salt thereof according to claim 1, wherein R¹= 2-4-NO₂, R²= -H, and R³= -OH (26).

12. The compound of general formula (I) or a pharmaceutically acceptable salt thereof according to claim 1, wherein R¹= 2-4 NO₂, R²= -H, and R³= -CO₂H (29).

13. A method for obtaining the compound of general formula (I), called FM19G11, or a pharmaceutically acceptable salt thereof according to claims 1-2, comprising the steps of the following scheme:

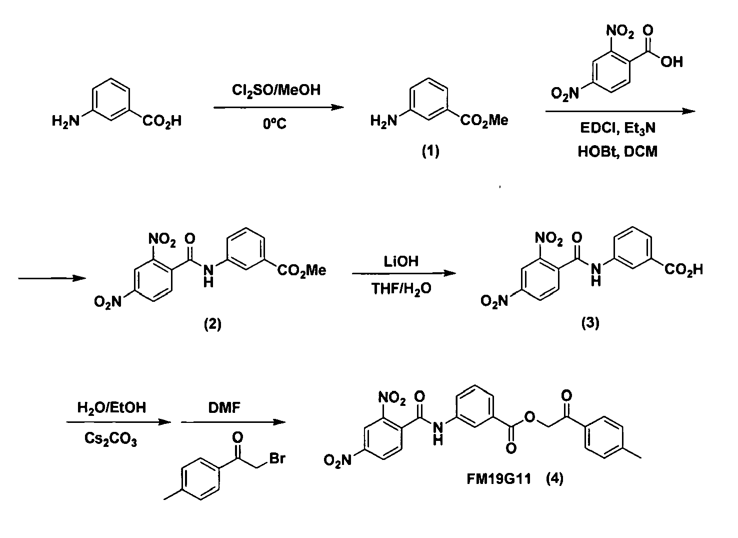

14. Use of one or more compounds of general formula (I) or a pharmaceutically acceptable salt thereof according to claims 1-12 for preparing a medicament for regulating the transcription of genes modulated by hypoxia-inducible transcription factor (HIF).

15. Use of one or more compounds of general formula (I) or a pharmaceutically acceptable salt thereof according to claim 14 for preparing a medicament which regulates the transcription of one or more genes selected from the group: HIF, VEGF, PHD3, MMRs, Sox2 and Oct3/4.

16. Use of one or more compounds of general formula (I) or a pharmaceutically acceptable salt thereof according to claim 14 for preparing a medicament which regulates the transcription of one or more genes selected from the group: Nanog, Notch 1, p53 and p300.

17. Use of one or more compounds of general formula (I) or a pharmaceutically acceptable salt thereof according to claims 15-16 for preparing a medicament which inhibits the transcription of HIF in its autocrine regulation under hypoxic conditions.

18. Use of one or more compounds of general formula (I) or a pharmaceutically acceptable salt thereof according to claim 17 for preparing a medicament which significantly inhibits the transcription of HIF 1□ and HIF 2□ under hypoxic conditions.

19. Use of one or more compounds of general formula (I) or a pharmaceutically acceptable salt thereof according to claim 15 for preparing a medicament which significantly inhibits the transcription of VEGF and/or PHD3 under hypoxic conditions.

20. Use of one or more compounds of general formula (I) or a pharmaceutically acceptable salt thereof according to claim 15 for preparing a medicament which induces the expression of MMR repair genes under hypoxic and normoxic conditions.

21. Use of one or more compounds of general formula (I) or a pharmaceutically acceptable salt thereof according to claim 20 for preparing a medicament which induces the expression of MMR repair genes under hypoxic and normoxic conditions wherein said genes are MSH6 and MLH1.

22. Use according to claims 20-21, **characterized in that** said medicament prevents the genomic instability associated with the tumor phenotype caused by hypoxic conditions.

23. Use of one or more compounds of general formula (I) or a pharmaceutically acceptable salt thereof according to claim 15 for preparing a medicament which induces the expression of Sox2 and Oct3/4 under normoxic conditions.

24. Use of one or more compounds of general formula (I) or a pharmaceutically acceptable salt thereof according to claim 16 for preparing a medicament which induces the expression of Nanog and/or Notch under normoxic conditions.

25. Use according to claims 23-24, **characterized in that** said medicament induces the increase of the population of stem cells, increasing their self-renewal.

26. Use of one or more compounds of general formula (I) or a pharmaceutically acceptable salt thereof according to claim 15 for preparing a medicament which reduces the expression levels of Sox2 and Oct3/4 under hypoxic conditions.

27. Use of one or more compounds of general formula (I) or a pharmaceutically acceptable salt thereof according to claim 16 for preparing a medicament which induces the expression of the p53 tumor suppressor under hypoxic conditions.

28. Use according to claim 27, **characterized in that** said induction occurs in adult human dental pulp stem cell.

29. Use according to claims 26-27, **characterized in that** the mode of action of said medicament induces the activation of the mTOR pathway.

30. Use according to claim 29, **characterized in that** said activation of the mTOR pathway causes a triggering of the response to DNA damage which results in the activation of p53 with G1/S phase cell cycle arrest in a p53-dependent manner.

31. Use of one or more compounds of general formula (I) or a pharmaceutically acceptable salt thereof according to claim 16 for preparing a medicament which modulates the expression of p300 under hypoxic conditions.

32. Use according to claim 31, **characterized in that** said medicament is capable of chromatin remodeling by epigenetic events.

33. Use of one or more compounds of general formula (I) or a pharmaceutically acceptable salt thereof according to any of claims 14-32 for preparing a medicament for the treatment of angiogenic pathologies.

34. Use of one or more compounds of general formula (I) or a pharmaceutically acceptable salt thereof according to any of claims 14-33 for preparing a medicament for the treatment of cancer.

35. Use of one or more compounds of general formula (I) or a pharmaceutically acceptable salt thereof according to any of claims 14-34, wherein the cancer can be breast cancer, colon cancer, bladder cancer, head and neck cancer, lung cancer, ovarian cancer, prostate cancer, testicular cancer, uterine cancer, thyroid cancer, gastric cancer, liver cancer, lymphoma or melanoma.

36. Use of one or more compounds of general formula (I) or a pharmaceutically acceptable salt thereof according to any of claims 14-33 for preparing a medicament for the treatment of tumors.

37. Use of one or more compounds of general formula (I) or a pharmaceutically acceptable salt thereof according to claims 14-33 for preparing medicaments for the treatment of pathologies which cause inflammation.

38. Use of one or more compounds of general formula (I) or a pharmaceutically acceptable salt thereof according to claims 14-33 for its use in regenerative medicine.

39. Use of one or more compounds of general formula (I) or a pharmaceutically acceptable salt thereof according to claim 38 relating to stem cell differentiation.

40. Use of one or more compounds of general formula (I) or a pharmaceutically acceptable salt thereof according to claims 14-33 for preparing a medicament for the treatment of pathologies which cause tissue degeneration.

41. A pharmaceutical composition comprising one or more compounds of general formula (I) or a pharmaceutically acceptable salt thereof according to claims 1-12 in a pharmaceutically effective amount, a carrier or diluent and/or a physiologically acceptable adjuvant.

42. The pharmaceutical composition according to claim 41, wherein the compound of general formula (I) is FM19G11 (4) or a pharmaceutically acceptable salt thereof.

43. The pharmaceutical composition according to claims 41-42, which can be used to treat or prevent angiogenic pathologies according to claims 14-40.

44. A kit comprising the pharmaceutical composition according to claims 41-43 with one or more compounds of general formula (I), a carrier or diluent and/or one or more adjuvants, which can be together or separate in one or more containers depending on the administration route of the composition in each case.
